# EUROPÄISCHE PATENTSCHRIFT

(11) **EP 3 815 291 B1**
(45) Veröffentlichungstag und Bekanntmachung des Hinweises auf die Patenterteilung: **25.10.2023**
(21) Anmeldenummer: 19731243.2
(22) Anmeldetag: 13.06.2019
(51) Int. Cl.: H04L 9/00, G06F 21/64, G06Q 30/00, G16H 80/00, G06Q 10/08

(54) **FÄLSCHUNGSSICHERUNG UND ABGABEKONTROLLE VON VERBRAUCHSGÜTERN**
PROTECTION AGAINST FALSIFICATION AND HANDOVER CONTROL OF CONSUMER GOODS
SÉCURITÉ CONTRE LA FALSIFICATION ET CONTRÔLE DE REMISE DE BIENS DE CONSOMMATION

(30) Priorität: 26.06.2018 DE 102018115348
(43) Veröffentlichungstag der Anmeldung: 05.05.2021
(73) Patentinhaber: Bundesdruckerei GmbH Technology Intellectual Property, 10969 Berlin (DE)
(72) Erfinder: RÜCKRIEMEN, Jörg, 10999 Berlin (DE)
(74) Vertreter: Richardt Patentanwälte PartG mbB
(86) Internationale Anmeldenummer: PCT/EP2019/065541
(87) Internationale Veröffentlichungsnummer: WO 2020/001996

(56) Entgegenhaltungen:
- WO-A1-2017/165909
- US-A1- 2018 117 447
- US-A1- 2018 130 034
- Alessandro Chiesa ET AL: "The MediLedger Project 2017 Progress Report", , 28. Februar 2018 (2018-02-28), XP055619759, Gefunden im Internet: URL:https://uploads-ssl.webflow.com/59f37d 05831e85000160b9b4/5aaadbf85eb6cd21e9f0a73 b_MediLedger%202017%20Progress%20Report.pd f [gefunden am 2019-09-09]

## Beschreibung

Die Erfindung betrifft ein Verfahren zur Fälschungssicherung und Abgabekontrolle von Verbrauchsgütern und Verwendung einer Blockchain sowie ein Blockchain-Netzwerk zum Ausführen des entsprechenden Verfahrens.

Fälschungen von Verbrauchsgütern, insbesondere im pharmazeutischen und medizinischen Bereich, können neben einem erheblichen wirtschaftlichen Schaden für die Hersteller der Originalverbrauchsgüter eine Gesundheitsgefahr für die Endverbraucher darstellen. Neben der Fälschungssicherung, bei welcher es unter anderem darum geht, zu verhindern, dass gefälschte Verbrauchgüter in offizielle Vertriebskette eingeschleust werden, besteht im Bereich der pharmazeutischen und medizinischen Verbrauchsgüter, insbesondere im Bereich der Betäubungsmittel, zudem die Notwendigkeit einer Abgabekontrolle, um Missbrauch vorzubeugen. Es geht somit nicht nur darum zu verhindern, dass Verbrauchsgüter unerlaubterweise in die Vertriebskette eingeschleust werden, sondern auch darum, zu verhindern, dass Verbrauchsgüter unerlaubterweise aus der entsprechenden Vertriebskette entnommen werden.

Die Publikation "The Mediledger Project 2017 Progress Report" des Mediledger Projects beschreibt ein blockchainbasiertes System zur Verfolgung legaler Eigentümerwechsel bei verschreibungspflichtigen Medikamenten zur Einhaltung des Drug Supply Chain Security Acts (DSCSA).

Die US 2018/117447 A1 beschreibt ein Gerät des Internets der Dinge (Internet of Thing/IoT), welches eine Kamera umfasst, die mit einem Prozessor gekoppelt ist, und einen drahtlosen Transceiver, der mit einem Prozessor gekoppelt ist. Blockchain Smart Contracts können mit dem Gerät verwendet werden, um einen sicheren Betrieb zu ermöglichen.

Die WO 2017/165909 A1 beschreibt ein Verfahren zum Aufzeichnen einer Verwahrkette und zum Identifizieren von Anomalien in der Kette. Das Verfahren umfasst: Definieren oder Identifizieren eines ersten Elements und Zuordnen eines ersten Elementidentifikators zu diesem; physisches Verbinden des ersten Elementidentifikators mit dem ersten Element; Definieren oder Identifizieren mindestens eines zweiten Elements und Zuordenen eines zweiten Elementidentifikators zu diesem; physisches Verbinden des zweiten Elementidentifikators mit dem zweiten Element; Aggregieren des mindestens ersten Elements und des zweiten Elements physisch zu einem aggregierten Element und Zuordnen eines aggregierten Elementidentifikator zu diesem; Definieren eines aggregierten Elementdatensatzes, der den aggregierten Elementidentifikator und den mindestens ersten Elementidentifikator und den mindestens zweiten Elementidentifikator enthält; Aufzeichnen der Identifikatoren als Datensätze in einer Datenbank.

Die US 2018/130034 A1 beschreibt ein blockchainbasierte System sowie Verfahren, welche umfassen: sichere Wallets; randomisierte, sichere Identifikatoren zum eindeutigen Identifizieren diskreter Gegenstände; und kryptographisch sichere, zeitgestempelte Blockchains. Rollenbasierte sichere Wallets enthalten private kryptografische Schlüssel zum digitalen Signieren von Transaktionen zur Aufzeichnung in einer Blockchain. Operationen, die mit rollenbasierten Wallets durchzuführen sind, können auf der Grundlage der mit den jeweiligen Wallets verbundenen Privilegien zugelassen oder eingeschränkt werden. Eine Mehrzahl von Blockchains kann ferner zur Nachverfolgung von Transaktionen verwendet werden, bei denen es um unterschiedliche Rechnungseinheiten geht.

Der Erfindung liegt daher die Aufgabe zugrunde, ein effizientes und sicheres Verfahren zur Fälschungssicherung und Abgabekontrolle von Verbrauchsgütern zu schaffen.

Die der Erfindung zugrundeliegende Aufgabe wird jeweils mit den Merkmalen der unabhängigen Patentansprüche gelöst. Ausführungsformen der Erfindung sind in den abhängigen Patentsprüchen angegeben.

Ausführungen umfassen ein Verfahren zur Fälschungssicherung von Verbrauchsgütern unter Verwendung einer Blockchain. Die Blockchain speichert für jedes der Verbrauchsgüter eine kryptografisch gesicherte Historie. Die Blockchain wird von einem Blockchain-Netzwerk bereitgestellt, welches eine Mehrzahl von Blockchain-Servern umfasst. Die Blockchain-Server sind jeweils dazu konfiguriert, unter Verwendung eines von der Blockchain umfassten Programmmoduls zusätzliche Blöcke für die Blockchain zu erstellen und zu der Blockchain hinzuzufügen.

Das Verfahren umfasst zur kryptografisch gesicherten Speicherung einer Historie eines ersten Verbrauchsgutes:
- im Fall einer Herstellung des ersten Verbrauchsgutes, Empfangen einer ersten Registrierungsanforderung zum Registrieren des ersten Verbrauchsgutes in der Blockchain durch einen der Blockchain-Server des Blockchain-Netzwerks, wobei die erste Registrierungsanforderung einen Herstellerdatensatz mit Herstellungsdaten des ersten Verbrauchsgutes und mit einem Herstellerprüfwert umfasst, wobei der Herstellerprüfwert zur Prüfung einer Berechtigung zur Übertragung des ersten Verbrauchsgutes durch einen Hersteller des ersten Verbrauchsgutes dient,
- Ausführen erster Programminstruktionen des Programmoduls durch den die erste Registrierungsanforderung empfangenden Blockchain-Server, wobei das Ausführen der ersten Programminstruktionen eine Prüfung umfasst, ob die erste Registrierungsanforderung ein oder mehrere erste Voraussetzungen erfüllen, und, falls die ersten Voraussetzungen erfüllt sind, ein Hinzufügen des Herstellerdatensatzes zu einem Eintrag in einem zusätzlichen Block der Blockchain, wodurch das erste Verbrauchsgut in der Blockchain registriert, der Herstellerprüfwert dem ersten Verbrauchsgut als ein aktueller Prüfwert zugeordnet und dem Hersteller als einem aktuellen Besitzer des ersten Verbrauchsgutes die Berechtigung zur Übertragung des ersten Verbrauchsgutes zugeordnet wird.

Ferner umfasst das Verfahren ein oder mehrmals:
- im Fall eines Besitzerwechsels des ersten Verbrauchsgutes, Empfangen einer Übertragungsbestätigungsanforderung zur Bestätigung des Besitzerwechsels des ersten Verbrauchsgutes durch einen der Blockchain-Server des Blockchain-Netzwerks, wobei die Übertragungsbestätigungsanforderung einen Identifikator des ersten Verbrauchsgut, einen Berechtigungsnachweis des aktuellen Besitzers vor dem Besitzerwechsel zur Übertragung des ersten Verbrauchsgutes und einen weiteren Prüfwert umfasst, wobei der weitere Prüfwert zur zukünftigen Prüfung einer Berechtigung zur Übertragung des ersten Verbrauchsgutes durch einen Empfänger des ersten Verbrauchsgutes als zukünftigem Besitzers dient,
- Ausführen zweiter Programminstruktionen des Programmoduls durch den die Übertragungsbestätigungsanforderung empfangenden Blockchain-Server, wobei das Ausführen der zweiten Programminstruktionen eine Prüfung unter Verwendung des aktuellen Prüfwerts umfasst, ob die Übertragungsbestätigungsanforderung ein oder mehrere zweite Voraussetzungen erfüllt, und, falls die zweiten Voraussetzungen erfüllt sind, ein Hinzufügen des weiteren Prüfwerts und des Identifikators des ersten Verbrauchsgutes zu einem Eintrag in einem zusätzlichen Block der Blockchain, wodurch der weitere Prüfwert dem ersten Verbrauchsgut als aktueller Prüfwert in Ersatz zu dem bisherigen aktuellen Prüfwert zugeordnet wird und wobei dem weiteren Besitzer als aktuellem Besitzer des ersten Verbrauchsgutes in Ersatz zu dem bisherigen aktuellen Besitzer die Berechtigung zur Übertragung des ersten Verbrauchsgutes zugeordnet wird.

Ferner umfasst das Verfahren:
- im Fall einer Endabgabe des ersten Verbrauchsgutes an einen Endverbraucher, Empfangen einer Endabgabebestätigungsanforderung zur Bestätigung der Endabgabe des ersten Verbrauchsgutes durch einen der Blockchain-Server des Blockchain-Netzwerks, wobei die Endabgabebestätigungsanforderung den Identifikator des ersten Verbrauchsgutes und einen Berechtigungsnachweis des aktuellen Besitzers vor der Endabgabe zur Übertragung des ersten Verbrauchsgutes umfasst,
- Ausführen dritter Programminstruktionen des Programmoduls durch den die Endabgabebestätigungsanforderung empfangenden Blockchain-Server, wobei das Ausführen der dritten Programminstruktionen eine Prüfung unter Verwendung des aktuellen Prüfwerts umfasst, ob die Endabgabebestätigungsanforderung des aktuellen Besitzers ein oder mehrere dritte Voraussetzungen erfüllt, und, falls die dritten Voraussetzungen erfüllt sind, ein Hinzufügen des Identifikators des ersten Verbrauchsgutes und eines Endabgabevermerk zu einem Eintrag in einem zusätzlichen Block der Blockchain, wodurch der Endabgabevermerk dem ersten Verbrauchsgut zugeordnet wird, wobei der Endabgabevermerk festlegt, dass das erste Verbrauchsgut von weiteren Übertragungen ausgeschlossen ist.

Ausführungsformen können den Vorteil haben, dass sie eine Erstellung einer vollständigen kryptografischen gesicherten Historie einzelner Verbrauchsgüter ermöglichen, anhand derer der Weg des entsprechenden Verbrauchsguts von seiner Herstellung bis zur Endabgabe an einen Endverbraucher lückenlos nachvollzogen werden kann. Unter Verwendung eines solchen Track & Trace Verfahrens können die Wege der Verbrauchsgüter vollständig rückverfolgt werden bis zu ihrem Ursprung und Sicherheitslücken in der Vertriebskette können ausgeschlossen werden. Zudem kann somit sichergestellt werden, dass keines der entsprechenden Verbrauchsgüter unzulässigerweise aus der Vertriebskette entnommen wurde. Die Blockchain bietet eine vollständige Dokumentation der entsprechenden Vertriebskette und kann mithin auch als Nachweis dienen, dass die entsprechenden Verbrauchsgüter entsprechend geltenden Vorschriften gehandhabt wurden.

Im Zuge der Herstellung eines Verbrauchsgutes trägt es der Hersteller unter Verwendung einer entsprechenden Registrierungsanforderung in der Blockchain ein. Mithin ist das Verbrauchsgut registriert und es kann anhand der Blockchain jederzeit nachverfolgt werden, ob das entsprechende Verbrauchsgut sich noch in der Vertriebskette befindet und, falls ja, wo es sich befindet oder ob es bereits an den Endverbraucher abgegeben wurde und gegebenenfalls an wen. Als Ausgangspunkt wird dem entsprechenden Verbrauchsgut ein Herstellerprüfwert zugeordnet. Dieser Herstellerprüfwert dient zur Prüfung der Berechtigung des Herstellers zur Übertragung, insbesondere legitimen Übertragung des Verbrauchsgutes. Bei einem entsprechenden Herstellerprüfwert kann es sich beispielsweise um einen öffentlichkryptografischen Schlüssel eines asymmetrischen Schlüsselpaares handeln. In diesem Fall dient beispielsweise eine Signatur mit einem privaten kryptografischen Schlüssel des entsprechenden Schlüsselpaares als Nachweis der Berechtigung, welcher mithilfe des Herstellerprüfwerts geprüft werden kann. Ferner kann es sich bei dem Herstellerprüfwert um eine Abwandlungen eines solchen öffentlichen kryptografischen Schlüssels handeln. Beispielsweise wird der Herstellerprüfwert durch ein- oder mehrmaligen Hashen des entsprechenden öffentlichen kryptografischen Schlüssels aus diesem berechnet.

Eine entsprechende Registrierungsanforderung eines Herstellers wird von einem Blockchain-Server des Blockchain-Netzwerks empfangen, durch Ausführung erster Programminstruktionen des Programmmoduls auf ihre Gültigkeit geprüft und falls die Prüfung positiv ausfällt, in die Blockchain eingetragen. Die Prüfung umfasst beispielsweise eine Prüfung, ob die entsprechende Registrierungsanforderung von einem autorisierten Hersteller stammt. In diese Prüfung kann beispielsweise anhand einer Signaturprüfung eine Signatur in der Registrierungsanforderung erfolgen. Ferner kann die Prüfung eine Prüfung des Herstellerprüfwerts umfassen, ob es sich bei dem entsprechenden Herstellerprüfwert um einen autorisierten Herstellerprüfwert handelt. Schließlich kann die Prüfung sich zudem inhaltlich auf die Registrierungsanforderung erstrecken, d. h. ob der Herstellerdatensatz vollständig ist.

Wird ein Block in die Blockchain eingetragen, welcher einen Prüfwert für ein Verbrauchsgut umfasst, so gilt beispielsweise stets der zuletzt in die Blockchain eingetragene Prüfwert als der aktuelle und damit für das entsprechende Verbrauchsgut gültige Prüfwert. Voraussetzung hierfür ist natürlich, dass die entsprechende Eintragung gültig ist, wobei die entsprechende Gültigkeit durch die Voraussetzungsprüfung im Zuge der Eintragung im Allgemeinen in ausreichendem Maße sichergestellt werden kann. Auf dem Vertriebsweg vom Hersteller zum Endverbraucher wechselt das entsprechende Verbrauchsgut mehrfach den Besitzer. Beispielsweise wird ein entsprechendes Verbrauchsgut hergestellt, wird auf dem Weg zu einem Großhändler an einen Lieferdienst übergeben, wechselt auf dem Weg zu einem Großhändler gegebenenfalls mehrfach den Lieferdienst bzw. wird in entsprechenden Verpackungsstationen und Logistiklagern zwischengelagert, wird an den Großhändler übergeben, wird auf dem Weg zum Händler an einen Lieferdienst übergeben, wird gegebenenfalls wieder zwischengelagert bzw. in Logistikzentren verteilt, wird an den Einzelhändler übergeben und von diesem schließlich an den Endverbraucher. Gegebenenfalls erfolgt auch die Übergabe an den Endverbraucher wiederum über einen Lieferdienst, etwa im Falle Verkaufs über das Internet.

All diese Besitzerwechsel auf dem Weg zum Endverbraucher können in der Blockchain in einfacher und effizienter Weise gespeichert werden.

Hierbei wird dem entsprechenden Verbrauchsgut bei jedem Besitzerwechsel ein neuer Prüfwert zugeordnet, welcher es dem neuen Besitzer ermöglicht, über das entsprechende Verbrauchsgut zu verfügen. Die Übertragungsberechtigung wird dabei von dem Vorbesitzer durch einen entsprechenden Berechtigungsnachweis nachgewiesen. Nach der Übertragung besitzt der Vorbesitzer keinerlei Verfügungsgewalt mehr über das entsprechende Verbrauchsgut, da seine Übertragungsberechtigung ihre Gültigkeit verliert. Mithin ist von nun an der neue Besitzer für das entsprechende Verbrauchsgut verantwortlich.

Die Übertragbarkeit des entsprechenden Verbrauchsguts endet mit der Endabgabe an den Endverbraucher. Im Zuge der Endabgabe wird anstatt eines neuen Prüfwerts ein Endabgabevermerk in die Blockchain eingetragen. Dieser Endabgabevermerk verhindert es, dass das entsprechende Verbrauchsgut weiter übertragen werden kann. Somit kann beispielsweise verhindert werden, dass ein Verbrauchsgut nach der Abgabe an einen Endverbraucher erneut in die Vertriebskette eingefügt werden kann. Insbesondere kann durch beispielsweise ein Austauschen des entsprechenden Verbrauchsguts gegen eine Fälschung verhindert werden.

Ausführungsformen können vorteilhaft sein, da sie es erlauben, für medizinische und pharmazeutische Verbrauchsgüter, insbesondere Betäubungsmittel, eine in sich geschlossenen endlichen Vertriebskette von Hersteller bis zum Patienten zu implementieren und manipulationssicher abzuspeichern. Hierbei kann die entsprechende Endabgabe sowohl in einer Apotheke als auch in einer medizinischen Einrichtung, wie etwa einer Praxis oder einem Krankenhaus erfolgen. Insbesondere im Fall von Betäubungsmitteln ermöglichen es Ausführungsformen für jedes einzelne Betäubungsmittel eine lückenlose Abgabekontrolle zu implementieren und dadurch Missbrauch vorzubeugen.

Unter einer "Blockchain" wird hier und im Folgenden eine geordnete Datenstruktur verstanden, welche eine Mehrzahl von miteinander verketteten Datenblöcken umfasst. Insbesondere wird unter einer Blockchain eine geordnete Datenstruktur verstanden, bei welcher jeder der Blöcke (außer dem ersten Block) einen Prüfwert, beispielsweise einen Hash-Wert, seines Vorgängerblocks umfasst und somit anhand jedes Blocks die Gültigkeit aller seiner Vorgängerblocks geprüft und ggf. bestätigt werden kann. Beispiele einer Blockchain vergleiche https://en.wikipedia.org/wiki/Block_chain_(database) und "Mastering Bitcoin", Chapter 7, The Blockchain, Seite 161 ff. Das Konzept der Blockchain wurde beispielsweise im Jahre 2008 in einem White Paper unter dem Pseudonym Satoshi Nakamoto zu Bitcoin beschrieben ("Bitcoin: Peer-to-Peer Electronic Cash System" (https://bitcoin.org/bitcoin.pdf)). Die darin beschriebene Blockchain besteht aus einer Reihe von Datenblöcken, in denen jeweils ein oder mehrere Einträge bzw.

Transaktionen zusammengefasst und mit einer Prüfsumme in Form eines Hashwerts versehen sind. Zusätzliche Blöcke der Blockchain werden beispielsweise in einem rechenintensiven Prozess erzeugt, der auch als sogenanntes Mining bezeichnet wird. Diese zusätzlich erzeugten Blöcke werden anschließend der Blockchain hinzugefügt und über ein Netzwerk an alle Teilnehmer, bzw. Knoten des Netzwerks, verbreitet.

Ausführungsformen können den Vorteil haben, dass die Blockchain durch die Speicherung kryptografischer Prüfsumme, d.h. Hashwerten, des vorangehenden Blocks im jeweils nachfolgenden Block ein hohes Maß an Sicherheit gegenüber nachträglichen Manipulationen bietet. Bei einer Blockchain werden die Einträge bzw. Transaktionen eines Blocks beispielsweise durch einen Merkle-Baum paarweise miteinander gehasht und nur der letzte auf diese Weise erhaltene Hashwert des Blocks, der sogenannte Root-Hashwert bzw. Wurzelhashwert, als Prüfsumme beispielsweise in einem Header des Blocks vermerkt. Das Verketten der Blöcke kann dann unter Verwendung dieser Root-Hashwerte überprüft werden. Jeder Block der Blockchain enthält in seinem Header beispielsweise den Hash des gesamten vorherigen Blockheaders. Somit wird die Reihenfolge der Blöcke eindeutig festgelegt und es entsteht eine Kettenstruktur. Durch die so implementierte Verkettung der einzelnen Blöcke miteinander wird erreicht, dass ein nachträgliches Modifizieren vorangegangener Blöcke bzw. einzelner Transaktionen praktisch ausgeschlossen ist, da hierfür die Hashwerte aller nachfolgenden Blöcke in kurzer Zeit ebenfalls neu berechnet werden müssten.

Der erste Block in der Blockchain ist vorgegeben und wird Genesisblock genannt. Nach Ausführungsformen sind die öffentlichen kryptografischen Schlüssel eines oder mehrerer Provider, welche zum Erstellen von Programmodulen berechtigt sind, in dem Genesisblock gespeichert. Der Genesisblock ist aufgrund der zuvor beschriebenen Kettenstruktur, derjenige Block, dessen Einträge das höchste Maß an Sicherheit aufweisen, da zu seiner Änderung die gesamte Blockchain durch eine neue Blockchain ersetzt werden müsste. Mithin kann der Eintrag des öffentlichen kryptografischen Schlüssels in den Genesisblock einen Vertrauensanker mit einem ausreichenden Maß an Sicherheit darstellen, sodass beispielsweise keine zusätzliche PKI-Prüfung notwendig ist, um der Authentizität des öffentlichen kryptografischen Schlüssels zu vertrauen.

Vielmehr kann die Blockchain ausgehend von öffentlichen Schlüsseln, welche beispielsweise als der Programmodule und/oder als Teil eines Genesisblocks in die Blockchain eingetragen werden als Root-Keys oder Wurzelschlüssel für ein oder mehrere PKIs dienen, welche durch die Blockchain implementiert werden. So können beispielsweise weitere öffentliche Schlüssel in die Blockchain eingetragen werden, wenn die zugrundeliegenden Eintragungs- bzw. Registrierungsanfragen mit einer Signatur versehen sind, deren Gültigkeit sich unter Verwendung eines bereits in der Blockchain gültig eingetragenen öffentlichen Schlüssel bestätigen lässt.

Nach Ausführungsformen kann eine Blockchain durch eine Anpassung der notwendigen Rechenintensität für die Erstellung jeweils zusätzlicher Blöcke die Sicherheit der Blockchain anpassen. Die für die Erstellung zusätzlicher Blöcke notwendige Rechenintensität lässt sich beispielsweise über Anforderungen an den Hashwert des zu erstellenden zusätzlichen Blocks steuern. Der resultierende Hash-Wert ist nicht vorhersagbar, vielmehr handelt es sich um eine zufallsverteilte Zahl. Es lässt sich aber berechnen, wieviel Zeit in Abhängigkeit von der aufgewendeten Rechenleistung im statistischen Mittel zum Auffinden eines gültigen zusätzlichen Blocks notwendig ist. Der Hashwert eines Blocks lässt sich beispielsweise durch Hinzufügen und Variieren eines Nounce variieren. Aufgrund der Kettenstruktur können Daten, die einmal in einer Blockchain gespeichert sind, nicht mehr geändert oder entfernt werden, ohne große Teile der Blockchain zu ersetzen. Eine solche Ersetzung scheidet jedoch als Folge einer ausreichend rechenintensiven Generierung zusätzlicher Blöcke aus. Bekannte Ausführungsformen einer Blockchain, wie etwa im Fall der Kryptowährung Bitcoin, basieren auf einer Anonymität der an den Transaktionen beteiligten Partner. Demgegenüber kann durch oben beschriebene Signatur der in die Transaktionen eingetragenen Hashwerte, deren Authentizität belegt und ihr Ursprung nachgewiesen werden. Hierdurch kann die Fälschungssicherheit verbessert werden.

Eine Anforderung an einen gültigen Block kann beispielsweise darin bestehen, dass der Hashwert des Headers des Blocks kleiner gleich einem Grenzwert ist. Die Hashwertberechnung kann beispielsweise mit dem Secure Hash Algorithm (SHA) SHA 256 erfolgen. Der resultierende Hash-Wert ist in diesem Fall eine Zufallszahl zwischen 0 und 2²⁵⁶-1. Die Wahrscheinlichkeit, dass beim Anwenden des Hashalgorithmus einen bestimmten Hash herauskommt, ist somit (maximaler Hash-Wert+1)⁻¹, im Fall des SHA 256-Algorithums also 2⁻²⁵⁶. Die Wahrscheinlichkeit, dass der resultierende Hash-Wert kleiner gleich einem Grenzwert bzw. Zielwert (engl. target) ist, beträgt daher (target)/(max. Hash-Wert). Für einen beispielhaften maximalen Grenzwert von (2¹⁶-1)·2²⁰⁸ beträgt die Wahrscheinlichkeit [(2¹⁶-1)·2²⁰⁸]/ 2²⁵⁶ ≈ 2⁻³². Die Schwierigkeit S eine Hash-Wert zu erhalten, welcher kleiner gleich einem gewählten Grenzwert bzw. target ist, kann in Abhängigkeit eines maximalen Grenzwerts bzw. max. target wie folgt angegeben werden: S = (max. target)/target. Mithin ist die Wahrscheinlichkeit einen Hash-Wert zu erhalten, welcher kleiner gleich dem gewählten Grenzwert ist, für das zuvor gegebene Beispiel: 2⁻³²/S. Als Beispiel sei ein Computersystem mit einer bestimmten Hashrate betrachtet, welches im Durchschnitt alle x·Sek. einen Hash-Wert findet, welcher kleiner gleich dem gewählten Grenzwert ist. Soll das Computersystem anstelle aller x·Sek. im Durchschnitt alle y·Sek. einen Treffer erzielen, so kann die Schwierigkeit entsprechend angepasst werden: Sy=(x/y)·S. Entsprechende Anpassungen der Schwierigkeit können auch dazu verwendet werden die Trefferrate bei Veränderungen des Computersystems, z.B. Veränderungen der Rechenleistung durch Erhöhen oder Verringern der Anzahl an Blockchain-Servern, konstant zu halten. Wird die Schwierigkeit so angepasst, dass alle y·Sek. ein Treffer erzielt wird, kann die Hashrate R des Computersystems wie folgt parametrisiert werden: R = (2³²·S)/(y·Sek.).

Werden gültige Blöcke durch ein rechenintensive Verfahren, wie das zuvor Beschriebene erzeugt, so vertrauen die Teilnehmer des Blockchain-Netzwerks der längsten gültigen Blockchain, da hinter dieser die meiste Rechenleistung steht und somit angenommen werden kann, dass diese von der Mehrheit der Teilnehmer als gültig anerkannt wird. Kommt es beispielsweise dazu, dass ein Fork, d.h. eine Verzweigung, in der Blockchain entsteht, setzt sich irgendwann der Fork mit der größeren Kettenlänge durch, da anzunehmen ist, dass hinter diesem die Mehrheit der Teilnehmer steht.

Eine Blockchain kann beispielsweise auch in Form einer privaten, d.h. zugangsbeschränkten, Blockchain implementiert werden, wobei nur eine ausgewählte Gruppe von Teilnehmern eine Berechtigung zum Hinzufügen gültiger Blöcke und/oder zum Lesen von Blöcken besitzt. Eine entsprechende Berechtigung kann beispielsweise mittels einer Signatur unter Verwendung eines privaten kryptografischen Schlüssels nachgewiesen werden. Der private kryptografische Schlüssel kann zu einem asymmetrischen Schlüsselpaar gehören, zu welchem auch ein öffentlicher kryptografischer Schlüssel gehört, mit dem die Signatur geprüft werden kann. Dem asymmetrischen Schlüsselpaar kann zudem beispielsweise ein Zertifikat zugeordnet sein, welches die Berechtigung zum Erzeugen eines gültigen Blocks der Blockchain belegt. Dieses Zertifikat kann ferner einer PKI zugeordnet sein, welche die Authentizität des Zertifikats belegt. Nach einer weiteren Ausführungsform kann beispielsweise für jeden Teilnehmer aus der ausgewählte Gruppe ein öffentlicher Schlüssel in der Blockchain hinterlegt sein, beispielsweise in einem Genesisblock. Anhand dieser öffentlichen Schlüssel kann geprüft werden, ob Signaturen von Blöcken und damit die entsprechenden Blöcke selbst gültig sind. Mit anderen Worten kann die Blockchain in diesem Fall selbst eine eigenständige PKI bereitstellen.

Ein Konsens über eine Gültigkeit eines zusätzlichen Blocks für die Blockchain bzw. über ein Hinzufügen eines zusätzlichen Blocks zu der Blockchain kann auch auf andere Weise in einer Blockchain implementiert werden. So kann etwa ein Konsens erreicht werden, indem über eine Aufnahme vorgeschlagener Einträge in die Blockchain abgestimmt wird. Beispielsweise führt jeder Teilnehmer eine eindeutige Liste anderer Teilnehmer, welchen er als Gruppe vertraut. Bei diesen Teilnehmern kann es sich beispielsweise um Blockchain-Server handeln. Jeder Teilnehmer kann zusätzliche Einträge vorschlagen, die in einen zusätzlichen Block der Blockchain aufgenommen werden sollen. Über die Aufnahme und damit die Anerkennung der Gültigkeit der vorgeschlagenen Einträge wird abgestimmt. So stimmt beispielsweise jeder Teilnehmer nur über diejenigen Vorschläge ab, welche von Teilnehmer seiner Liste stammen. Mit anderen Worten werden für die Entscheidung, ob ein Vorschlag für einen zusätzlichen Eintrag als gültig anerkannt wird, d.h. ob bezüglich der Gültigkeit dieses Eintrages ein Konsens zwischen den Teilnehmern besteht, nur die Stimmen derjenigen Teilnehmer berücksichtig, die von der Liste desjenigen Teilnehmers umfasst sind, der den entsprechenden Vorschlag macht. Damit ein Vorschlag für einen Eintrag als gültig angenommen wird, muss ein bestimmter Minimumanteil an stimmberechtigten Teilnehmern mit Ja stimmen, beispielsweise 80%. Alle vorgeschlagenen Einträge, die dieses Kriterium erfüllen werden in die Blockchain aufgenommen. Eine solche Abstimmung kann mehrere Runden umfassen. Alle anderen Vorschläge, die das zuvor genannte Kriterium nicht erfüllen, werden verworfen oder bei der Abstimmung über den nächsten Block der Blockchain erneut zur Abstimmung gestellt. Die zuvor genannten Listen stellen Untergruppen des Blockchain-Netzwerks dar, denen der Teilnehmer, welcher die jeweilige Liste führt, als Gruppe insgesamt traut, ohne dass dies erfordert, dass er jedem einzelnen Teilnehmer der Liste traut. Ein Beispiel für ein solches Konsensverfahren bietet der Ripple Protokoll Konsens Algorithmus (David Schwartz et al.: "The Ripple Protocol Consensus Algorithm", Ripple Labs Inc., 2014,
https://ripple.com/files/ripple_consensus_whitepaper.pdf).

Beispielsweise kann es sich bei der Blockchain um eine private oder öffentliche Blockchain handeln. Beispielsweise handelt es sich um eine Bitcoin-, Litecoin- oder Ethereum-Blockchain.

Ein "Programmmodul" bezeichnet hier ein eigenständiges Programm, welches in einer Blockchain gespeichert ist. Das Programmmodul kann dazu konfiguriert sein das Erstellen von dem Programmmodul zugeordneten Einträgen in der Blockchain zu steuern. Das Programmodul kann in einem Block der Blockchain oder über mehrere Blöcke der Blockchain verteilt gespeichert sein. Jeder Urkundenspezifikation ist beispielsweise ein individuelles Programmmodul zugeordnet. Bei einem Programmmodul kann es sich beispielsweise um einen "smart contract" handeln, wie er beispielsweise in der Open Source Ethereum Blockchain implementierbar ist. Unter einem "Programm" bzw. "Programminstruktionen" wird hier ohne Einschränkung jede Art von Computerprogramm verstanden, welches maschinenlesbare Instruktionen zur Steuerung einer Funktionalität des Computers umfasst.

Unter einem "Zertifikat" wird hier ein digitales Zertifikat verstanden, welches auch als Public-Key-Zertifikat bezeichnet wird. Durch solche Zertifikate basierend auf asymmetrischen Schlüsselpaaren wird eine so genannte Public Key Infrastructure (PKI) realisiert. Bei einem solchen Zertifikat handelt es sich um strukturierte Daten, die dazu dienen, einen öffentlichen Schlüssel eines asymmetrischen Kryptosystems einer Identität, wie zum Beispiel einer Person oder einer Vorrichtung, zuzuordnen. Ein Zertifikat kann beispielsweise einen öffentlichen Schlüssel beinhalten und signiert sein. Alternativ sind auch Zertifikate basierend auf zero-knowledge Kryptosystemen möglich. Beispielsweise kann das Zertifikat dem Standard X.509 oder einem anderen Standard entsprechen. Beispielsweise handelt es sich bei dem Zertifikat um ein CV-Zertifikat oder auch Card Verifiable Certificate (CVC). Eine Implementierung von solchen CVCs ist beispielsweise in der ISO/IEC 7816-8 spezifiziert.

Die PKI stellt ein System zum Ausstellen, Verteilen und Prüfen digitaler Zertifikate. Ein digitales Zertifikat dient in einem asymmetrischen Kryptosystem dazu die Authentizität eines öffentlichen Schlüssels und seinen zulässigen Anwendungs- und Geltungsbereich zu bestätigen. Eine solche Bestätigung kann auch über einen Eintrag in der Blockchain erfolgen, in welchem Fall diese Einträge als Zertifikate zu verstehen sind. Das digitale Zertifikat ist selbst durch eine digitale Signatur geschützt, deren Echtheit mit dem öffentlichen Schlüssel des Ausstellers des Zertifikates geprüft werden kann. Um die Authentizität des Ausstellerschlüssels zu prüfen, wird wiederum ein digitales Zertifikat verwendet. Auf diese Weise lässt sich eine Kette von digitalen Zertifikaten aufbauen, die jeweils die Authentizität des öffentlichen Schlüssels bestätigen, mit dem das vorhergehende Zertifikat geprüft werden kann. Eine solche Kette von Zertifikaten bildet einen sogenannten Validierungspfad oder Zertifizierungspfad. Auf die Echtheit des letzten Zertifikats, des sogenannten Wurzelzertifikats, und des durch dieses Zertifikat zertifizierten Schlüssels, müssen sich die Teilnehmer der PKI ohne ein weiteres Zertifikat verlassen können. Das Wurzelzertifikat wird von einer sogenannten Wurzelzertifizierungsinstanz verwaltet, auf deren als gesichert vorausgesetzten Authentizität die Authentizität aller Zertifikate der PKI zurückgeht.

Ein Zertifikat kann einer elektronischen Signatur zugeordnet sein, wenn der zu dem öffentlichen Schlüssel gehörende private Schlüssel zur Generierung der zu prüfenden elektronischen Signatur verwendet wurde. Dadurch, dass ein Zertifikat in Assoziation mit einem öffentlichen Schlüssel der Allgemeinheit zur Verfügung gestellt wird, wird es Nutzern asymmetrischer Kryptosysteme ermöglicht den öffentlichen Schlüssel einer Identität, beispielsweise einer Person, einer Organisation, oder einem Computersystem, zuzuordnen.

Asymmetrische Schlüsselpaare werden für eine Vielzahl von Kryptosystemen eingesetzt und spielen auch bei der Signatur elektronischer Dokumente eine wichtige Rolle. Ein asymmetrisches Schlüsselpaar besteht aus einem öffentlichen Schlüssel, welcher zur Ver- und/oder Entschlüsselung von Daten verwendet wird und an Dritte, beispielsweise an einen Dienstanbieter, weitergegeben werden darf sowie einem privaten Schlüssel, welcher zur Ver- und/oder Entschlüsselung von Daten verwendet wird und im Regelfall geheim gehalten werden muss. Der öffentliche Schlüssel ermöglicht es jedermann, Daten für den Inhaber des privaten Schlüssels zu verschlüsseln, digitale Signaturen von dessen Dokumenten zu prüfen oder ihn zu authentifizieren. Ein privater Schlüssel ermöglicht es seinem Inhaber, mit dem öffentlichen Schlüssel verschlüsselte Daten zu entschlüsseln oder digitale Signaturen für elektronische Dokumente zu erstellen. Eine mit einem privaten Schlüssel erstellte Signatur kann mit dem zugehörigen öffentlichen Schlüssel verifiziert werden.

Digitale Signaturen werden zum sicheren elektronischen Datenaustausch, beispielsweise im Internet, eingesetzt und ermöglichen die Prüfung von Identitäten und/oder Berechtigungen und der Unverfälschtheit der ausgetauschten Daten. Um dies zu gewährleisten, ist in der Regel eine Public-Key-Infrastruktur notwendig, die die Gültigkeit der verwendeten Schlüssel durch Zertifikate bestätigt.

Die Erstellung einer digitalen Signatur, im Folgenden auch lediglich als "Signatur" bezeichnet, ist ein kryptografisches Verfahren, bei dem zu beliebigen Daten, zum Beispiel einem elektronischen Dokument, ein weiterer Datenwert, welcher als "Signatur" bezeichnet wird, berechnet wird. Die Signatur kann zum Beispiel ein verschlüsselter Hashwert des elektronischen Dokumentes sein, insbesondere ein mit einem privaten Schlüssel eines einem Zertifikat zugeordneten kryptografischen Schlüsselpaares verschlüsselter Hashwert. Ein entsprechendes Verschlüsseln eines Hashwerts wird mithin als Signieren des Hashwerts bezeichnet. Die Besonderheit einer solchen Signatur besteht darin, dass deren Urheberschaft und Zugehörigkeit zu einer bestimmten Person oder Instanz durch jeden Dritten geprüft werden kann.

Unter einer digitalen Signatur wird hier auch ein digitales Siegel verstanden, welches nicht einer natürlichen Person, sondern einer juristischen Person zugeordnet ist. Ein digitales Siegel dient somit nicht der Abgabe einer Willenserklärung einer einzelnen Person, sondern einer Institution als Herkunftsnachweis. Es kann somit den Ursprung und die Unversehrtheit digitaler Dokumente sicherstellen und nachweisen, dass diese von einer bestimmten juristischen Person stammen.

Unter einem "Speicher" werden hier sowohl flüchtige als auch nicht flüchtige Speicher, insbesondere elektronische Speicher bzw. digitale Speichermedien verstanden.

Unter einem "nichtflüchtigen Speicher" wird hier ein elektronischer Speicher zur dauerhaften Speicherung von Daten verstanden. Ein nichtflüchtiger Speicher kann als nichtänderbarere Speicher konfiguriert sein, der auch als Read-Only Memory (ROM) bezeichnet wird, oder als änderbarer Speicher, der auch als Non-Volatile Memory (NVM) bezeichnet wird. Insbesondere kann es sich hierbei um ein EEPROM, beispielsweise ein Flash-EEPROM, kurz als Flash bezeichnet, handeln. Ein nichtflüchtiger Speicher zeichnet sich dadurch aus, dass die darauf gespeicherten Daten auch nach Abschalten der Energieversorgung erhalten bleiben.

Unter einem "flüchtigen elektronischen Speicher" wird hier ein Speicher zur vorübergehenden Speicherung von Daten, welcher dadurch gekennzeichnet ist, dass alle Daten nach dem Abschalten der Energieversorgung verloren gehe. Insbesondere kann es sich hierbei um einen flüchtigen Direktzugriffsspeicher, der auch als Random-Access Memory (RAM) bezeichnet wird, oder einen flüchtigen Arbeitsspeicher des Prozessors handeln.

Unter einem "geschützten Speicherbereich" wird hier ein Bereich eines elektronischen Speichers verstanden, auf den ein Zugriff, das heißt ein Lesezugriff oder ein Schreibzugriff, nur über einen Prozessor des entsprechenden elektronischen Geräts möglich ist. Nach Ausführungsformen ist der Zugriff von dem mit dem Speicher gekoppelten Prozessor nur dann möglich, wenn eine hierzu erforderliche Bedingung erfüllt ist. Hierbei kann es sich zum Beispiel um eine kryptografische Bedingung, insbesondere eine erfolgreiche Authentisierung und/oder eine erfolgreiche Berechtigungsprüfung, handeln.

Unter einem "Prozessor" wird hier und im Folgenden eine Logikschaltung verstanden, die zur Ausführung von Programminstruktionen dient. Die Logikschaltung kann auf einem oder mehreren diskreten Bauelementen implementiert sein, insbesondere auf einem Chip. Insbesondere wird unter einem "Prozessor" ein Mikroprozessor oder ein Mikroprozessorsystem aus mehreren Prozessorkernen und/oder mehreren Mikroprozessoren verstanden.

Unter einer "Kommunikationsschnittstelle" wird hier eine Schnittstelle verstanden, über die Daten empfangen und gesendet werden können, wobei die Kommunikationsschnittstelle kontaktbehaftet oder kontaktlos konfiguriert sein kann. Bei der Kommunikationsschnittstelle kann es sich um eine interne Schnittstelle oder um eine externe Schnittstelle handeln, welche beispielsweise mittels eines Kabels oder kabellos mit einem zugeordneten Gerät verbunden ist.

Eine Kommunikation kann beispielsweise über ein Netzwerk erfolgen. Unter einem "Netzwerk" wird hier jedes Übertragungsmedium mit einer Anbindung zur Kommunikation verstanden, insbesondere eine lokale Verbindung oder ein lokales Netzwerk, insbesondere ein Local Area Network (LAN), ein privates Netzwerk, insbesondere ein Intranet, und ein virtuelles privates Netzwerk (Virtual Private Network - VPN). Beispielsweise kann ein Computersystem eine Standardfunkschnittstelle zur Anbindung an ein WLAN aufweisen. Ferner kann es sich um ein öffentliches Netzwerk, wie beispielsweise das Internet handeln. Je nach Ausführungsform kann diese Verbindung auch über ein Mobilfunknetz hergestellt werden.

Unter einem "Mobilfunknetzwerk" wird hier und im Folgenden ein digitales zellulares Mobilfunknetzwerk verstanden, welches nach einem Mobilfunkstandard wie zum Beispiel GSM, UMTS, LTE, CDMA oder einem anderen Standard aufgebaut sein kann.

Nach Ausführungsformen umfasst das Verfahren ferner:
- im Fall einer Vernichtung eines zweiten in der Blockchain registrierten Verbrauchsgutes, Empfangen einer Vernichtungsbestätigungsanforderung zur Bestätigung der Vernichtung des zweiten Verbrauchsgutes durch einen der Blockchain-Server des Blockchain-Netzwerks, wobei die Vernichtungsbestätigungsanforderung den Identifikator des zweiten Verbrauchsgutes und einen Berechtigungsnachweis des aktuellen Besitzers vor der Vernichtung zur Übertragung des zweiten Verbrauchsgutes umfasst,
- Ausführen vierter Programminstruktionen des Programmoduls durch den die Vernichtungsbestätigungsanforderung empfangenden Blockchain-Server, wobei das Ausführen der vierten Programminstruktionen eine Prüfung unter Verwendung des aktuellen Prüfwerts umfasst, ob die Vernichtungsbestätigungsanforderung des aktuellen Besitzers ein oder mehrere vierte Voraussetzungen erfüllt, und, falls die vierten Voraussetzungen erfüllt sind, ein Hinzufügen des Identifikators des zweiten Verbrauchsgutes und eines Vernichtungsvermerks zu einem Eintrag in einem zusätzlichen Block der Blockchain, wodurch der Vernichtungsvermerks dem zweiten Verbrauchsgut zugeordnet wird, wobei der Vernichtungsvermerk die Vernichtung des zweiten Verbrauchsgutes bestätigt und festlegt, dass das zweite Verbrauchsgut von weiteren Übertragungen ausgeschlossen ist.

Ausführungsformen können den Vorteil haben, dass sie auch im Fall einer Vernichtung des entsprechenden Verbrauchsguts eine lückenlose Kontrolle in der Historie des entsprechenden Verbrauchsguts ermöglichen. Wird ein solches Verbrauchsgut, beispielsweise aufgrund eines Ablaufs eines Verfalldatums vernichtet, anstatt an einen Endverbraucher, wie etwa im Fall eines Betäubungsmittels ein Patient, abgegeben werden, so wird auch dies in der Blockchain erfasst. Somit kann sichergestellt werden, dass jedes in der Blockchain protokollierte Verbrauchsgut letztendlich entweder seiner bestimmungsgemäßen Verwendung, d. h. einer Abgabe an einen Endverbraucher, zugeführt wird oder aber vernichtet wird.

Als Identifikator der Verbrauchsgüter in der Blockchain können entweder den einzelnen Verbrauchsgütern zugeordnete individuelle Identifikatoren dienen, welche zusätzlich zu den Prüfwerten in der Blockchain erfasst werden, oder es kann hierzu der Herstellerprüfwert dienen, falls dieser für jedes der Verbrauchsgüter individuell vergeben wird. Beispielsweise umfasst jeder Eintrag in die Blockchain, bei welchem, welcher unter Verwendung zu einem bestimmten Verbrauchsgut hin die Blockchain, welcher auf den Herstellungseintrag in die Blockchain, welcher auf einen Registrierungseintrag eines bestimmten Verbrauchsguts folgt und sich auf dasselbe Verbrauchsgut bezieht, neben einem verbrauchsgutindividuellen Prüfwert des als neuen aktueller Besitzer einzutragenden Besitzers einen verbrauchsgutindividuellen Prüfwert des Vorbesitzers. Somit ergibt sich eine Kette von verbrauchsgutindividuellen Prüfwerten, anhand derer alle Einträge in der Blockchain identifiziert werden können, welche sich auf dieses konkrete Verbrauchsgut beziehen. Wird ein Prüfwert in die Blockchain eingetragen, so beziehen sich alle weiteren Einträge, welche denselben verbrauchsgutindividuellen Prüfwert referenzieren, auf dasselbe Verbrauchsgut.

Nach Ausführungsformen ist den in der Blockchain registrierten Verbrauchsgütern jeweils maximal ein aktueller Prüfwert zugeordnet, bei welchem es sich jeweils um den dem entsprechenden Verbrauchsgut zuletzt zugordneten Prüfwert handelt. Ausführungsformen können den Vorteil haben, dass für jedes Verbrauchsgut eindeutig festgelegt wird, wer in dessen Besitz ist und für die Handhabung des entsprechenden Verbrauchsguts verantwortlich ist. Dabei ist der Besitzer der Einzige, welcher eine Berechtigung zur Verfügung über das entsprechende Verbrauchsgut hat, womit die Möglichkeiten einer Manipulation, etwa durch Einschleusen eines gefälschten Verbrauchsguts oder eine unberechtigte Entnahme eines Verbrauchsguts aus der Vertriebskette, effektiv verhindert werden kann.

Nach Ausführungsformen sind die Herstellerprüfwerte der in der Blockchain registrierten Verbrauchsgüter eindeutig für die einzelnen Verbrauchsgüter und werden jeweils als Identifikator der entsprechenden Verbrauchsgüter verwendet.

Ausführungsformen können den Vorteil haben, dass kein zusätzlicher Identifikator für das Verbrauchsgut in die Blockchain eingeführt werden muss. Vielmehr gibt der Hersteller einen Prüfwert, welcher individuell für einzelne Verbrauchsgüter vergeben wird als Identifikator des entsprechenden Verbrauchsguts. So kann, wie zuvor erläutert, eine eindeutige Referenzsicherungskette implementiert, wobei die weiteren Prüfwerte jeweils verbrauchsgutindividuelle Prüfwerte sind, welche jeweils in Kombination mit dem verbrauchsgutindividuellen Prüfwert des Vorbesitzers eingetragen werden, so dass sich eine eindeutige Kettenverbindung innerhalb der Blockchain ergibt, anhand derer alle Einträge identifiziert werden können, welche sich auf das selbe Verbrauchsgut beziehen.

Nach Ausführungsformen wird ein Herstellerprüfwert für mehrere der in der Blockchain registrierten Verbrauchsgüter verwendet und die individuellen Identifikatoren der in der Blockchain registrierten Verbrauchsgüter sind unabhängig von den Herstellerprüfwerten.

Ausführungsformen können den Vorteil haben, dass anhand des Herstellerprüfwertes der Hersteller identifiziert werden kann. Zur Identifikation des Verbrauchsgutes umfasst in diesem Fall beispielsweise jeder dem entsprechenden Verbrauchsgut zugeordneter Eintrag in die Blockchain einen zusätzlichen verbrauchsgutindividuellen Identifikator. Sollen alle zu einem individuellen Verbrauchsgut gehörenden Einträge in der Blockchain identifiziert werden, so muss lediglich nach all denjenigen Einträgen gesucht werden, den entsprechenden individuellen Identifikator aufweisen.

Nach Ausführungsformen sind die Herstellerprüfwerte jeweils aus einem herstellerindividuellen Seed Key abgeleitet und unter Verwendung des herstellerindividuellen Seed Keys auf ihre Gültigkeit überprüfbar.

Ausführungsformen können den Vorteil haben, dass anhand des Seed Keys festgestellt werden kann, ob es sich bei einem Herstellerprüfwert um einen gültigen Herstellerprüfwert handelt. Somit kann beispielsweise der Herstellerprüfwert als Nachweis für eine Eintragungsberechtigung des Herstellers dienen. Ebenso können nach Ausführungsformen auch die Prüfwerte der weiteren Besitzer jeweils aus einem besitzerindividuellen Seed Key abgeleitet sein und sich anhand des entsprechenden besitzerindividuellen Seed Key auf ihre Gültigkeit prüfbar sein.

Nach Ausführungsformen handelt es sich bei den Prüfwerten jeweils um verbrauchsgüterindividuelle Prüfwerte.

Ausführungsformen können den Vorteil haben, dass anhand der verbrauchsgutindividuellen Prüfwerte eine verbrauchsgutindividuelle Prüfwertkette ermittelt werden kann, anhand derer alle Einträge in der Blockchain ermittelt werden können, welche sich auf das entsprechende Verbrauchsgut beziehen.

Nach Ausführungsformen wird ein Prüfwert für mehrere Verbrauchsgüter verwendet. Ausführungsformen können den Vorteil haben, dass die Prüfwerte als Identifikatoren den entsprechenden Besitzer dienen können, während die Verbrauchsgüter anhand eines unabhängigen verbrauchsgutindividuellen Identifikators zu identifizieren sind.

Nach Ausführungsformen sind die Prüfwerte jeweils aus einem besitzerindividuellen Seed Key abgeleitet und unter Verwendung des besitzerindividuellen Seed Keys auf ihre Gültigkeit überprüfbar. Ausführungsformen können den Vorteil haben, dass anhand des Seed Keys festgestellt werden kann, ob es sich bei einem Prüfwert um einen gültigen Prüfwert handelt.

Nach Ausführungsformen umfassen die Herstellungsdaten des ersten Verbrauchsgutes ein oder mehrere der folgenden Angaben: eine Bezeichnung des ersten Verbrauchsgutes, eine Bezeichnung des Hersteller des ersten Verbrauchsguts, eine Artikelnummer des ersten Verbrauchsguts, einen Status des ersten Verbrauchsguts, ein Verfalldatum des ersten Verbrauchsguts.

Ausführungsformen können den Vorteil haben, dass die individuellen Verbrauchsgüter anhand der Herstellungsdaten vollständig identifiziert werden können und die Herstellungsdaten alle für das entsprechende Verbrauchsgut relevanten Ausgangsdaten definiert. Beispielsweise kann anhand eines Verfalldatums ermittelt werden, ob das entsprechende Verbrauchsgut vor einer Abgabe an einen Endverbraucher zu vernichten ist.

Nach Ausführungsformen umfasst die Endabgabebestätigungsanforderung einen Identifikator des Endverbrauchs. Das Ausführen der dritten Programminstruktionen des Programmoduls umfasst, falls die dritten Voraussetzungen erfüllt sind, in Ergänzung zu dem Identifikator des ersten Verbrauchsgutes und dem Endabgabevermerk ein Hinzufügen des Identifikator des Endverbrauchs zu dem entsprechenden Eintrag in dem entsprechenden zusätzlichen Block der Blockchain, wodurch der Identifikator des ersten Verbrauchsgutes dem Identifikator des Endverbrauchs zugeordnet wird.

Ausführungsformen können den Vorteil haben, dass eindeutig nachvollzogen werden kann, an welchen Endverbraucher das Verbrauchsgut abgegeben wurde. Beispielsweise kann somit sichergestellt werden, dass ein individueller Endverbraucher nur eine begrenzte Menge an Verbrauchsgütern, beispielsweise innerhalb eines begrenzten Zeitraums erhält, und somit Missbrauch effektiv vorgebeugt werden kann. Dabei kann der Identifikator den Endverbraucher eindeutig für jedermann identifizieren oder der Identifikator kann anonymisiert sein, so dass nur mit Zusatzwissen der entsprechende Endverbraucher identifiziert werden kann. Beispielsweise kann der Identifikator verschlüsselt sein. So lange bei jeder Endabgabe an denselben Endverbraucher derselbe Identifikator zum Einsatz kommt, kann selbst ohne Entschlüsselung des Identifikators effektiv und effizient erkannt werden, wenn ein möglicher Missbrauch vorliegt. Nach alternativen Ausführungsformen kann der Identifikator des Endverbrauchers soweit anonymisiert sein, dass bei jeder Endabgabe ein anderer Identifikator für denselben Endverbraucher verwendet wird und lediglich mit Wissen, welche Identifikatoren welchem Endverbraucher zugeordnet sind, auf die tatsächlichen Endverbraucher geschlossen werden kann.

Nach Ausführungsformen umfasst die Vernichtungsbestätigungsanforderung Identifikatoren von ein oder mehreren Zeugen der Vernichtung des zweiten Verbrauchsgutes. Das Ausführen der vierten Programminstruktionen des Programmoduls umfasst, falls die vierten Voraussetzungen erfüllt sind, in Ergänzung zu dem Identifikator des zweiten Verbrauchsgutes und dem Vernichtungsvermerks ein Hinzufügen der Identifikatoren der ein oder mehreren Zeugen zu dem entsprechenden Eintrag in dem entsprechenden zusätzlichen Block der Blockchain, wodurch die Identifikatoren der ein oder mehreren Zeugen dem Vernichtungsvermerk des zweiten Verbrauchsgutes zugeordnet werden.

Ausführungsformen können den Vorteil haben, dass durch einen Eintrag von Identifikatoren, welche Zeugen der Vernichtung eines entsprechenden Verbrauchsguts bezeugen, sichergestellt werden kann, dass die eingetragene Vernichtung auch tatsächlich stattgefunden hat.

Nach Ausführungsformen ist eine der ersten Voraussetzungen eine gültige Signatur des Herstellerdatensatzes. Das Ausführen der ersten Programminstruktionen umfasst das Prüfen der Signatur des Herstellerdatensatzes unter Verwendung eines ersten öffentlichen kryptografischen Schlüssel des Herstellers des ersten Verbrauchsgutes. Ausführungsformen können den Vorteil haben, dass anhand der Signatur des Herstellerdatensatzes sichergestellt werden kann, dass nur berechtigte Hersteller Verbrauchsgüter in der Blockchain registrieren.

Nach Ausführungsformen wird der erste öffentliche kryptografische Schlüssel des Herstellers zur Prüfung der Signatur des Herstellerdatensatzes von der Blockchain zur Verfügung gestellt. Ausführungsformen können den Vorteil haben, dass eine Prüfung der Signatur allein unter Verwendung der von der Blockchain bereitgestellten Daten erfolgen kann. Mithin stellt die Blockchain beispielsweise eine eigenständige PKI zur Prüfung von Signaturen bereit.

Nach Ausführungsformen ist jedes in der Blockchain registrierte Verbrauchsgut mit einem maschinenlesbaren Code versehen, wobei der maschinenlesbare Code einen Identifikator des entsprechenden Verbrauchsguts umfasst.

Bei dem maschinenlesbaren Code kann es sich beispielsweise um einen ein- oder zweidimensionalen graphischen Code handeln, welcher mittels eines optoelektronischen Lesegeräts lesbar ist. Der Code kann gängige Schrift-, Zahl- und/oder Sonderzeichen umfassen. ferner kann der Code geometrische Formen, wie etwa Striche und Punkte unterschiedlicher Dimensionierung umfassen. Bei dem Code handelt es sich beispielsweise um einen eindimensionalen Strichcode, einen gestapelten eindimensionalen Strichcode, einen Array-Code, wie etwa einen Matrix-Code, z.B. einen QR-Code, oder einen Punktcode. Ferner kann es sich um einen zusammengesetzten Code (Composite-Codes) wie etwa einen Doppelcode handeln. Ein Doppelcode setzt sich beispielsweise zusammen aus einem linearen Barcode und einem zusätzlichen zweidimensionalen Code. Ferner kann es sich um einen eindimensionalen Barcode, welcher zusätzlich die zweite Dimension in Form von unterschiedlich langen Strichen zur Codierung nutzt. Auch höherdimensionale Codes mit drei oder mehr Dimensionen können implementiert werden, etwa über eine Verwendung von unterschiedlichen Farben.

Bei dem maschinenlesbaren Code kann es sich ferner um in einem Speicher, etwa eines Chips, oder ein RFID-Tags, gespeicherte Daten handeln, welche mit einem Lesegerät ausgelesen werden können.

Ausführungsformen können den Vorteil haben, dass für ein einzelnes Verbrauchsgut anhand des maschinenlesbaren Codes dessen Identität automatisch erfasst und mit der Blockchain abgeglichen werden kann. Bei dem entsprechenden maschinenlesbaren Code handelt es sich beispielsweise um einen auf dem Verbrauchsgut und/oder auf einer Verpackung des Verbrauchsgutes angebrachten Beschriftung, etwa in Form eines QR-Codes oder Barcodes, welcher maschinell erfasst werden kann. Alternativerweise kann es sich bei dem maschinenlesbaren Code auch um einen Code handeln, welcher von einem auslesbaren elektronischen Speichermedium bereitgestellt wird, z. B. einem RFID-Tag, welcher mit einem entsprechenden Lesegerät ausgelesen werden kann.

Nach Ausführungsformen dient der Identifikator zudem als Berechtigungsnachweis zum Auslesen von Daten aus der Blockchain. Empfängt beispielsweise ein Blockchain-Server eine Leseanfrage, welche eine gültigen Identifikator eines Verbrauchsgutes angibt, so überträgt der entsprechende Blockchain-Server die angefragten Daten an den Sender der Anfrage. Nach Ausführungsformen kann die Zugriffsberechtigung auf Basis des Identifikators des entsprechenden Verbrauchs beschränkt sein. Beispielsweise kann die Auskunft lediglich eine Vollständigkeit der Protokollierung in der Lieferkette bestätigen. Nach alternativen Ausführungsformen werden selektive Daten aus den Einträgen in der Blockchain zur Verfügung gestellt. Beispielsweise wird kein Identifikator des Endverbrauchers bereitgestellt oder dieser wird lediglich in anonymisierter Form bereitgestellt. Nach Ausführungsformen wird der Lesezugriff auf alle dem entsprechen Verbrauchsgut zugeordneten Einträge gewährt.

Nach Ausführungsformen umfasst das Verfahren ferner:
- im Fall eines Verpackens einer Gruppe von in der Blockchain registrierten Verbrauchsgütern in einem gemeinsamen Verpackungsbehältnis, Empfangen einer Bündelungsbestätigungsanforderung zur Bestätigung einer Bündelung von Berechtigungen zur Übertragung der Verbrauchsgütern der Gruppe von Verbrauchsgütern, wobei die Bündelungsbestätigungsanforderung einen Identifikatoren jedes Verbrauchsgüter der Gruppe von Verbrauchsgütern, einen Berechtigungsnachweis des aktuellen Besitzers jedes Verbrauchsgüter der Gruppe von Verbrauchsgütern vor der Verpackung zur Übertragung des entsprechenden Verbrauchsgutes, einen Gruppenidentifikator und einen Gruppenerstellerprüfwert umfasst, wobei der Gruppenerstellerprüfwert zur Prüfung einer Berechtigung zur Übertragung der Verbrauchsgüter der Gruppe von Verbrauchsgütern zusammen als Gruppe durch einen Gruppenersteller als einem aktuellen Gruppenbesitzer der Gruppe von Verbrauchsgütern dient,
- Ausführen fünfter Programminstruktionen des Programmoduls durch den die Bündelungsbestätigungsanforderung empfangenden Blockchain-Server, wobei das Ausführen der fünften Programminstruktionen eine Prüfung unter Verwendung der aktuellen Prüfwerte der Verbrauchsgüter der Gruppe von Verbrauchsgütern umfasst, ob die Bündelungsbestätigungsanforderung ein oder mehrere fünfte Voraussetzungen erfüllt, und, falls die fünften Voraussetzungen erfüllt sind, ein Hinzufügen des Gruppenerstellerprüfwerts, des Gruppenidentifikators und der Identifikatoren der Verbrauchsgüter der Gruppe von Verbrauchsgütern zu einem Eintrag in einem zusätzlichen Block der Blockchain, wodurch eine Bündelung der Verbrauchsgüter der Gruppe von Verbrauchsgütern in der Blockchain registriert wird, wodurch der Gruppenerstellerprüfwert den Verbrauchsgütern der Gruppe von Verbrauchsgütern als gemeinsamer aktueller Prüfwert in Ersatz zu den bisherigen aktuellen Prüfwerten der einzelnen Verbrauchsgüter der Gruppe von Verbrauchsgütern zugeordnet wird und wobei dem Gruppenersteller als aktuellem Gruppenbesitzer der Gruppe von Verbrauchsgütern in Ersatz zu dem bisherigen aktuellen Besitzer der einzelnen Verbrauchsgüter der Gruppe von Verbrauchsgütern die Berechtigung zur Übertragung der Verbrauchsgüter der Gruppe von Verbrauchsgütern zusammen als Gruppe zugeordnet wird.

Ausführungsformen können den Vorteil haben, dass Verbrauchsgüter, welche beispielsweise zum Zwecke der Lieferung zusammengepackt werden, effektiv nachverfolgt werden können. Beispielsweise umfasst die Gruppe nur ein Verbrauchsgut. Im Fall, dass die Gruppe nur ein Verbrauchsgut umfasst, stellt der Gruppenidentifikator einen Identifikator der Verpackung des entsprechenden Verbrauchsguts dar. In diesem Fall wird die Verpackung des Verbrauchsguts und deren Handhabung in der Blockchain protokolliert. Dies ist insbesondere vorteilhaft, wenn das entsprechende Verbrauchsgut im Zuge des Vertriebs ein- oder mehrfach umverpackt wird.

Nach Ausführungsformen umfasst die Gruppe von Verbrauchsgütern eine Mehrzahl von Verbrauchsgüter. Somit können Verbrauchsgüter beispielsweise effektiv nachverfolgt werden, wenn sie in größeren Einheiten bzw. Gruppen geliefert werden, als sie letztendlich an den Endverbraucher abgegeben werden.

Nach Ausführungsformen umfasst das Verfahren ferner:
- im Fall eines Besitzerwechsels des Verpackungsbehältnisses mit der Gruppe von Verbrauchsgütern, Empfangen einer Gruppenübertragungsbestätigungsanforderung zur Übertragung der Gruppe durch einen der Blockchain-Server des Blockchain-Netzwerks, wobei die Gruppenübertragungsbestätigungsanforderung den Gruppenidentifikator, einen Berechtigungsnachweis des aktuellen Gruppenbesitzers vor dem Besitzerwechsel zur Übertragung der Gruppe von Verbrauchsgütern und einen weiteren Gruppenprüfwert umfasst, wobei der weitere Gruppenprüfwert zur Prüfung einer Berechtigung zur Übertragung der Gruppe von Verbrauchsgütern durch einen Empfänger der Gruppe von Verbrauchsgütern als zukünftigem Gruppenbesitzer dient,
- Ausführen sechster Programminstruktionen des Programmoduls durch den die Gruppenübertragungsbestätigungsanforderung empfangenden Blockchain-Server, wobei das Ausführen der sechsten Programminstruktionen eine Prüfung unter Verwendung des aktuellen Gruppenprüfwerts umfasst, ob die Gruppenübertragungsbestätigungsanforderung ein oder mehrere sechste Voraussetzungen erfüllt, und, falls die sechsten Voraussetzungen erfüllt sind, ein Hinzufügen des weiteren Gruppenprüfwerts und des Gruppenidentifikators einem Eintrag in einem zusätzlichen Block der Blockchain, wodurch der weitere Gruppenprüfwert dem ersten Verbrauchsgut als aktueller Gruppenprüfwert in Ersatz zu dem bisherigen aktuellen Gruppenprüfwert zugeordnet wird und wobei dem weiteren Gruppenbesitzer als aktuellem Gruppenbesitzer in Ersatz zu dem bisherigen aktuellen Gruppenbesitzer die Berechtigung zur Übertragung der Gruppe von Verbrauchsgutes zugeordnet wird.

Ausführungsformen können den Vorteil haben, dass sie eine effektive und effiziente Nachverfolgung von Verpackungsbehältnissen der Verbrauchsgüter ermöglichen.

Nach Ausführungsformen umfasst das Verfahren ferner:
- im Fall eines Entpackens des Verpackungsbehältnisses, Empfangen einer Entbündelungsbestätigungsanforderung zur Bestätigung einer Auflösung der Bündelung der Berechtigungen zur Übertragung der Verbrauchsgüter der Gruppe von Verbrauchsgütern, wobei die Entbündelungsbestätigungsanforderung den Gruppenidentifikator der Gruppe, einen Berechtigungsnachweis des aktuellen Gruppenbesitzers der Gruppe vor dem Entpacken und einen oder mehrere weitere Prüfwerte zur Prüfung einer Berechtigung zur Übertragung der Verbrauchsgütern der Gruppe von Verbrauchsgütern durch einen zukünftigen Besitzer der Verbrauchsgüter der Gruppe von Verbrauchsgütern dient,
- Ausführen siebter Programminstruktionen des Programmoduls durch den die Entbündelungsbestätigungsanforderung empfangenden Blockchain-Server, wobei das Ausführen der siebten Programminstruktionen eine Prüfung unter Verwendung der aktuellen Gruppenprüfwerts umfasst, ob die Entbündelungsbestätigungsanforderung ein oder mehrere siebte Voraussetzungen erfüllt, und, falls die siebten Voraussetzungen erfüllt sind, ein Hinzufügen der ein oder mehreren weiteren Prüfwerte und der Identifikatoren der Verbrauchsgüter der aufgelösten Gruppe von Verbrauchsgütern zu ein oder mehreren Einträgen in ein oder mehreren zusätzlichen Blöcken der Blockchain, wodurch die ein oder mehreren weiteren Prüfwerte jeweils ein oder mehreren der Verbrauchsgüter der aufgelösten Gruppe von Verbrauchsgütern als aktuelle Prüfwert in Ersatz zu dem gemeinsamen Gruppenprüfwert zugeordnet werden und wobei dem bisherigen aktuellen Gruppenbesitzer als aktuellem Besitzer der einzelnen Verbrauchsgüter der aufgelösten Gruppe von Verbrauchsgütern die Berechtigung zur individuellen Übertragung der einzelnen Verbrauchsgüter der aufgelösten Gruppe von Verbrauchsgütern zugeordnet wird.

Ausführungsformen können den Vorteil haben, dass sie neben der Nachverfolgung der Verpackungsbehältnisse eine effektive und effiziente Nachverfolgung der einzelnen Verbrauchsgüter erlauben, wenn diese aus den Verpackungsbehältnissen entnommen werden.

Nach Ausführungsformen ist den in der Blockchain registrierten Gruppen von Verbrauchsgütern maximal ein aktueller Gruppenprüfwert zugeordnet, bei welchem es sich jeweils um den der entsprechenden Gruppe zuletzt zugordneten Gruppenprüfwert handelt. Ausführungsformen können den Vorteil haben, dass die Gruppenprüfwerte die Verpackungsbehältnisse eindeutig identifizieren können.

Nach Ausführungsformen sind die Gruppenerstellerprüfwerte der in der Blockchain registrierten Gruppen von Verbrauchsgüter eindeutig für die einzelnen Gruppen und werden jeweils als Gruppenidentifikatoren der entsprechenden Gruppen von Verbrauchsgütern verwendet. Ausführungsformen können den Vorteil haben, dass ein Gruppenerstellerprüfwert als Gruppenidentifikator dienen kann. Hierzu handelt es sich beispielsweise bei allen weiteren Gruppenprüfwerten jeweils um gruppenindividuelle Prüfwerte, so dass analog zur vorbeschriebenen verbrauchsgutindividuellen Prüfwertkette eine gruppenindividuelle Prüfwertkette implementiert werden kann, anhand derer alle Einträge in der Blockchain identifiziert werden können, welche sich auch in die entsprechende Gruppe beziehen.

Nach Ausführungsformen wird ein Gruppenerstellerprüfwerte für mehrere der in der Blockchain registrierten Gruppen von Verbrauchsgüter verwendet und die individuellen Gruppenidentifikatoren der in der Blockchain registrierten Gruppen sind unabhängig von den Gruppenerstellerprüfwerten. Ausführungsformen können den Vorteil haben, dass der Gruppenerstellerprüfwert zugleich als ein Gruppenerstelleridentifikator dienen kann. In diesem Fall ist zur Identifizierung der Gruppe ein zusätzlicher eindeutiger Gruppenidentifikator notwendig, welche jedem der Einträge zu der entsprechenden Gruppe hinzugefügt wird.

Nach Ausführungsformen handelt es sich bei den Gruppenprüfwerten jeweils um gruppenindividuelle Gruppenprüfwerte. Ausführungsformen können den Vorteil haben, dass anhand der Gruppenprüfwerte, bei welchen es sich um gruppenindividuelle Gruppenprüfwerte handelt, eine eindeutige Gruppenprüfwertkette implementiert werden kann, welche individuell für jede Gruppe ist und anhand derer alle Einträge in der Blockchain identifiziert werden können, welche sich auf die entsprechende Gruppe beziehen.

Nach Ausführungsformen wird ein Gruppenprüfwert für mehrere Gruppen von Verbrauchsgütern verwendet. Ausführungsformen können den Vorteil haben, dass Gruppenprüfwerte, welche für mehrere Gruppen von Verbrauchsgütern verwendet werden, als Identifikatoren der Besitzer der entsprechenden Gruppen verwendet werden können. Mit anderen Worten, einem individuellen Besitzer von Gruppen ein besitzerindividueller Gruppenprüfwert zugeordnet wird, welcher für alle Gruppen verwendet wird, die sich im Besitz des entsprechenden Besitzers befinden. Mithin kann anhand des Gruppenprüfwertes der Besitzer einer Gruppe identifiziert werden, nicht aber die Gruppe selbst, für welche ein eigenständiger Identifikator notwendig ist.

Nach Ausführungsformen sind die Gruppenerstellerprüfwerte jeweils aus einem gruppenerstellerindividuellen Seed Key abgeleitet und unter Verwendung des gruppenerstellerindividuellen Seed Keys auf ihre Gültigkeit überprüfbar.

Ausführungsformen können den Vorteil haben, dass anhand des Seed Keys in die Gültigkeit eines Gruppenerstellerprüfwerts geprüft werden kann und der entsprechende Gruppenerstellerprüfwert somit zugleich als Berechtigung des Gruppenerstellers zum Eintrag des entsprechenden Gruppenerstellerprüfwertes dienen kann.

Nach Ausführungsformen ist jedes Verpackungsbehältnis einer in der Blockchain registrierten Gruppe von Verbrauchsgütern mit einem maschinenlesbaren Code versehen, wobei der maschinenlesbare Code einen Gruppenidentifikator der entsprechenden Gruppe umfasst.

Ausführungsformen können den Vorteil haben, dass die Verpackungsbehältnisse ebenfalls über einen maschinenlesbaren Code, z. B. einen auf Druck beispielsweise in Form eines QR-Codes oder ein elektronisches Speicherelement, wie etwa einen RFID-Tag, verfügen. Somit können die einzelnen Verpackungsbehältnisse automatisch identifiziert und durch einen Abgleich mit den Einträgen in der Blockchain überprüft werden. Nach Ausführungsformen dient dabei der Gruppenidentifikator als Zugriffsberechtigung zum Auslesen der entsprechenden Datensätze aus der Blockchain, welcher der durch den Gruppenidentifikator identifizierten Gruppen zugeordnet sind. Nach Ausführungsformen kann es sich bei der entsprechenden Zugriffsberechtigung um eine beschränkte Zugriffsberechtigung handeln, bei welcher nur bestimmte für die entsprechende Gruppe gespeicherte Daten bereitgestellt werden und/oder eine Bestätigung, dass die eingetragenen Daten konsistent sind. Nach alternativen Ausführungsformen können alle die Gruppe betreffenden in die Blockchain eingetragenen Daten eingesehen werden.

Nach Ausführungsformen ist eine der fünften Voraussetzungen eine gültige Signatur der Bündelungsbestätigungsanforderung. Das Ausführen der fünften Programminstruktionen umfasst das Prüfen der Signatur der Bündelungsbestätigungsanforderung unter Verwendung eines zweiten öffentlichen kryptografischen Schlüssels des Gruppenerstellers der Gruppe von Verbrauchsgütern. Ausführungsformen können den Vorteil haben, dass anhand einer Signaturprüfung der Bündelungsbestätigungsanforderung geprüft werden kann, ob ein Ersteller auch tatsächlich zum Eintragen der von ihm erstellten Gruppe berechtigt ist.

Nach Ausführungsformen wird der zweite öffentliche kryptografische Schlüssel des Gruppenerstellers zur Prüfung der Signatur der Bündelungsbestätigungsanforderung von der Blockchain zur Verfügung gestellt. Ausführungsformen können den Vorteil haben, dass die Blockchain alle zur Signaturprüfung notwendigen Daten selbst bereitstellten. Somit kann die Blockchain beispielsweise eine eigenständige PKI bereitstellen.

Nach Ausführungsformen handelt es sich bei den Verbrauchsgütern um medizinische und/oder pharmazeutische Verbrauchgüter.

Ausführungsformen können den Vorteil haben, dass insbesondere in fälschungsanfälligen Verbrauchsgütern eine effiziente und effektive Fälschungssicherung sowie Abgabeprotokollierung implementiert werden kann. Somit kann neben einer Vermeidung von wirtschaftlichen Schäden auch gesundheitlichen Schäden vorgebeugt werden, welche durch gefälschte medizinische und/oder pharmazeutische Verbrauchsgüter entstehen können. Darüber hinaus kann ein Missbrauch insbesondere von pharmazeutischen Verbrauchsgütern effektiv unterbunden werden.

Nach Ausführungsformen handelt es sich bei den Verbrauchsgütern um Medikamente.

Nach Ausführungsformen umfasst das Verfahren ferner:
- im Fall eines Ausstellens eines Rezepts für ein erstes Medikament, Empfangen einer zweiten Registrierungsanforderung zum Registrieren des ersten Rezepts in der Blockchain durch einen der Blockchain-Server des Blockchain-Netzwerks, wobei die zweite Registrierungsanforderung einen Austellerdatensatz mit Ausstellungsdaten des Rezepts umfasst, wobei die Ausstellungdaten einen Rezeptidentifikator umfassen, wobei es sich bei dem Rezept um ein Rezept für ein erstes Medikament in Form des ersten Verbrauchsguts handelt,
- Ausführen achter Programminstruktionen des Programmoduls durch den die zweite Registrierungsanforderung empfangenden Blockchain-Server, wobei das Ausführen der achten Programminstruktionen eine Prüfung umfasst, ob die zweite Registrierungsanforderung ein oder mehrere achte Voraussetzungen erfüllen, und, falls die achten Voraussetzungen erfüllt sind, ein Hinzufügen des Ausstellerdatensatzes zu einem Eintrag in einem zusätzlichen Block der Blockchain, wodurch das erste Rezept in der Blockchain registriert wird,
- im Fall der Endabgabe des ersten Verbrauchsgutes an den Endverbraucher, falls die dritten Voraussetzungen durch die Endabgabebestätigungsanforderung zur Bestätigung der Endabgabe des ersten Verbrauchsgutes erfüllt sind, Hinzufügen des Rezeptidentifikators zu dem Eintrag in dem zusätzlichen Block der Blockchain mit dem Identifikator des ersten Verbrauchsguts und dem Endabgabevermerk.

Ausführungsform können den Vorteil haben, dass die Blockchain neben einer Sicherung in der Vertriebskette des Verbrauchsguts im Fall eines verschreibungspflichtigen Medikaments eine effiziente und effektive Abgabekontrolle implementieren kann. Somit kann sichergestellt werden, dass eine Abgabe eines verschreibungspflichtigen Medikaments nur an einen Endverbraucher, d.h. Patienten, erfolgt, welcher durch ein Rezept seine Empfangsberechtigung für das entsprechende verschreibungspflichtige Medikament nachweisen kann.

Nach Ausführungsformen ist das in der Blockchain registrierte Rezept mit einem maschinenlesbaren Code versehen, wobei der maschinenlesbare Code des Rezepts einen Identifikator des Rezepts umfasst.

Ausführungsformen können den Vorteil haben, dass anhand des maschinenlesbaren Codes, etwa einem QR-Code oder einem RFID-Tag, ein Rezept vollautomatisch erkannt werden kann. Eine Zuordnung von Rezept und verschreibungspflichtigem Medikament zu einem in dem Rezept identifizierten berechtigten Empfänger des verschreibungspflichtigen Medikaments kann somit vollautomatisch erfolgen. Insbesondere kann sichergestellt werden, dass eine Ausgabe eines verschreibungspflichtigen Medikaments nur auf Vorlage eines gültigen Rezepts erfolgt. Die Blockchain bietet somit den Vorteil, dass neben den Verbrauchsgütern auch die Rezepte nachverfolgt werden können und eine Fälschungssicherung implementiert werden kann.

Nach Ausführungsformen umfasst die zweite Registrierungsanforderung zum Registrieren des Rezepts einen Endabgabeprüfwert, wobei der Endabgabeprüfwert zur Prüfung einer Berechtigung zur Endabgabe eines Medikaments gemäß dem Rezept dient. Der maschinenlesbare Code des Rezepts umfasst einen Nachweis zur Berechtigung zur Endabgabe eines Medikaments gemäß dem Rezept. Die im Fall der Endabgabe des ersten Verbrauchsgutes an den Endverbraucher empfangene Endabgabebestätigungsanforderung umfasst den von dem ersten Rezept maschinengelesenen Code.

Nach Ausführungsformen handelt es sich bei den Medikamenten um Betäubungsmittel. Nach Ausführungsformen handelt es sich bei den Rezepten um Betäubungsmittelrezepte.

Nach Ausführungsformen umfasst eine der achten Voraussetzungen eine gültige Signatur der zweiten Registrierungsanforderung ist und wobei das Ausführen der achten Programminstruktionen das Prüfen der Signatur der zweiten Registrierungsanforderung unter Verwendung eines dritten öffentlichen kryptografischen Schlüssel des Ausstellers des Rezepts.

Ausführungsformen können den Vorteil haben, dass anhand einer Signaturprüfung sichergestellt werden kann, dass nur Rezepte in der Blockchain registriert werden, welche von berechtigten Ausstellern ausgestellt wurden.

Nach Ausführungsformen wird der dritte öffentliche kryptografische Schlüssel des Ausstellers des Rezepts zur Prüfung der Signatur der zweiten Registrierungsanforderung von der Blockchain zur Verfügung gestellt.

Ausführungsformen können den Vorteil haben, dass eine entsprechende Signaturprüfung zur Registrierung von Rezepten in der Blockchain allein auf Basis von Information erfolgen kann, welche von der Blockchain selbst zur Verfügung gestellt werden. Ausführungsformen können somit den Vorteil haben, dass die Blockchain beispielsweise eine eigenständige PKI implementieren kann.

Nach Ausführungsformen umfasst das Verfahren ferner:
- Empfangen einer dritten Registrierungsanforderung zum Registrieren eines Herstellers von Verbrauchsgütern durch einen Blockchain-Server, wobei die dritte Registrierungsanforderung signiert ist,
- Ausführen neunter Programminstruktionen des Programmoduls durch den die dritte Registrierungsanforderung empfangenden Blockchain-Server, wobei die Ausführung der neunten Programminstruktionen eine Prüfung unter Verwendung eines vierten öffentlichen kryptografischen Schlüssels, ob die Signatur der dritten Registrierungsanforderung gültig ist, und, falls die Signatur der dritten Registrierungsanforderung gültig ist, ein Registrieren des Herstellers in der Blockchain, wobei das Registrieren ein Hinzufügen eines Herstelleridentifikators zu einem Eintrag in einem zusätzlichen Blocks zu der Blockchain umfasst.

Ausführungsformen können den Vorteil haben, dass sichergestellt werden kann, dass nur offiziell registrierte Hersteller von Verbrauchsgütern, die Blockchain nutzen können.

Nach Ausführungsformen wird der vierte öffentliche kryptografische Schlüssel zur Prüfung der Signatur der dritten Registrierungsanforderung von der Blockchain zur Verfügung gestellt.

Nach Ausführungsformen umfasst das Verfahren ferner:
- Empfangen einer vierten Registrierungsanforderung zum Registrieren eines potentiellen Besitzers durch einen Blockchain-Server, wobei die vierte Registrierungsanforderung signiert ist,
- Ausführen zehnter Programminstruktionen des Programmoduls durch den die vierte Registrierungsanforderung empfangenden Blockchain-Server, wobei die Ausführung der zehnten Programminstruktionen eine Prüfung unter Verwendung eines fünften öffentlichen kryptografischen Schlüssels, ob die Signatur der vierten Registrierungsanforderung gültig ist, und, falls die Signatur der vierten Registrierungsanforderung gültig ist, ein Registrieren des potentiellen Besitzers in der Blockchain, wobei das Registrieren ein Hinzufügen eines Identifikators des potentiellen Besitzers zu einem Eintrag in einem zusätzlichen Blocks zu der Blockchain umfasst.

Ausführungsformen können den Vorteil haben, dass sichergestellt werden kann, dass nur offiziell registrierte potentielle Besitzer als Besitzer fungieren können, an welche die Verbrauchsgüter übertragen werden, Blockchain nutzen können. Somit kann sichergestellt werden, dass nur berechtigte Teilnehmer die Verbrauchsgüter handhaben dürfen.

Nach Ausführungsformen wird der fünfte öffentliche kryptografische Schlüssel zur Prüfung der Signatur der dritten Registrierungsanforderung von der Blockchain zur Verfügung gestellt.

Nach Ausführungsformen umfasst das Verfahren ferner:
- Empfangen einer fünften Registrierungsanforderung zum Registrieren eines Ausstellers von Rezepten durch einen Blockchain-Server, wobei die fünfte Registrierungsanforderung signiert ist,
- Ausführen elfter Programminstruktionen des Programmoduls durch den die fünfte Registrierungsanforderung empfangenden Blockchain-Server, wobei die Ausführung der elften Programminstruktionen eine Prüfung unter Verwendung eines sechsten öffentlichen kryptografischen Schlüssels, ob die Signatur der fünften Registrierungsanforderung gültig ist, und, falls die Signatur der fünften Registrierungsanforderung gültig ist, ein Registrieren des Austellers in der Blockchain, wobei das Registrieren ein Hinzufügen eines Ausstelleridentifikators zu einem Eintrag in einem zusätzlichen Blocks zu der Blockchain umfasst.

Ausführungsformen können sicherstellen, dass nur offizielle berechtigte Aussteller von Rezepten dazu in die Lage versetzt werden, die Blockchain zu nutzen und gültige Rezepte zu registrieren.

Nach Ausführungsformen wird der sechste öffentliche kryptografische Schlüssel zur Prüfung der Signatur der fünften Registrierungsanforderung von der Blockchain zur Verfügung gestellt.

Nach Ausführungsformen umfasst das Verfahren ferner:
- Empfangen einer Leseanfrage zum Lesen von Einträgen zu dem ersten Verbrauchsgut durch einen der Blockchain-Server, wobei die Leseanfrage einen Identifikator des ersten Verbrauchsgutes umfasst,
- Ausführen zwölfter Programminstruktionen des Programmoduls durch den die Leseanfrage empfangenden Blockchain-Server, wobei die Ausführung der zwölften Programminstruktionen eine Prüfung durch den Blockchain-Server umfasst, ob das erste Verbrauchsgut in der Blockchain registriert ist, und, falls das erste Verbrauchsgut in der Blockchain registriert ist, ein Senden von Kopien der Einträge zu dem ersten Verbrauchsgut an einen Absender der Leseanfrage.

Ausführungsformen können den Vorteil haben, dass neben den Blockchain-Servern weitere Personen und/oder Institutionen zum Lesen von Daten aus der Blockchain berechtigt werden. Eine entsprechende Berechtigung kann beispielsweise durch den Besitz eines entsprechenden Verbrauchsguts vorliegen, wenn ein Identifikator des Verbrauchsguts eine entsprechende Leseberechtigung darstellt. Nach Ausführungsformen können unterschiedliche Leseberechtigungen vergeben werden, sodass in Abhängigkeit von der entsprechenden Leseberechtigung unterschiedliche Daten der Blockchain einsehbar sind.

Nach Ausführungsformen umfassen die Einträge zu dem ersten Verbrauchsgut einen Identifikator des Endverbrauchers und/oder einen Ausstellungsdatensatz eines dem ersten Verbrauchsgut zugeordneten Rezepts, wobei die Prüfung eine Signaturprüfung der Leseanfrage unter Verwendung eines siebten öffentlichen Schlüssels umfasst.

Nach Ausführungsformen wird der siebte öffentliche kryptografische Schlüssel zur Prüfung der Signatur der Leseanfrage von der Blockchain zur Verfügung gestellt. Ausführungsformen können den Vorteil haben, dass die Blockchain eine eigenständige PKI implementieren kann.

Ausführungen umfassen ein Blockchain-Netzwerk mit Mehrzahl von Blockchain-Servern zur Fälschungssicherung von Verbrauchsgütern unter Verwendung einer von dem Blockchain-Netzwerk bereitgestellten Blockchain. Die Blockchain speichert für jedes der Verbrauchsgüter eine kryptografisch gesicherte Historie. Die Blockchain-Server sind jeweils dazu konfiguriert, unter Verwendung eines von der Blockchain umfassten Programmmoduls zusätzliche Blöcke für die Blockchain zu erstellen und zu der Blockchain hinzuzufügen.

Das Blockchain-Netzwerk ist zur kryptografisch gesicherten Speicherung einer Historie eines ersten Verbrauchsgutes konfiguriert zum:
- im Fall einer Herstellung des ersten Verbrauchsgutes, Empfangen einer ersten Registrierungsanforderung zum Registrieren des ersten Verbrauchsgutes in der Blockchain durch einen der Blockchain-Server des Blockchain-Netzwerks, wobei die erste Registrierungsanforderung einen Herstellerdatensatz mit Herstellungsdaten des ersten Verbrauchsgutes und mit einem Herstellerprüfwert umfasst, wobei der Herstellerprüfwert zur Prüfung einer Berechtigung zur Übertragung des ersten Verbrauchsgutes durch einen Hersteller des ersten Verbrauchsgutes dient,
- Ausführen erster Programminstruktionen des Programmoduls durch den die erste Registrierungsanforderung empfangenden Blockchain-Server, wobei das Ausführen der ersten Programminstruktionen eine Prüfung umfasst, ob die erste Registrierungsanforderung ein oder mehrere erste Voraussetzungen erfüllen, und, falls die ersten Voraussetzungen erfüllt sind, ein Hinzufügen des Herstellerdatensatzes zu einem Eintrag in einem zusätzlichen Block der Blockchain, wodurch das erste Verbrauchsgut in der Blockchain registriert, der Herstellerprüfwert dem ersten Verbrauchsgut als ein aktueller Prüfwert zugeordnet und dem Hersteller als einem aktuellen Besitzer des ersten Verbrauchsgutes die Berechtigung zur Übertragung des ersten Verbrauchsgutes zugeordnet wird.

Ferner ist das Blockchain-Netzwerk konfiguriert zum ein- oder mehrmaligen:
- im Fall eines Besitzerwechsels des ersten Verbrauchsgutes, Empfangen einer Übertragungsbestätigungsanforderung zur Bestätigung des Besitzerwechsels des ersten Verbrauchsgutes durch einen der Blockchain-Server des Blockchain-Netzwerks, wobei die Übertragungsbestätigungsanforderung einen Identifikator des ersten Verbrauchsgut, einen Berechtigungsnachweis des aktuellen Besitzers vor dem Besitzerwechsel zur Übertragung des ersten Verbrauchsgutes und einen weiteren Prüfwert umfasst, wobei der weitere Prüfwert zur zukünftigen Prüfung einer Berechtigung zur Übertragung des ersten Verbrauchsgutes durch einen Empfänger des ersten Verbrauchsgutes als zukünftigem Besitzers dient,
- Ausführen zweiter Programminstruktionen des Programmoduls durch den die Übertragungsbestätigungsanforderung empfangenden Blockchain-Server, wobei das Ausführen der zweiten Programminstruktionen eine Prüfung unter Verwendung des aktuellen Prüfwerts umfasst, ob die Übertragungsbestätigungsanforderung ein oder mehrere zweite Voraussetzungen erfüllt, und, falls die zweiten Voraussetzungen erfüllt sind, ein Hinzufügen des weiteren Prüfwerts und des Identifikators des ersten Verbrauchsgutes zu einem Eintrag in einem zusätzlichen Block der Blockchain, wodurch der weitere Prüfwert dem ersten Verbrauchsgut als aktueller Prüfwert in Ersatz zu dem bisherigen aktuellen Prüfwert zugeordnet wird und wobei dem weiteren Besitzer als aktuellem Besitzer des ersten Verbrauchsgutes in Ersatz zu dem bisherigen aktuellen Besitzer die Berechtigung zur Übertragung des ersten Verbrauchsgutes zugeordnet wird.

Ferner ist das Blockchain-Netzwerk konfiguriert zum:
- im Fall einer Endabgabe des ersten Verbrauchsgutes an einen Endverbraucher, Empfangen einer Endabgabebestätigungsanforderung zur Bestätigung der Endabgabe des ersten Verbrauchsgutes durch einen der Blockchain-Server des Blockchain-Netzwerks, wobei die Endabgabebestätigungsanforderung den Identifikator des ersten Verbrauchsgutes und einen Berechtigungsnachweis des aktuellen Besitzers vor der Endabgabe zur Übertragung des ersten Verbrauchsgutes umfasst,
- Ausführen dritter Programminstruktionen des Programmoduls durch den die Endabgabebestätigungsanforderung empfangenden Blockchain-Server, wobei das Ausführen der dritten Programminstruktionen eine Prüfung unter Verwendung des aktuellen Prüfwerts umfasst, ob die Endabgabebestätigungsanforderung des aktuellen Besitzers ein oder mehrere dritte Voraussetzungen erfüllt, und, falls die dritten Voraussetzungen erfüllt sind, ein Hinzufügen des Identifikators des ersten Verbrauchsgutes und eines Endabgabevermerk zu einem Eintrag in einem zusätzlichen Block der Blockchain, wodurch der Endabgabevermerk dem ersten Verbrauchsgut zugeordnet wird, wobei der Endabgabevermerk festlegt, dass das erste Verbrauchsgut von weiteren Übertragungen ausgeschlossen ist.

Nach Ausführungsformen ist das Blockchain-Netzwerk dazu konfiguriert ein oder mehrere der zuvor genannten Ausführungsformen des Verfahrens zum manipulationssicheren Ausstellen und Speichern einer Mehrzahl von elektronischen Urkunden unter Verwendung der Blockchain auszuführen.

Die Verwendung von Ordinalzahlen wie erstes, zweites, drittes etc. dient hier, soweit sich aus dem konkreten Zusammenhang nicht eindeutig etwas anderes ergibt, allein der Unterscheidung voneinander verschiedener Elemente und soll keine bestimmte Reihenfolge implizieren.

Im Weiteren werden Ausführungsformen der Erfindung mit Bezugnahme auf die Zeichnungen näher erläutert. Es zeigen
- Figuren 1: schematische Blockdiagramme einer Ausführungsform eines exemplarischen Systems zur Fälschungssicherung und Abgabekontrolle von Verbrauchsgütern,
- Figur 2: ein schematisches Blockdiagramm eines Verfahrens zum Eintragen von manipulationssicherem Speichern von Daten zu einem Verbrauchsgut,
- Figur 3: ein schematisches Blockdiagramm einer Ausführungsform eines exemplarischen Verfahrens zum Auslesen von Daten aus einer Blockchain zur Fälschungssicherung und Abgabekontrolle eines Verbrauchsguts,
- Figur 4: ein Flussdiagramm einer Ausführungsform eines Verfahrens zum manipulationssicheren Speichern einer Abgabe eines Verbrauchsguts,
- Figur 5: ein Flussdiagramm einer Ausführungsform eines Verfahrens zum manipulationssicheren Speichern einer Bündelung von ein oder mehreren Verbrauchsgütern,
- Figur 6: ein Flussdiagramm einer Ausführungsform eines Verfahrens zum manipulationssicheren Speichern einer Vernichtung eines Verbrauchsgutes,
- Figur 7: ein Flussdiagramm einer Ausführungsform eines exemplarischen Verfahrens zum Registrieren eines Rezepts in der Blockchain,
- Figur 8: ein Flussdiagramm einer Ausführungsform eines exemplarischen Verfahrens zum Lesen von Daten aus der Blockchain,
- Figur 9: ein Flussdiagramm einer Ausführungsform eines exemplarischen Verfahrens zum Registrieren eines Herstellers von Verbrauchsgütern in der Blockchain,
- Figur 10: ein Flussdiagramm einer Ausführungsform eines exemplarischen Verfahrens zum Speichern und Registrieren eines potenziellen Besitzers von Verbrauchsgütern in der Blockchain,
- Figur 11: ein Flussdiagramm einer Ausführungsform eines exemplarischen Verfahrens zum Registrieren eines Rezeptausstellers in der Blockchain.

Elemente der nachfolgenden Ausführungsformen, die einander entsprechen, werden mit denselben Bezugszeichen gekennzeichnet.

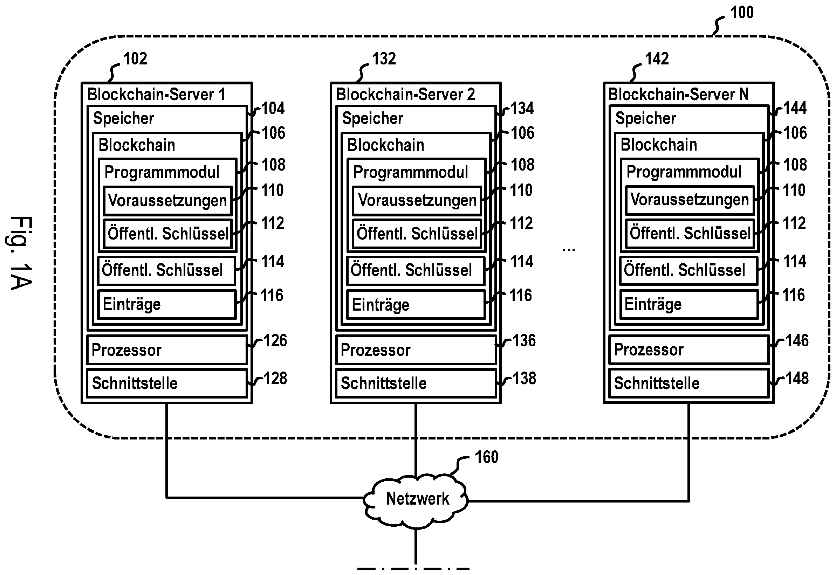

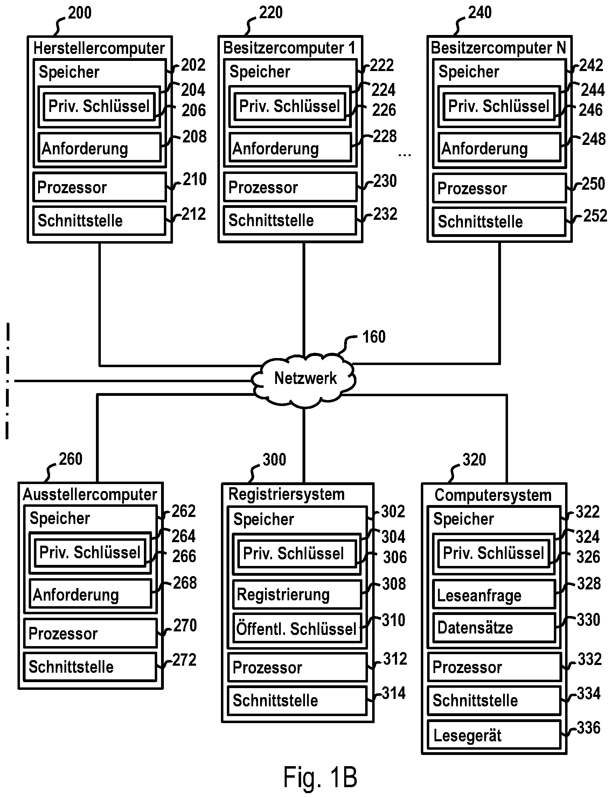

Figuren 1A und 1B zeigen eine Ausführungsform eines exemplarischen Systems zur Fälschungssicherung und Abgabekontrolle, wie beispielsweise verschreibungspflichtigen Medikamenten, insbesondere Betäubungsmittel. Das System umfasst ein Blockchain-Netzwerk 100 mit einer Mehrzahl von Blockchain-Servern 102, 132, 142. Die Blockchain-Server 102, 132, 142 umfassen jeweils einen Speicher 104, 134, 144, in welchem jeweils eine Kopie in der Blockchain 106 gespeichert ist. Ferner umfassen die Blockchain-Server 102, 132, 142 jeweils einen Prozessor 126, 136, 146. Durch Ausführen von Programminstruktionen werden die Prozessoren 126, 136, 146 dazu veranlasst, das Verfahren zur Fälschungssicherung und Abgabekontrolle von Verbrauchsgütern auszuführen. Die Blockchain-Server 102, 132, 142 umfassen zudem jeweils eine Kommunikationsschnittstelle 128, 138, 148, über welche sie mit dem Netzwerk 160 kommunizieren können. Die Blockchain 106, von welcher eine vollständige Kopie in jedem der Blockchain-Server 102, 132, 142 gespeichert ist, umfasst ein Programmmodul 108 mit einer Mehrzahl von Programminstruktionen, welche von den Prozessoren 126, 136, 146 ausgeführt werden können, wenn diese auf die Blockchain zugreifen. Die Programminstruktionen implementieren Voraussetzungen 110 zum Eintragen von Daten in die Blockchain. Ferner umfasst das Programmmodul ein oder mehrere öffentliche Schlüssel 112, welche als Root Keys, d. h. Wurzelschlüssel, einer eigenständigen PKI dienen, welche von der Blockchain 106 bereitgestellt wird. Die von der Blockchain 106 bereitgestellte PKI umfasst eine Mehrzahl von öffentlichen Schlüsseln 114, deren Gültigkeit von dem öffentlichen Schlüssel 112 abgeleitet ist, d. h. die entsprechenden öffentlichen Schlüssel 114 werden lediglich unter der Voraussetzung in die Blockchain 106 eingetragen, dass die entsprechenden Eintragungsregistrierungen mit einer Signatur versehen sind, deren Authentizität mit einem bereits in der Blockchain registrierten öffentlichen Schlüssel 112, 114 geprüft werden kann. Ferner umfasst die Blockchain eine Mehrzahl von Einträgen 116, welche in einer Mehrzahl von Blöcken in der Blockchain angeordnet sind. Die Blöcke der Blockchain 106 sind kryptografisch miteinander verkettet. In den Einträgen 116 werden Daten zu den Verbrauchsgütern von deren Herstellern bis zu deren Abgabe an einen Endverbraucher, d. h. einen Patienten im Fall eines verschreibungspflichtigen Medikaments, oder deren Vernichtung, z. B. im Fall eines Ablaufs eines Verfalldatums, eines Überschreitens eines Verfalldatums gespeichert. Über das Netzwerk 160 ist das Blockchain-Netzwerk 100 weiterer Computersysteme 200, 220, 240, 260, 300, 320 mit dem Blockchain-Netzwerk 100 kommunikativ verbunden. Bei dem Netzwerk 160 kann es sich um ein öffentliches Netzwerk, wie etwa das Internet, und/oder ein privates Netzwerk, wie etwa ein Intranet, handeln. Beispielsweise kann es sich bei dem Netzwerk 160 zumindest teilweise um ein mobiles Kommunikationsnetzwerk handeln.

Über das Netzwerk 160 ist beispielsweise ein Herstellercomputersystem 200 mit dem Blockchain-Netzwerk 100 verbunden, sodass das Herstellercomputersystem Registrierungsanforderungen 208 zur Registrierung von Verbrauchsgüter, welche der entsprechende Hersteller hergestellt hat, erstellen und über das Netzwerk 160 an einen der Blockchain-Server 102, 132, 142 des Blockchain-Netzwerks 100 senden kann. Das Herstellercomputersystem 200 umfasst beispielsweise einen Prozessor 210 zum Ausführen von Programminstruktionen, eine Kommunikationsschnittstelle 212 zur Kommunikation über das Netzwerk 160 sowie einen elektronischen Speicher 202 zum Speichern von Daten. In dem Speicher 202 ist beispielsweise in einem geschützten Speicherbereich 204 ein privater kryptografischer Schlüssel 206 eines asymmetrischen Schlüsselpaars gespeichert. Der öffentliche Schlüssel 114 des entsprechenden asymmetrischen Schlüsselpaars ist beispielsweise in der Blockchain hinterlegt. Die Hinterlegung des öffentlichen Schlüssels 114 kann beispielsweise das Ergebnis eines Registrierungsverfahrens zum Registrieren des Herstellers sein, welchem das Herstellercomputersystem 200 zugeordnet ist. Ferner kann der Speicher 202 eine Registrierungsanforderung 208 zum Registrieren eines Verbrauchsguts in der Blockchain 106 umfassen. Die entsprechende Anforderung 208 wird mit dem privaten Schlüssel 206 signiert und über die Kommunikationsschnittstelle 212 und dem Herstellercomputersystem 200 über das Netzwerk 160 an einen der Blockchain-Server 102, 132, 142 gesendet. Der empfangende Blockchain-Server 102, 132, 142 führt Programminstruktionen des Programmmoduls 108 aus, welches von der Blockchain 106 bereitgestellt wird. Im Zuge des Ausführens der entsprechenden Programminstruktionen wird die empfangene Registrierungsanforderung 208 zum Registrieren eines Verbrauchsguts geprüft. Die Überprüfung umfasst beispielsweise eine Prüfung gemäß den Voraussetzungen 110, welche das Programmmodul 108 implementiert. So wird die Registrierungsanforderung etwa auf eine gültige Signatur mit einem privaten kryptografischen Schlüssel 206 geprüft, der einem asymmetrischen Schlüsselpaar zugeordnet ist und dessen öffentlicher Schlüssel 114 in der Blockchain 106 als gültiger öffentlicher Schlüssel hinterlegt ist. Ferner wird die Anforderung 208 inhaltlich geprüft, d. h. ein von der Anforderung 208 umfasster Herstellungsdatensatz wird auf die darin enthaltenen Herstellungsdaten geprüft.

Ferner können über das Netzwerk 160 eine Mehrzahl von Besitzercomputersystemen 220, 240 mit dem Blockchain-Netzwerk 100 verknüpft sein. Die Besitzercomputersysteme 220, 240 umfassen jeweils einen Prozessor 230, 250 zum Ausführen von Programminstruktionen zur Implementierung des Verfahrens und zur Fälschungssicherung und Abgabekontrolle von Verbrauchsgütern, Kommunikationsschnittstellen 232, 252 zur Kommunikation über das Netzwerk 160 sowie einen Speicher 222, 242. In geschützten Speicherbereichen 224, 244 der Speicher 224, 242 ist jeweils zumindest ein privater kryptografischer Schlüssel 226, 246 gespeichert. Die entsprechenden privaten kryptografischen Schlüssel 226, 246 gehören jeweils zu asymmetrischen Schlüsselpaaren, deren öffentlicher kryptografischer Schlüssel 114 in der Blockchain 106 als gültige Signaturprüfschlüssel hinterlegt sind. Somit können beispielsweise unter Verwendung der Blockchain Signaturen geprüft werden, welche unter Verwendung der privaten kryptografischen Schlüssel 226, 246 erstellt wurden. In den elektronischen Speichern 222, 242 können ferner Übertragungsbestätigungsanforderungen 228, 248 zur Bestätigung von Besitzerwechseln von Verbrauchsgütern gespeichert sein. Ist ein Verbrauchsgut einem Besitzer zugeordnet, so kann dieser unter Verwendung seines Besitzercomputersystems, beispielsweise eines Besitzercomputersystems 220, 240, ein Übertragungsbestätigungsanforderung 228, 248, erstellen, mit dem privaten kryptografischen Schlüssel 226, 246 verschlüsseln und über das Netzwerk 146 an das Blockchain-Netzwerk 100 senden. Einer der Blockchain-Server 102, 132, 142 empfängt die entsprechende Anforderung 228, 248 und prüft diese unter Ausführung von Programminstruktionen, welche das Programmmodul 108 der Blockchain 106 bereitstellt, darauf, ob die entsprechenden Anforderungen 228, 248 Voraussetzungen 110 erfüllen, welche das Programmmodul 108 definierten. Erfüllen die Anforderungen 228, 248 die Voraussetzungen 110, sind sie z. B. mit einem privaten kryptografischen Schlüssel 226, 246 signiert und gilt ein Nachweis des Besitzers des privaten kryptografischen Schlüssels 226, 246 als Berechtigungsnachweis zum Übertragen eines Verbrauchsguts, so wird die entsprechende Übertragung als Eintrag in einem zusätzlichen Block der Blockchain von einem der Blockchain-Server 102, 132, 142 vermerkt.

Über das Netzwerk 160 kann ferner ein Ausstellercomputersystem 260 mit dem Blockchain-Netzwerk 100 kommunikativ verbunden sein. Das Ausstellercomputersystem 260 umfasst beispielsweise einen Prozessor 270 zum Ausführen von Programminstruktionen, eine Kommunikationsschnittstelle 272 zur Kommunikation über das Netzwerk 160 sowie einen elektronischen Speicher 262 mit einem geschützten Speicherbereich 264, in welchem ein privater kryptografischer Schlüssel 266 gespeichert ist. Der private kryptografische Schlüssel 266 gehört beispielsweise zu einem asymmetrischen Schlüsselpaar, dessen öffentlicher Schlüssel 114 in der Blockchain 106 als gültiger Schlüssel gespeichert ist, sodass mit dem entsprechenden öffentlichen kryptografischen Schlüssel 114 die Signaturen auf ihre Gültigkeit hin geprüft werden können, welche mit den privaten kryptografischen Schlüssel 266 erstellt wurden. Ferner umfasst der elektronische Speicher 262 beispielsweise eine Registrierungsanforderung 268 zum Registrieren eines Rezepts in der Blockchain 106. Das Rezept ist beispielsweise ein Rezept über eines der Verbrauchsgüter, falls es sich bei den Verbrauchsgütern um verschreibungspflichtige Medikamente handelt. Das entsprechende Rezept kann somit beispielsweise über die Blockchain 106 durch Registrieren der Registrierungsanforderung 268 ausgestellt und im Fall einer Abgabe eines der verschreibungspflichtigen Medikamente an einen Patienten in der Blockchain durch einen zusätzlichen Eintrag mit dem abgegebenen verschreibungspflichtigen Medikament verknüpft werden.

Ferner kann ein Registrierungscomputersystem 300 über das Netzwerk 160 mit dem Blockchain-Netzwerk 100 kommunikativ verbunden sein. Das Registrierungscomputersystem 300 umfasst dann beispielsweise einen Prozessor 312 zum Ausführen von Programminstruktionen, eine Kommunikationsschnittstelle 314 zur Kommunikation über das Netzwerk 160 sowie einen elektronischen Speicher 302 mit einem geschützten Speicherbereich 304, in welchem ein privater kryptografischer Schlüssel 306 gespeichert ist. Der private kryptografische Schlüssel 306 kann zu einem asymmetrischen Schlüsselpaar gehören, welches einen öffentlichen Schlüssel 114 einen öffentlichen kryptografischen Schlüssel 112, 114 umfasst, der als gültiger öffentlicher Schlüssel zur Prüfung von Signaturen in der Blockchain 106 hinterlegt ist. Beispielsweise handelt es sich bei dem Registrierungscomputersystem 300 um ein Computersystem, welches das Programmmodul 108 hergestellt und in der Blockchain 106 gespeichert hat. Bei dem in dem Registrierungscomputersystem 300 zugeordneten öffentlichen Schlüssel 112 der Blockchain 106 handelt es sich in diesem Fall beispielsweise um einen Root Key, d. h. Wurzelschlüssel, welche eine Wurzel einer der Blockchain 106 zugeordneten PKI bildet. Zur Implementierung der entsprechenden PKI erstellt das Registrierungscomputersystem 300 Registrierungsanforderungen 308 zum Registrieren von öffentlichen Schlüsseln 310. Die entsprechenden Registrierungsanforderungen werden mit dem privaten kryptografischen Schlüssel 306 des Registrierungscomputersystems 300 signiert. Die entsprechenden Signaturen können mit dem öffentlichen Schlüssel 112 der Blockchain 106 auf ihre Gültigkeit hin überprüft werden. Die Registrierungsanforderungen 308 werden über das Netzwerk 160 von dem Registrierungscomputersystem 300 an einen der Blockchain-Server 102, 132, 142 gesendet. Die entsprechenden Blockchain-Server 102, 132, 142 führen die Programminstruktionen des Programmmoduls 108 der Blockchain 106 aus und überprüfen die Signaturen der Registrierungsanforderung 308. Ist die Registrierungsanforderung 308 mit einer gültigen Signatur versehen, wird der entsprechende öffentliche Schlüssel 310 in die Blockchain 106 eingetragen.

Ferner kann das Blockchain-Netzwerk 100 über das Netzwerk 160 mit einem Computersystem 320 verbunden sein. Bei dem Computersystem 320 kann es sich beispielsweise um ein Computersystem eines Endverbrauchers eines Verbrauchsguts handeln. Beispielsweise handelt es sich bei dem Endverbraucher um einen Patienten, welcher unter Verwendung eines in der Blockchain 106 registrierten Rezepts ein verschreibungspflichtiges Medikament erhält. Das Computerprogramm umfasst beispielsweise einen Prozessor 232 zum Ausführen von Programminstruktionen, eine Kommunikationsschnittstelle 334 zur Kommunikation über das Netzwerk 160 mit dem Blockchain-Netzwerk 100 sowie einen elektronischen Speicher 322. In dem elektronischen Speicher 322 ist beispielsweise eine Leseanfrage 328 zum Auslesen von Datensätzen 330 aus der Blockchain 106 gespeichert. Nach Ausführungsformen umfasst das Computersystem 320 beispielsweise ein Lesegerät 336 zum Lesen eines maschinenlesbaren Codes, welches auf dem Verbrauchsgut, beispielsweise einem Medikament, angebracht ist. Bei dem maschinenlesbaren Code kann es sich beispielsweise um einen QR-Code handeln, welcher mit einem visuellen Scanner des Lesegeräts 336 gescannt werden kann oder es kann sich beispielsweise um einen RFID-Tag handeln, welcher mit dem Lesegerät 336 über eine Funkverbindung ausgelesen werden kann. Der maschinenlesbare Code stellt beispielsweise einen Identifikator des Verbrauchsguts bereit. Der entsprechende Identifikator kann beispielsweise als Leseberechtigung zum Lesen von Datensätzen 330 aus der Blockchain 106 dienen, welchen dementsprechend Verbrauchsgut zugeordnet sind. Umfasst die Leseanfrage 328 den entsprechenden Identifikator und wird die Leseanfrage 328 von einem Blockchain-Server 102, 132, 142 über das Netzwerk 160 empfangen, so wird dem Computersystem 320 beispielsweise als Antwort die Datensätze 330 zugesendet. Nach alternativen Ausführungsformen umfasst das Computersystem 320 in einem geschützten Speicherbereich 324 des Speichers 322 einen privaten kryptografischen Schlüssel 326, welcher zu einem asymmetrischen Schlüsselpaar gehört, dessen öffentlicher kryptografischer Schlüssel 114 in der Blockchain 106 gespeichert ist. Ferner ist das Computersystem 320 in diesem Fall nicht notwendigerweise einem Endverbraucher eines der Verbrauchsgüter zugeordnet. Der entsprechende private kryptografische Schlüssel 326 kann als Leseberechtigungsnachweis zum Lesen von Datensätzen 330 aus der Blockchain 106 dienen. Wird eine Leseanfrage 328 mit dem privaten kryptografischen Schlüssel 326 signiert, das Netzwerk 160 an einen der Blockchain-Server 102, 132, 142 gesendet, so überprüft der entsprechende Blockchain-Server 102, 132, 142 unter Verwendung von Programminstruktionen des Programmmoduls 108 der Blockchain 106, ob die Signatur gültig ist. Ist die Signatur gültig, so werden beispielsweise die angefragten Datensätze 330 über das Netzwerk 160 an das Computersystem 320 gesendet.

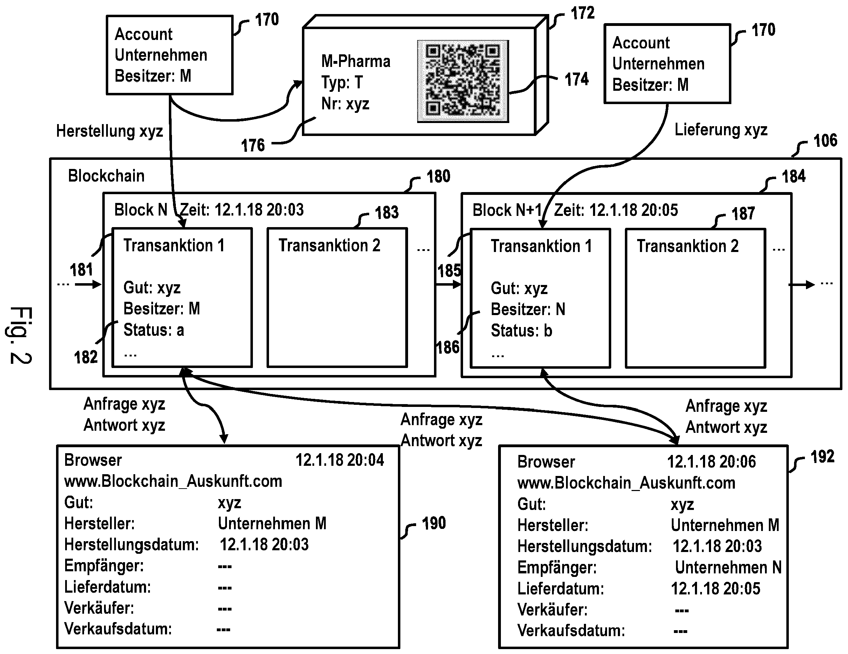

Figur 2 zeigt ein schematisches Blockdiagramm einer Ausführungsform des Verfahrens Fälschungssicherung und Abgabekontrolle eines Verbrauchsguts, wie beispielsweise einem verschreibungspflichtigen Medikament 172. Der Hersteller des entsprechenden Medikaments umfasst beispielsweise einen Account 170, welcher ihn als Besitzer ausweist. Der entsprechende Account 170 kann beispielsweise durch eine Registrierung eines dem Hersteller zugeordneten öffentlichen kryptografischen Schlüssels in der Blockchain 106 implementiert sein. Der Hersteller stellt ein verschreibungspflichtiges Medikament 172 her, welches mit Herstellungsdaten 176 versehen ist, sowie einen maschinenlesbaren Code 174, beispielsweise in Form eines QR-Codes. Ferner wird die Herstellung des verschreibungspflichtigen Medikaments 172 in einen Block 180 der Blockchain eingetragen. Hierzu wird ein Eintrag 181 von mehreren Einträgen 181, 183 des Blocks 180 der Blockchain 106 erstellt. Der entsprechende Eintrag umfasst Herstellungsdaten 182 des Medikaments 172. Die entsprechenden Daten der Blockchain 106 zu dem verschreibungspflichtigen Medikament 172 können über eine Anfrage abgefragt werden, wobei das Ergebnis einer solchen Abfrage beispielsweise die in einem Browser dargestellten Daten 190 sind. Die entsprechenden Daten identifizieren beispielsweise das verschreibungspflichtige Medikament 172, deren Hersteller sowie das Herstellungsdatum. Wird das verschreibungspflichtige Medikament 172 von dem Hersteller an einen Lieferanten ausgeliefert, so wird beispielsweise unter Verwendung des Accounts 170 ein weiterer Eintrag 185 in einen weiteren Block 184 der Blockchain 106 erstellt, welcher entsprechende Daten 186 umfasst. Werden die in dem verschreibungspflichtigen Medikament 172 zugeordneten Daten der Blockchain 106 nach Erstellung des Blocks 184 ausgelesen, so ergibt sich beispielsweise die Browserdarstellung 192, welche neben Herstellungsdaten auch Daten zu dem Empfänger des verschreibungspflichtigen Medikaments sowie das Lieferdatum umfasst. Auf diese Weise kann die vollständige Lieferkette des verschreibungspflichtigen Medikaments 172 von der Herstellung über die Lieferung bis zur Endabgabe lückenlos anhand der Blockchain 106 nachvollzogen werden.

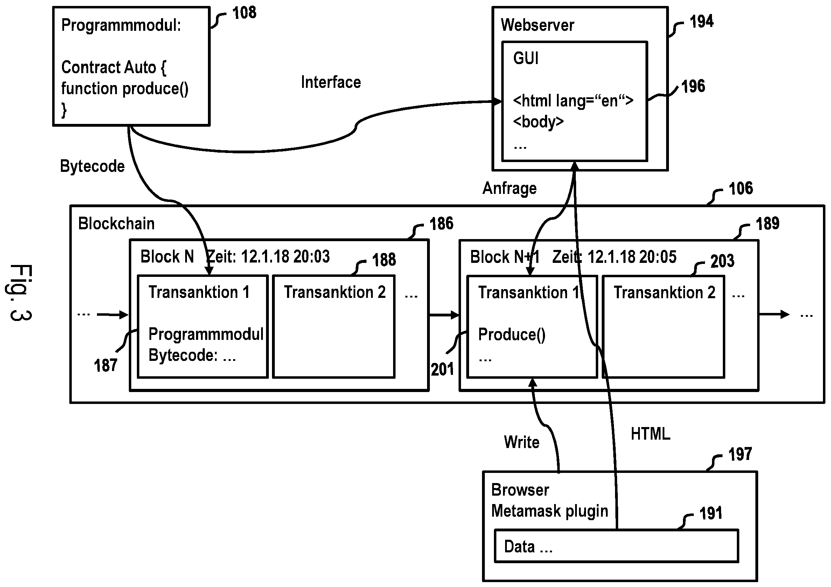

Figur 3 zeigt ein schematisches Blockdiagramm eines Verfahrens zum Auslesen von Daten aus der Blockchain 106. Die Blockchain 106 umfasst beispielsweise die Blöcke 186, 189, wobei einer der Blöcke 186 das Programmmodul 108 umfasst. Das entsprechende Programmmodul wird beispielsweise als Bytecode in einem Eintrag 187 des Blocks 186 gespeichert. Ferner werden in Einträgen 201, 203 weiterer Blöcke 189 der Blockchain 106 Daten zu den Verbrauchsgütern gespeichert. Die entsprechenden Daten umfassen beispielsweise einen Herstellungsdatensatz, der in dem Eintrag 201 gespeichert wird. Durch ein Ausführen des Programmmoduls 108 wird beispielsweise auf einen Webserver 194, welcher beispielsweise von einem Blockchain-Server bereitgestellt werden kann, eine grafische Benutzeroberfläche 196 bereitgestellt, auf die mittels eines Browsers 197 mit Metamask-Plug zugegriffen werden kann. Die entsprechende Benutzeroberfläche wird in dem Browser angezeigt und es kann eine Anfrage zum Schreiben von Daten in einen Block 189 der Blockchain 106 über die grafische Benutzeroberfläche 196 des Webservers 194 unter Verwendung des Programmmoduls 108 gestellt werden. Wird die Eintragungsanfrage positiv beschieden, so werden Daten 191 aus dem Browser 197 in einen Eintrag 101 des Blocks 189 geschrieben. Ebenso können unter Verwendung des Webservers 194 und der grafischen Benutzeroberfläche 196 in dem Block 189 gespeicherte Daten ausgelesen werden.

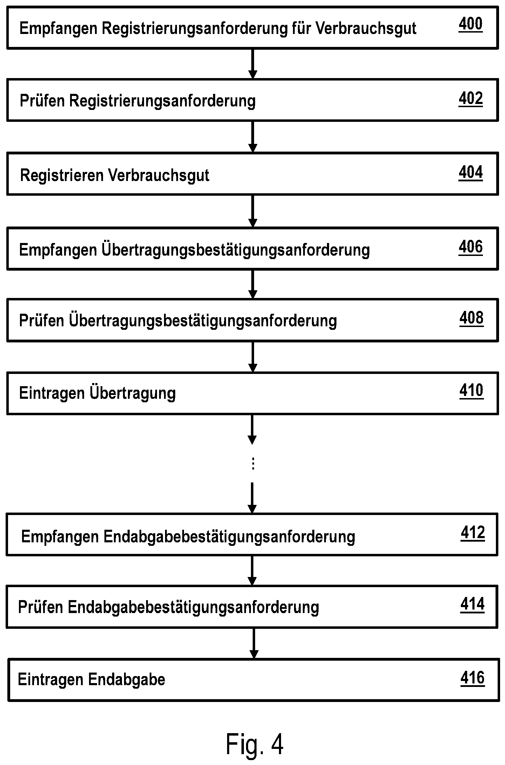

Figur 4 zeigt ein Flussdiagramm einer Ausführungsform eines Verfahrens zur Fälschungssicherung und Abgabekontrolle von Verbrauchsgütern. In Block 400 wird eine Registrierungsanforderung zum Registrieren einer Herstellung eines Verbrauchsguts von einem Blockchain-Server eines Blockchain-Netzwerks empfangen. Der Blockchain-Server führt Programminstruktionen eines von der Blockchain bereitgestellten Programmmoduls aus. In Block 402 erfolgt eine Überprüfung der Registrierungsanforderung. Dabei wird geprüft, ob die Registrierungsanforderung alle von dem Programmmodul vorgegebenen Voraussetzungen für eine Eintragung erfüllt, z. B. eine mit einem in der Blockchain registrierten öffentlichen Schlüssel prüfbare gültige Signatur sowie einen vollständigen Satz von Herstellungsdaten. In Schritt 404 folgt auf eine erfolgreiche Prüfung eine Registrierung des entsprechenden Verbrauchsguts durch Eintragen von Herstellungsdaten in einen zusätzlichen Block der Blockchain. In Block 406 wird eine Übertragungsbestätigungsanforderung von einem Blockchain-Server des Blockchain-Netzwerks empfangen. Die Übertragungsbestätigungsanforderung betrifft eine Bestätigung eines Besitzerwechsels des Verbrauchsguts. Beispielsweise wurde das entsprechende Verbrauchsgut von dem Hersteller an einen Lieferdienst übergeben. Die entsprechende Übertragungsbestätigungsanforderung umfasst beispielsweise einen Nachweis für eine Übertragungsberechtigung des aktuellen Besitzers des Verbrauchsguts, d. h. des Herstellers. Ein entsprechender Herstellerprüfwert wurde beispielsweise als Teil der Herstellungsdaten in Schritt 404 in der Blockchain eingetragen. Unter Verwendung des eingetragenen Herstellerprüfwerts kann in Block 408 eine Prüfung der Übertragungsbestätigungsanforderung erfolgen. Fällt die Prüfung positiv aus, so erfolgt in Block 410 eine Eintragung der Übertragungsbestätigung in die Blockchain. Ein entsprechender Eintrag umfasst beispielsweise einen Prüfwert des neuen Besitzers des Verbrauchsguts, welcher nun die Berechtigungsübertragungen des Verbrauchsguts besitzt. Im Folgenden kann es zu einer ein- oder mehrmaligen Wiederholung der Schritte 406 bis 410 kommen, d. h. zu weiteren Übertragungen des Verbrauchsguts im Zuge der Liefer- bzw. Vertriebskette. Schließlich wird in Block 412 eine Endabgabebstätigungsanforderung empfangen. Wird die Endabgabebestätigungsanforderung empfangen, prüft der Blockchain-Server in Block 416 die empfangene Bestätigungsanforderung auf ihre Gültigkeit. Ist die entsprechende Endabgabebestätigungsanforderung gültig, so wird die entsprechende Endabgabe in Block 416 in die Blockchain eingetragen, wobei der Eintrag in die Blockchain einen Endabgabevermerk umfasst. Der Endabgabevermerk sorgt dafür, dass keine weiteren Übertragungen des entsprechenden Verbrauchsguts in die Blockchain mehr eingetragen werden können.

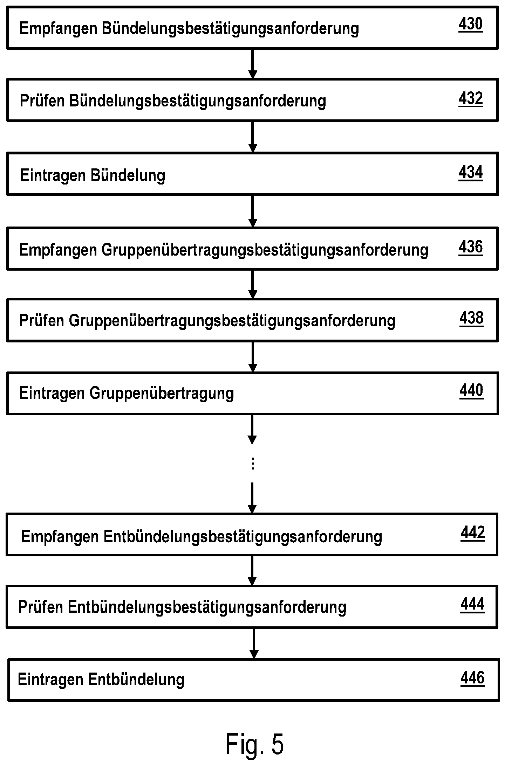

Figur 5 zeigt ein Flussdiagramm einer Ausführungsform einer Bündelung von ein oder mehreren Verbrauchsgütern in einem gemeinsamen Verpackungsbehältnis. Werden ein oder mehrere Verbrauchsgüter als eine Gruppe in einem gemeinsamen Verpackungsverhältnis verpackt, so wird beispielsweise in Block 430 von einem Blockchain-Server des Blockchain-Netzwerks eine Bindungsbestätigungsanforderung empfangen. Diese empfangene Bindungsbestätigungsanforderung wird in Block 432 durch Ausführung von Programminstruktionen des Programmmoduls, welches in die Blockchain der Blockchain-Server zur Verfügung stellt, auf ihre Gültigkeit überprüft. Ist die Bindungsbestätigungsanforderung gültig, weist sie beispielsweise eine gültige Signatur auf und umfasst alle notwendigen Daten bezüglich der Erstellung der Bündelung bzw. des Verpackungsbehältnisses, so erfolgt in Block 434 eine Eintragung der Bindung in die Blockchain. In Block 436 wird eine Gruppenübertragungsbestätigungsanforderung von einem der Blockchain-Server empfangen. Der entsprechende Blockchain-Server überprüft durch Ausführen von Programminstruktionen des Programmmoduls in Block 438 die Gruppenübertragungsbestätigungsanforderung auf ihre Gültigkeit. Hierfür muss beispielsweise eine Übertragungsberechtigung nachgewiesen werden. Erfüllt der Berechtigungsnachweis einen Gruppenprüfwert, welcher im Zuge des Eintrags der Bündelung in Block 434 in die Blockchain eingetragen worden ist, und umfasst die Gruppenübertragungsbestätigungsanforderung zudem alle für eine entsprechende Eintragung notwendigen Informationen, so erfolgt die Eintragung in Block 440. Im Folgenden werden die Blöcke 436 bis 440 gegebenenfalls ein- oder mehrmals wiederholt, falls es zu ein- oder mehrmaligen weiteren Übertragungen der entsprechenden Gruppe bzw. des entsprechenden Verpackungsbehältnisses kommt. In Block 442 wird eine Entbündelungsbestätigungsanforderung empfangen. In Block 444 wird die entsprechende Entbündelungsbestätigungsanforderung von einem Blockchain-Server des Blockchain-Netzwerks unter Verwendung von Programminstruktionen, welche das Programmmodul der Blockchain bereitstellt auf ihre Gültigkeit geprüft. Ist die Entbündelungsbestätigungsanforderung gültig, so erfolgt in Block 446 eine Eintragung der Entbündelung in die Blockchain. Das in Figur 5 gezeigte Verfahren kann beispielsweise im Zuge einer Umverpackung von Verbrauchsgütern, wie etwa verschreibungspflichtigen Medikamenten, ausgeführt werden und/oder im Zuge einer gemeinsamen Lieferung einer Mehrzahl von Verbrauchsgütern, wobei die Anzahl an Verbrauchsgütern größer als die Anzahl an Verbrauchsgütern ist, welche an einen einzelnen Endverbraucher abgegeben werden.

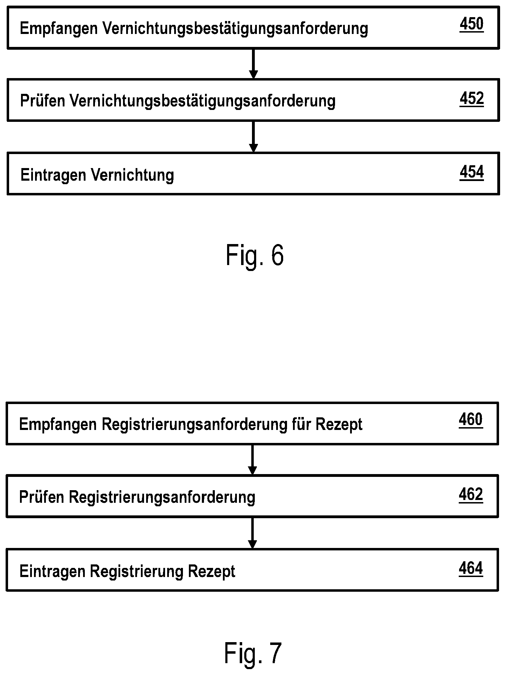

Figur 6 zeigt ein Flussdiagramm eines Verfahrens zum Einträgen einer Vernichtung eines Verbrauchsguts in die Blockchain. In Block 450 wird eine Vernichtungsbestätigungsanforderung von einem der Blockchain-Server empfangen. Der Blockchain-Server prüft in Schritt 452 die Vernichtungsbestätigungsanforderung auf ihre Gültigkeit. Ist die Vernichtungsbestätigungsanforderung gültig, d. h. umfasst sie beispielsweise eine gültige Signatur und alle für eine Gültigkeit notwendigen Angaben, so erfolgt in Block 454 eine Eintragung der Vernichtung in die Blockchain. Mithin kann sichergestellt werden, dass Verbrauchsgüter alle der in der Blockchain manipulationssicher gespeicherten Vertriebsketten von Verbrauchsgütern in sich geschlossen sind, d. h. entweder mit einer Abgabe an einen Endverbraucher oder einer Vernichtung des entsprechenden Verbrauchsguts enden.

Figur 7 zeigt ein Flussdiagramm eines Verfahrens zum Registrieren eines Rezepts für ein verschreibungspflichtiges Medikament in der Blockchain. In Block 460 wird eine Registrierungsanforderung für ein Rezept von einem Blockchain-Server des Blockchain-Netzwerks empfangen. In Block 462 wird geprüft, ob die Registrierungsanforderung alle für eine Registrierung des entsprechenden Rezepts notwendigen Informationen und/oder eine gültige Signatur umfasst. Ist die entsprechende Registrierungsanforderung für das Rezept gültig, so erfolgt in Block 464 eine Eintragung der Registrierung des Rezepts in der Blockchain. Nach Ausführungsformen kann zusätzlich ein Rezept in Papierform ausgestellt werden, welches beispielsweise mit einem maschinenlesbaren Code versehen ist, anhand dessen die für das Rezept relevanten Daten aus der Blockchain identifiziert und ausgelesen werden können. Somit kann anhand der Blockchain die Authentizität des Rezepts überprüft werden.

Nach alternativen Ausführungsformen kann die Ausstellung eines Rezepts auch ausschließlich in der Blockchain erfolgen. Hierzu muss der Endverbraucher bei der Endabgabe des verschreibungspflichtigen Medikaments einen Nachweis vorliegen, dass ihm das Rezept zugeordnet ist. Dies kann beispielsweise durch einen Identifikator, etwa in Form eines Ausweises oder eines signierten Datensatzes erfolgen, wobei der entsprechende Datensatz mit einem dem Rezept zugeordneten privaten kryptografischen Schlüssel signiert ist.

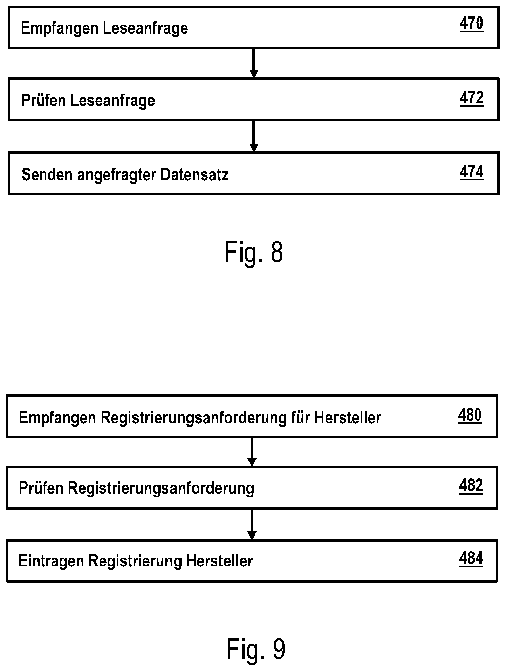

Figur 8 zeigt ein Flussdiagramm eines Verfahrens zum Lesen von Daten aus der Blockchain. In Block 470 wird eine Leseanfrage von einem der Blockchain-Server des Blockchain-Netzwerks empfangen, in Block 472 wird die Leseanfrage auf ihre Gültigkeit unter Verwendung des Programmmoduls der Blockchain geprüft. Ist die Leseanfrage gültig, d. h. umfasst sie beispielsweise eine gültige Signatur oder legt sie einen anderen Gültigkeitsnachweis, wie beispielsweise einen Identifikator eines in der Blockchain protokollierten Verbrauchsguts vor, so wird der angefragte Datensatz in Block 474 in Antwort auf die Leseanfrage an den Anfragenden zurückgesendet.

Figur 9 zeigt ein Verfahren zum Registrieren eines Herstellers von Verbrauchsgütern in der Blockchain. In Block 480 wird eine Registrierungsanforderung für einen Herstellen von einem Blockchain-Server des Blockchain-Netzwerks empfangen, der entsprechende Blockchain-Server führt Programminstruktionen des Programmmoduls aus, wodurch er dazu veranlasst wird, in Block 482 die Registrierungsanforderung auf ihre Gültigkeit zu überprüfen, ist die Registrierungsanforderung gültig, z. B. mit einer gültigen Signatur versehen und/oder umfasst sie alle für eine Registrierung notwendigen Angaben, so erfolgt in Block 484 eine Registrierung des entsprechenden Herstellers. Im Zuge der Registrierung des entsprechenden Herstellers wird beispielsweise ein dem Hersteller zugeordneter öffentlicher kryptografischer Schlüssel in der Blockchain hinterlegt. Mithilfe dieses in der Blockchain hinterlegten öffentlichen kryptografischen Schlüssels können Signaturen eines registrierten Herstellers unter Verwendung der Blockchain auf ihre Gültigkeit geprüft werden. Mit anderen Worten kann eine Blockchain somit ihre eigene PKI implementieren mit einem in dem Programmmodul gespeicherten öffentlichen kryptografischen Schlüssel als Wurzelschlüssel der PKI.

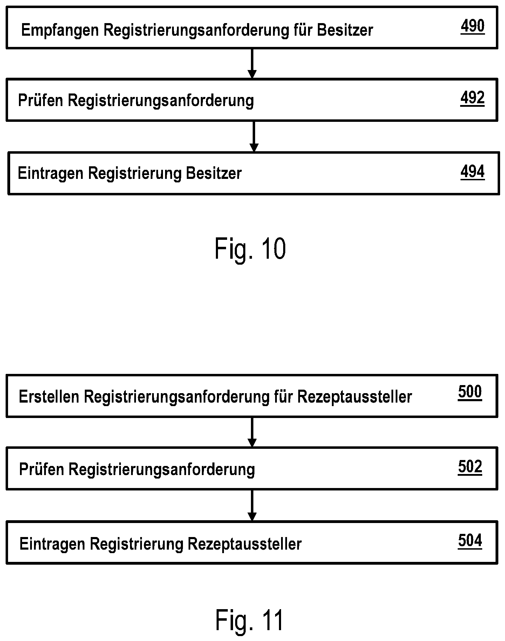

Figur 10 zeigt ein Verfahren zum Registrieren eines potenziellen Besitzers des Verbrauchsguts in der Blockchain. Das Verfahren gemäß Figur 10 entspricht dem Verfahren gemäß Block 490, wobei in Block 490 eine entsprechende Registrierungsanforderung empfangen wird, in Block 492 wird diese Registrierungsanforderung auf ihre Gültigkeit überprüft und im Fall einer Gültigkeit, z. B. einer gültigen Signatur und/oder einem Vorliegen aller notwendigen Daten wird die entsprechende Registrierung in Block 494 in die Blockchain eingetragen.

Figur 11 zeigt schließlich ein Flussdiagramm eines Verfahrens zum Registrieren eines Rezeptausstellers in der Blockchain. Das Verfahren entspricht dem Verfahren der Figuren 9 und 10, wobei in Block 500 eine Registrierungsanforderung zum Registrieren eines Rezeptausstellers in der Blockchain von einem Blockchain-Server des Blockchain-Netzwerks empfangen wird, in Block 552 erfolgt eine Gültigkeitsprüfung der Registrierungsanforderung und in Block 504 auf eine positive Prüfung hin ein Eintrag der entsprechenden Registrierungsdaten des Rezeptausstellers in der Blockchain.

### Bezugszeichenliste

- 100: Blockchain-Netzwerk
- 102: Blockchain-Server
- 104: Speicher
- 106: Blockchain
- 108: Programmmodul
- 110: Voraussetzungen
- 112: öffentlicher Schlüssel
- 114: öffentlicher Schlüssel
- 116: Einträge
- 126: Prozessor
- 128: Kommunikationsschnittstelle
- 132: Blockchain-Server
- 134: Speicher
- 136: Prozessor
- 138: Kommunikationsschnittstelle
- 142: Blockchain-Server
- 144: Speicher
- 146: Prozessor
- 148: Kommunikationsschnittstelle
- 160: Netzwerk
- 170: Account
- 172: Medikament
- 174: maschinenlesbarer Code
- 176: Herstellungsdaten
- 180: Block
- 181: Eintrag
- 182: Herstellungsdaten
- 183: Eintrag
- 184: Block
- 185: Eintrag
- 186: Block
- 187: Eintrag
- 188: Eintrag
- 189: Block
- 190: Daten
- 191: Daten
- 192: Daten
- 194: Webserver
- 196: grafische Benutzeroberfläche
- 197: Browser
- 200: Herstellercomputersystem
- 201: Eintrag
- 202: Speicher
- 203: Eintrag
- 204: geschützter Speicherbereich
- 206: privater Schlüssel
- 208: Anforderung
- 210: Prozessor
- 212: Kommunikationsschnittstelle
- 220: Besitzercomputersystem
- 222: Speicher
- 224: geschützter Speicherbereich
- 226: privater Schlüssel
- 228: Anforderung
- 230: Prozessor
- 232: Kommunikationsschnittstelle
- 240: Besitzercomputersystem
- 242: Speicher
- 244: geschützter Speicherbereich
- 246: privater Schlüssel
- 248: Anforderung
- 250: Prozessor
- 252: Kommunikationsschnittstelle
- 260: Ausstellercomputersystem
- 262: Speicher
- 264: geschützter Speicherbereich
- 266: privater Schlüssel
- 268: Anforderung
- 270: Prozessor
- 272: Kommunikationsschnittstelle
- 300: Registrierungscomputersystem
- 302: Speicher
- 304: geschützter Speicherbereich
- 306: privater Schlüssel
- 308: Registrierungsanforderung
- 310: öffentlicher Schlüssel
- 312: Prozessor
- 314: Kommunikationsschnittstelle
- 320: Computersystem
- 322: Speicher
- 324: geschützter Speicherbereich
- 326: privater Schlüssel
- 328: Leseanfrage
- 330: Datensätze
- 332: Prozessor
- 334: Kommunikationsschnittstelle
- 340: Lesegerät

## Patentansprüche

1. Verfahren zur Fälschungssicherung und Abgabekontrolle von Verbrauchsgütern unter Verwendung einer Blockchain (106), wobei es sich bei den Verbrauchsgütern um Medikamente (172) handelt, wobei die Blockchain (106) für jedes der Verbrauchsgüter eine kryptografisch gesicherte Historie speichert, wobei die Blockchain (106) von einem Blockchain-Netzwerk (100) bereitgestellt wird, welches eine Mehrzahl von Blockchain-Servern (102, 132, 142) umfasst, wobei die Blockchain-Server (102, 132, 142) jeweils dazu konfiguriert sind, unter Verwendung eines von der Blockchain (106) umfassten Programmmoduls (108) zusätzliche Blöcke für die Blockchain (106) zu erstellen und zu der Blockchain (106) hinzuzufügen, wobei jedes in der Blockchain (106) registrierte Verbrauchsgut mit einem maschinenlesbaren Code (174) versehen ist, wobei der maschinenlesbare Code (174) einen Identifikator des entsprechenden Verbrauchsguts umfasst,
wobei das Verfahren zur kryptografisch gesicherten Speicherung einer Historie eines ersten Verbrauchsgutes umfasst:
• im Fall einer Herstellung des ersten Verbrauchsgutes, Empfangen einer ersten Registrierungsanforderung (208) zum Registrieren des ersten Verbrauchsgutes in der Blockchain (106) durch einen der Blockchain-Server (102, 132, 142) des Blockchain-Netzwerks (100), wobei die erste Registrierungsanforderung (208) einen Herstellerdatensatz (182) mit Herstellungsdaten (176) des ersten Verbrauchsgutes und mit einem Herstellerprüfwert umfasst, wobei der Herstellerprüfwert zur Prüfung einer Berechtigung zur Übertragung des ersten Verbrauchsgutes durch einen Hersteller des ersten Verbrauchsgutes dient,
• Ausführen erster Programminstruktionen des Programmoduls (108) durch den die erste Registrierungsanforderung (208) empfangenden Blockchain-Server (102, 132, 142), wobei das Ausführen der ersten Programminstruktionen eine Prüfung umfasst, ob die erste Registrierungsanforderung (208) ein oder mehrere erste Voraussetzungen (110) erfüllen, und, falls die ersten Voraussetzungen (110) erfüllt sind, ein Hinzufügen des Herstellerdatensatzes (182) zu einem Eintrag (116, 181,201) in einem zusätzlichen Block (180, 189) der Blockchain (106), wodurch das erste Verbrauchsgut in der Blockchain (106) registriert, der Herstellerprüfwert dem ersten Verbrauchsgut als ein aktueller Prüfwert zugeordnet und dem Hersteller als einem aktuellen Besitzer des ersten Verbrauchsgutes die Berechtigung zur Übertragung des ersten Verbrauchsgutes zugeordnet wird,
ferner umfasst das Verfahren ein oder mehrmals:
• im Fall eines Besitzerwechsels des ersten Verbrauchsgutes, Empfangen einer Übertragungsbestätigungsanforderung (208, 228, 248) zur Bestätigung des Besitzerwechsels des ersten Verbrauchsgutes durch einen der Blockchain-Server (102, 132, 142) des Blockchain-Netzwerks (100), wobei die Übertragungsbestätigungsanforderung (208, 228, 248) einen Identifikator des ersten Verbrauchsgut, einen Berechtigungsnachweis des aktuellen Besitzers vor dem Besitzerwechsel zur Übertragung des ersten Verbrauchsgutes und einen weiteren Prüfwert umfasst, wobei der weitere Prüfwert zur zukünftigen Prüfung einer Berechtigung zur Übertragung des ersten Verbrauchsgutes durch einen Empfänger des ersten Verbrauchsgutes als zukünftigem Besitzers dient,
• Ausführen zweiter Programminstruktionen des Programmoduls (108) durch den die Übertragungsbestätigungsanforderung (208, 228, 248) empfangenden Blockchain-Server (102, 132, 142), wobei das Ausführen der zweiten Programminstruktionen eine Prüfung unter Verwendung des aktuellen Prüfwerts umfasst, ob die Übertragungsbestätigungsanforderung (208, 228, 248) ein oder mehrere zweite Voraussetzungen (110) erfüllt, und, falls die zweiten Voraussetzungen (110) erfüllt sind, ein Hinzufügen des weiteren Prüfwerts und des Identifikators des ersten Verbrauchsgutes zu einem Eintrag (116, 185) in einem zusätzlichen Block (184) der Blockchain (106), wodurch der weitere Prüfwert dem ersten Verbrauchsgut als aktueller Prüfwert in Ersatz zu dem bisherigen aktuellen Prüfwert zugeordnet wird und wobei dem weiteren Besitzer als aktuellem Besitzer des ersten Verbrauchsgutes in Ersatz zu dem bisherigen aktuellen Besitzer die Berechtigung zur Übertragung des ersten Verbrauchsgutes zugeordnet wird,
ferner umfasst das Verfahren:
• im Fall einer Endabgabe des ersten Verbrauchsgutes an einen Endverbraucher, Empfangen einer Endabgabebestätigungsanforderung (228, 248) zur Bestätigung der Endabgabe des ersten Verbrauchsgutes durch einen der Blockchain-Server (102, 132, 142) des Blockchain-Netzwerks (100), wobei die Endabgabebestätigungsanforderung (228, 248) den Identifikator des ersten Verbrauchsgutes und einen Berechtigungsnachweis des aktuellen Besitzers vor der Endabgabe zur Übertragung des ersten Verbrauchsgutes umfasst,
• Ausführen dritter Programminstruktionen des Programmoduls (108) durch den die Endabgabebestätigungsanforderung (228, 248) empfangenden Blockchain-Server (102, 132, 142), wobei das Ausführen der dritten Programminstruktionen eine Prüfung unter Verwendung des aktuellen Prüfwerts umfasst, ob die Endabgabebestätigungsanforderung (228, 248) des aktuellen Besitzers ein oder mehrere dritte Voraussetzungen (110) erfüllt, und, falls die dritten Voraussetzungen (110) erfüllt sind, ein Hinzufügen des Identifikators des ersten Verbrauchsgutes und eines Endabgabevermerk zu einem Eintrag (116) in einem zusätzlichen Block der Blockchain (106), wobei der Endabgabevermerk anstatt eines neuen Prüfwerts eingetragen wird, wodurch der Endabgabevermerk dem ersten Verbrauchsgut zugeordnet wird, wobei der Endabgabevermerk festlegt, dass das erste Verbrauchsgut von weiteren Übertragungen ausgeschlossen ist,
wobei das Verfahren ferner umfasst:
• im Fall eines Ausstellens eines Rezepts für ein erstes Medikament (172), Empfangen einer zweiten Registrierungsanforderung (268) zum Registrieren des ersten Rezepts in der Blockchain (106) durch einen der Blockchain-Server (102, 132, 142) des Blockchain-Netzwerks (100), wobei die zweite Registrierungsanforderung (268) einen Austellerdatensatz mit Ausstellungsdaten des Rezepts umfasst, wobei die Ausstellungdaten einen Rezeptidentifikator umfassen, wobei es sich bei dem Rezept um ein Rezept für ein erstes Medikament (172) in Form des ersten Verbrauchsguts handelt,
• Ausführen achter Programminstruktionen des Programmoduls (108) durch den die zweite Registrierungsanforderung (268) empfangenden Blockchain-Server (102, 132, 142), wobei das Ausführen der achten Programminstruktionen eine Prüfung umfasst, ob die zweite Registrierungsanforderung (268) ein oder mehrere achte Voraussetzungen (110) erfüllen, und, falls die achten Voraussetzungen (110) erfüllt sind, ein Hinzufügen des Ausstellerdatensatzes zu einem Eintrag (116) in einem zusätzlichen Block der Blockchain (106), wodurch das erste Rezept in der Blockchain (106) registriert wird,
• im Fall der Endabgabe des ersten Verbrauchsgutes an den Endverbraucher, falls die dritten Voraussetzungen (110) durch die Endabgabebestätigungsanforderung (268) zur Bestätigung der Endabgabe des ersten Verbrauchsgutes erfüllt sind, Hinzufügen des Rezeptidentifikators zu dem Eintrag (116) in dem zusätzlichen Block der Blockchain (106) mit dem Identifikator des ersten Verbrauchsguts und dem Endabgabevermerk,
wobei das in der Blockchain (106) registrierte Rezept mit einem maschinenlesbaren Code versehen ist, wobei der maschinenlesbare Code des Rezepts einen Identifikator des Rezepts umfasst,
wobei die zweite Registrierungsanforderung (268) zum Registrieren des Rezepts einen Endabgabeprüfwert umfasst, wobei der Endabgabeprüfwert zur Prüfung einer Berechtigung zur Endabgabe eines Medikaments (172) gemäß dem Rezept dient,
wobei der maschinenlesbare Code des Rezepts einen Nachweis zur Berechtigung zur Endabgabe eines Medikaments (172) gemäß dem Rezept umfasst,
wobei die im Fall der Endabgabe des ersten Verbrauchsgutes an den Endverbraucher empfangene Endabgabebestätigungsanforderung (228, 248) den von dem ersten Rezept maschinengelesenen Code umfasst,
wobei eine der achten Voraussetzungen (110) eine gültige Signatur der zweiten Registrierungsanforderung (268) ist und wobei das Ausführen der achten Programminstruktionen das Prüfen der Signatur der zweiten Registrierungsanforderung (268) unter Verwendung eines dritten öffentlichen kryptografischen Schlüssel (114) des Ausstellers des Rezepts umfasst,
wobei der dritte öffentliche kryptografische Schlüssel (114) des Ausstellers des Rezepts zur Prüfung der Signatur der zweiten Registrierungsanforderung (268) von der Blockchain (106) zur Verfügung gestellt wird.

2. Verfahren nach Anspruch 1, wobei das Verfahren ferner umfasst:
• im Fall einer Vernichtung eines zweiten in der Blockchain (106) registrierten Verbrauchsgutes, Empfangen einer Vernichtungsbestätigungsanforderung zur Bestätigung der Vernichtung des zweiten Verbrauchsgutes durch einen der Blockchain-Server (102, 132, 142) des Blockchain-Netzwerks (100), wobei die Vernichtungsbestätigungsanforderung den Identifikator des zweiten Verbrauchsgutes und einen Berechtigungsnachweis des aktuellen Besitzers vor der Vernichtung zur Übertragung des zweiten Verbrauchsgutes umfasst,
• Ausführen vierter Programminstruktionen des Programmoduls (108) durch den die Vernichtungsbestätigungsanforderung empfangenden Blockchain-Server (102, 132, 142), wobei das Ausführen der vierten Programminstruktionen eine Prüfung unter Verwendung des aktuellen Prüfwerts umfasst, ob die Vernichtungsbestätigungsanforderung des aktuellen Besitzers ein oder mehrere vierte Voraussetzungen (110) erfüllt, und, falls die vierten Voraussetzungen (110) erfüllt sind, ein Hinzufügen des Identifikators des zweiten Verbrauchsgutes und eines Vernichtungsvermerks zu einem Eintrag (116) in einem zusätzlichen Block der Blockchain (106), wodurch der Vernichtungsvermerks dem zweiten Verbrauchsgut zugeordnet wird, wobei der Vernichtungsvermerk die Vernichtung des zweiten Verbrauchsgutes bestätigt und festlegt, dass das zweite Verbrauchsgut von weiteren Übertragungen ausgeschlossen ist, und/oder
wobei den in der Blockchain (106) registrierten Verbrauchsgütern jeweils maximal ein aktueller Prüfwert zugeordnet ist, bei welchem es sich jeweils um den dem entsprechenden Verbrauchsgut zuletzt zugordneten Prüfwert handelt.

3. Verfahren nach Anspruch nach einem der vorangehenden Ansprüche, wobei die Herstellerprüfwerte der in der Blockchain (106) registrierten Verbrauchsgüter eindeutig sind für die einzelnen Verbrauchsgüter und jeweils als Identifikator der entsprechenden Verbrauchsgüter verwendet werden oder
wobei ein Herstellerprüfwert für mehrere der in der Blockchain (106) registrierten Verbrauchsgüter verwendet wird und wobei die individuellen Identifikatoren der in der Blockchain (106) registrierten Verbrauchsgüter unabhängig von den Herstellerprüfwerten sind.

4. Verfahren nach einem der vorangehenden Ansprüche, wobei die Herstellerprüfwerte jeweils aus einem herstellerindividuellen Seed Key abgeleitet und unter Verwendung des herstellerindividuellen Seed Keys auf ihre Gültigkeit überprüfbar sind.

5. Verfahren nach einem der vorangehenden Ansprüche, wobei die Prüfwerte jeweils aus einem besitzerindividuellen Seed Key abgeleitet und unter Verwendung des besitzerindividuellen Seed Keys auf ihre Gültigkeit überprüfbar sind, und/oder
wobei die Herstellungsdaten (176) des ersten Verbrauchsgutes ein oder mehrere der folgenden Angaben umfassen: eine Bezeichnung des ersten Verbrauchsgutes, eine Bezeichnung des Hersteller des ersten Verbrauchsguts, eine Artikelnummer des ersten Verbrauchsguts, einen Status des ersten Verbrauchsguts, ein Verfalldatum des ersten Verbrauchsguts, und/oder
wobei die Endabgabebestätigungsanforderung (228, 248) einen Identifikator des Endverbrauchers umfasst und das Ausführen der dritten Programminstruktionen des Programmoduls (108), falls die dritten Voraussetzungen (110) erfüllt sind, in Ergänzung zu dem Identifikator des ersten Verbrauchsgutes und dem Endabgabevermerk ein Hinzufügen des Identifikator des Endverbrauchers zu dem entsprechenden Eintrag (116) in dem entsprechenden zusätzlichen Block der Blockchain (106) umfasst, wodurch der Identifikator des ersten Verbrauchsgutes dem Identifikator des Endverbrauchers zugeordnet wird.

6. Verfahren nach einem der Ansprüche 2 bis 5, wobei die Vernichtungsbestätigungsanforderung Identifikatoren von ein oder mehreren Zeugen der Vernichtung des zweiten Verbrauchsgutes umfasst und das Ausführen der vierten Programm instruktionen des Programmoduls (108), falls die vierten Voraussetzungen (110) erfüllt sind, in Ergänzung zu dem Identifikator des zweiten Verbrauchsgutes und dem Vernichtungsvermerks ein Hinzufügen der Identifikatoren der ein oder mehreren Zeugen zu dem entsprechenden Eintrag (116) in dem entsprechenden zusätzlichen Block der Blockchain (106) umfasst, wodurch die Identifikatoren der ein oder mehreren Zeugen dem Vernichtungsvermerk des zweiten Verbrauchsgutes zugeordnet werden.

7. Verfahren nach einem der vorangehenden Ansprüche, wobei eine der ersten Voraussetzungen (110) eine gültige Signatur des Herstellerdatensatzes (182) ist und wobei das Ausführen der ersten Programminstruktionen das Prüfen der Signatur des Herstellerdatensatzes (182) unter Verwendung eines ersten öffentlichen kryptografischen Schlüssel (114) des Herstellers des ersten Verbrauchsgutes umfasst.
wobei beispielsweise der erste öffentliche kryptografische Schlüssel des Herstellers (114) zur Prüfung der Signatur des Herstellerdatensatzes (182) von der Blockchain (106) zur Verfügung gestellt wird.

8. Verfahren nach einem der vorangehenden Ansprüche, wobei das Verfahren ferner umfasst:
• im Fall eines Verpackens einer Gruppe von in der Blockchain (106) registrierten Verbrauchsgütern in einem gemeinsamen Verpackungsbehältnis, Empfangen einer Bündelungsbestätigungsanforderung (208) zur Bestätigung einer Bündelung von Berechtigungen zur Übertragung der Verbrauchsgütern der Gruppe von Verbrauchsgütern, wobei die Bündelungsbestätigungsanforderung (208) einen Identifikatoren jedes Verbrauchsgüter der Gruppe von Verbrauchsgütern, einen Berechtigungsnachweis des aktuellen Besitzers jedes Verbrauchsgüter der Gruppe von Verbrauchsgütern vor der Verpackung zur Übertragung des entsprechenden Verbrauchsgutes, einen Gruppenidentifikator und einen Gruppenerstellerprüfwert umfasst, wobei der Gruppenerstellerprüfwert zur Prüfung einer Berechtigung zur Übertragung der Verbrauchsgüter der Gruppe von Verbrauchsgütern zusammen als Gruppe durch einen Gruppenersteller als einem aktuellen Gruppenbesitzer der Gruppe von Verbrauchsgütern dient,
• Ausführen fünfter Programminstruktionen des Programmoduls (108) durch den die Bündelungsbestätigungsanforderung (208) empfangenden Blockchain-Server (102, 132, 142), wobei das Ausführen der fünften Programm instruktionen eine Prüfung unter Verwendung der aktuellen Prüfwerte der Verbrauchsgüter der Gruppe von Verbrauchsgütern umfasst, ob die Bündelungsbestätigungsanforderung (208) ein oder mehrere fünfte Voraussetzungen (110) erfüllt, und, falls die fünften Voraussetzungen (110) erfüllt sind, ein Hinzufügen des Gruppenerstellerprüfwerts, des Gruppenidentifikators und der Identifikatoren der Verbrauchsgüter der Gruppe von Verbrauchsgütern zu einem Eintrag (116) in einem zusätzlichen Block (180, 189) der Blockchain (106), wodurch eine Bündelung der Verbrauchsgüter der Gruppe von Verbrauchsgütern in der Blockchain(106) registriert wird, wodurch der Gruppenerstellerprüfwert den Verbrauchsgütern der Gruppe von Verbrauchsgütern als gemeinsamer aktueller Prüfwert in Ersatz zu den bisherigen aktuellen Prüfwerten der einzelnen Verbrauchsgüter der Gruppe von Verbrauchsgütern zugeordnet wird und wobei dem Gruppenersteller als aktuellem Gruppenbesitzer der Gruppe von Verbrauchsgütern in Ersatz zu dem bisherigen aktuellen Besitzer der einzelnen Verbrauchsgüter der Gruppe von Verbrauchsgütern die Berechtigung zur Übertragung der Verbrauchsgüter der Gruppe von Verbrauchsgütern zusammen als Gruppe zugeordnet wird.

9. Verfahren nach Anspruch 8, wobei das Verfahren ferner umfasst:
• im Fall eines Besitzerwechsels des Verpackungsbehältnisses mit der Gruppe von Verbrauchsgütern, Empfangen einer Gruppenübertragungsbestätigungsanforderung (208, 228, 248) zur Übertragung der Gruppe durch einen der Blockchain-Server (102, 132, 142) des Blockchain-Netzwerks (100), wobei die Gruppenübertragungsbestätigungsanforderung (208, 228, 248) den Gruppenidentifikator, einen Berechtigungsnachweis des aktuellen Gruppenbesitzers vor dem Besitzerwechsel zur Übertragung der Gruppe von Verbrauchsgütern und einen weiteren Gruppenprüfwert umfasst, wobei der weitere Gruppenprüfwert zur Prüfung einer Berechtigung zur Übertragung der Gruppe von Verbrauchsgütern durch einen Empfänger der Gruppe von Verbrauchsgütern als zukünftigem Gruppenbesitzer dient,
• Ausführen sechster Programminstruktionen des Programmoduls (108) durch den die Gruppenübertragungsbestätigungsanforderung (208, 228, 248) empfangenden Blockchain-Server (102, 132, 142), wobei das Ausführen der sechsten Programminstruktionen eine Prüfung unter Verwendung des aktuellen Gruppenprüfwerts umfasst, ob die Gruppenübertragungsbestätigungsanforderung (208, 228, 248) ein oder mehrere sechste Voraussetzungen (110) erfüllt, und, falls die sechsten Voraussetzungen (110) erfüllt sind, ein Hinzufügen des weiteren Gruppenprüfwerts und des Gruppenidentifikators einem Eintrag (116) in einem zusätzlichen Block (184) der Blockchain (106), wodurch der weitere Gruppenprüfwert dem ersten Verbrauchsgut als aktueller Gruppenprüfwert in Ersatz zu dem bisherigen aktuellen Gruppenprüfwert zugeordnet wird und wobei dem weiteren Gruppenbesitzer als aktuellem Gruppenbesitzer in Ersatz zu dem bisherigen aktuellen Gruppenbesitzer die Berechtigung zur Übertragung der Gruppe von Verbrauchsgutes zugeordnet wird, und/oder
wobei das Verfahren ferner umfasst:
• im Fall eines Entpackens des Verpackungsbehältnisses, Empfangen einer Entbündelungsbestätigungsanforderung (228, 248) zur Bestätigung einer Auflösung der Bündelung der Berechtigungen zur Übertragung der Verbrauchsgüter der Gruppe von Verbrauchsgütern, wobei die Entbündelungsbestätigungsanforderung (228, 248) den Gruppenidentifikator der Gruppe, einen Berechtigungsnachweis des aktuellen Gruppenbesitzers der Gruppe vor dem Entpacken und einen oder mehrere weitere Prüfwerte zur Prüfung einer Berechtigung zur Übertragung der Verbrauchsgütern der Gruppe von Verbrauchsgütern durch einen zukünftigen Besitzer der Verbrauchsgüter der Gruppe von Verbrauchsgütern dient,
• Ausführen siebter Programminstruktionen des Programmoduls (108) durch den die Entbündelungsbestätigungsanforderung (228, 248) empfangenden Blockchain-Server (102, 132, 142), wobei das Ausführen der siebten Programminstruktionen eine Prüfung unter Verwendung der aktuellen Gruppenprüfwerts umfasst, ob die Entbündelungsbestätigungsanforderung (228, 248) ein oder mehrere siebte Voraussetzungen (110) erfüllt, und, falls die siebten Voraussetzungen (110) erfüllt sind, ein Hinzufügen der ein oder mehreren weiteren Prüfwerte und der Identifikatoren der Verbrauchsgüter der aufgelösten Gruppe von Verbrauchsgütern zu ein oder mehreren Einträgen (116) in ein oder mehreren zusätzlichen Blöcken der Blockchain (106), wodurch die ein oder mehreren weiteren Prüfwerte jeweils ein oder mehreren der Verbrauchsgüter der aufgelösten Gruppe von Verbrauchsgütern als aktuelle Prüfwert in Ersatz zu dem gemeinsamen Gruppenprüfwert zugeordnet werden und wobei dem bisherigen aktuellen Gruppenbesitzer als aktuellem Besitzer der einzelnen Verbrauchsgüter der aufgelösten Gruppe von Verbrauchsgütern die Berechtigung zur individuellen Übertragung der einzelnen Verbrauchsgüter der aufgelösten Gruppe von Verbrauchsgütern zugeordnet wird, und/oder
wobei den in der Blockchain (106) registrierten Gruppen von Verbrauchsgütern maximal ein aktueller Gruppenprüfwert zugeordnet ist, bei welchem es sich jeweils um den der entsprechenden Gruppe zuletzt zugordneten Gruppenprüfwert handelt, und/oder
wobei die Gruppenerstellerprüfwerte der in der Blockchain (106) registrierten Gruppen von Verbrauchsgüter eindeutig sind für die einzelnen Gruppen und jeweils als Gruppenidentifikatoren der entsprechenden Gruppen von Verbrauchsgütern verwendet werden, und/oder
wobei ein Gruppenerstellerprüfwerte für mehrere der in der Blockchain (106) registrierten Gruppen von Verbrauchsgüter verwendet wird und wobei die individuellen Gruppenidentifikatoren der in der Blockchain (106) registrierten Gruppen unabhängig von den Gruppenerstellerprüfwerten sind, und/oder
wobei es sich bei den Gruppenprüfwerten jeweils um gruppenindividuelle Gruppenprüfwerte handelt, und/oder
wobei ein Gruppenprüfwert für mehrere Gruppen von Verbrauchsgütern verwendet wird, und/oder
wobei die Gruppenerstellerprüfwerte jeweils aus einem gruppenerstellerindividuellen Seed Key abgeleitet und unter Verwendung des gruppenerstellerindividuellen Seed Keys auf ihre Gültigkeit überprüfbar sind, und/oder
wobei jedes Verpackungsbehältnis einer in der Blockchain (106) registrierten Gruppe von Verbrauchsgütern mit einem maschinenlesbaren Code (174) versehen ist, wobei der maschinenlesbare Code (174) einen Gruppenidentifikator der entsprechenden Gruppe umfasst, und/oder
wobei eine der fünften Voraussetzungen (110) eine gültige Signatur der Bündelungsbestätigungsanforderung (208) ist und wobei das Ausführen der fünften Programminstruktionen das Prüfen der Signatur der Bündelungsbestätigungsanforderung (208) unter Verwendung eines zweiten öffentlichen kryptografischen Schlüssels (114) des Gruppenerstellers der Gruppe von Verbrauchsgütern umfasst,
wobei beispielsweise der zweite öffentliche kryptografische Schlüssel (114) des Gruppenerstellers zur Prüfung der Signatur der Bündelungsbestätigungsanforderung (208) von der Blockchain (106) zur Verfügung gestellt wird.

10. Verfahren nach einem der vorangehenden Ansprüche, wobei es sich bei den Medikamenten (172) um Betäubungsmittel handelt und
wobei es sich bei den Rezepten um Betäubungsmittelrezepte handelt.

11. Verfahren nach einem der vorangehenden Ansprüche, wobei das Verfahren ferner umfasst:
• Empfangen einer dritten Registrierungsanforderung (308) zum Registrieren eines Herstellers von Verbrauchsgütern durch einen Blockchain-Server (102, 132, 142), wobei die dritte Registrierungsanforderung (308) signiert ist,
• Ausführen neunter Programminstruktionen des Programmoduls (108) durch den die dritte Registrierungsanforderung (308) empfangenden Blockchain-Server (102, 132, 142), wobei die Ausführung der neunten Programminstruktionen eine Prüfung unter Verwendung eines vierten öffentlichen kryptografischen Schlüssels (112, 114), ob die Signatur der dritten Registrierungsanforderung (308) gültig ist, und, falls die Signatur der dritten Registrierungsanforderung (308) gültig ist, ein Registrieren des Herstellers in der Blockchain (106), wobei das Registrieren ein Hinzufügen eines Herstelleridentifikators zu einem Eintrag (116) in einem zusätzlichen Blocks zu der Blockchain (106) umfasst.
wobei beispielsweise der vierte öffentliche kryptografische Schlüssel (112, 114) zur Prüfung der Signatur der dritten Registrierungsanforderung (308) von der Blockchain (106) zur Verfügung gestellt wird.

12. Verfahren nach einem der vorangehenden Ansprüche, wobei das Verfahren ferner umfasst:
• Empfangen einer vierten Registrierungsanforderung (308) zum Registrieren eines potentiellen Besitzers durch einen Blockchain-Server (102, 132, 142), wobei die vierte Registrierungsanforderung (308) signiert ist,
• Ausführen zehnter Programminstruktionen des Programmoduls (108) durch den die vierte Registrierungsanforderung (308) empfangenden Blockchain-Server (102, 132, 142), wobei die Ausführung der zehnten Programminstruktionen eine Prüfung unter Verwendung eines fünften öffentlichen kryptografischen Schlüssels (112, 114), ob die Signatur der vierten Registrierungsanforderung (308) gültig ist, und, falls die Signatur der vierten Registrierungsanforderung (308) gültig ist, ein Registrieren des potentiellen Besitzers in der Blockchain (106), wobei das Registrieren ein Hinzufügen eines Identifikators des potentiellen Besitzers zu einem Eintrag (116) in einem zusätzlichen Blocks zu der Blockchain (106) umfasst.
wobei beispielsweise der fünfte öffentliche kryptografische Schlüssel (112, 114) zur Prüfung der Signatur der dritten Registrierungsanforderung (308) von der Blockchain (106) zur Verfügung gestellt wird.

13. Verfahren nach einem der vorangehenden Ansprüche, wobei das Verfahren ferner umfasst:
• Empfangen einer fünften Registrierungsanforderung (308) zum Registrieren eines Ausstellers von Rezepten durch einen Blockchain-Server (102, 132, 142), wobei die fünfte Registrierungsanforderung (308) signiert ist,
• Ausführen elfter Programminstruktionen des Programmoduls (108) durch den die fünfte Registrierungsanforderung (308) empfangenden Blockchain-Server (102, 132, 142), wobei die Ausführung der elften Programminstruktionen eine Prüfung unter Verwendung eines sechsten öffentlichen kryptografischen Schlüssels (112, 114), ob die Signatur der fünften Registrierungsanforderung (308) gültig ist, und, falls die Signatur der fünften Registrierungsanforderung (308) gültig ist, ein Registrieren des Austellers in der Blockchain (106), wobei das Registrieren ein Hinzufügen eines Ausstelleridentifikators zu einem Eintrag (116) in einem zusätzlichen Blocks zu der Blockchain (106) umfasst.
wobei beispielsweise der sechste öffentliche kryptografische Schlüssel (112, 114) zur Prüfung der Signatur der fünften Registrierungsanforderung (308) von der Blockchain (106) zur Verfügung gestellt wird.

14. Verfahren nach einem der vorangehenden Ansprüche, wobei das Verfahren ferner umfasst:
• Empfangen einer Leseanfrage (328) zum Lesen von Einträgen (330) zu dem ersten Verbrauchsgut durch einen der Blockchain-Server (102, 132, 142), wobei die Leseanfrage (328) einen Identifikator des ersten Verbrauchsgutes umfasst,
• Ausführen zwölfter Programminstruktionen des Programmoduls (108) durch den die Leseanfrage (328) empfangenden Blockchain-Server (102, 132, 142), wobei die Ausführung der zwölften Programminstruktionen eine Prüfung durch den Blockchain-Server (102, 132, 142) umfasst, ob das erste Verbrauchsgut in der Blockchain (106) registriert ist, und, falls das erste Verbrauchsgut in der Blockchain (106) registriert ist, ein Senden von Kopien der Einträge (330) zu dem ersten Verbrauchsgut an einen Absender der Leseanfrage (328).
wobei beispielsweise die Einträge (330) zu dem ersten Verbrauchsgut einen Identifikator des Endverbrauchers und/oder einen Ausstellungsdatensatz eines dem ersten Verbrauchsgut zugeordneten Rezepts umfassen, wobei die Prüfung eine Signaturprüfung der Leseanfrage (328) unter Verwendung eines siebten öffentlichen Schlüssels (114) umfasst,
welcher zur Prüfung der Signatur der Leseanfrage (328) beispielsweise von der Blockchain (106) zur Verfügung gestellt wird.

15. Blockchain-Netzwerk (100) mit Mehrzahl von Blockchain-Servern (102, 132, 142) zur Fälschungssicherung und Abgabekontrolle von Verbrauchsgütern unter Verwendung einer von dem Blockchain-Netzwerk (100) bereitgestellten Blockchain (106), wobei es sich bei den Verbrauchsgütern um Medikamente (172) handelt, wobei die Blockchain (106) für jedes der Verbrauchsgüter eine kryptografisch gesicherte Historie speichert, wobei die Blockchain-Server (102, 132, 142) jeweils dazu konfiguriert sind, unter Verwendung eines von der Blockchain (106) umfassten Programmmoduls (108) zusätzliche Blöcke für die Blockchain (106) zu erstellen und zu der Blockchain (106) hinzuzufügen, wobei jedes in der Blockchain (106) registrierte Verbrauchsgut mit einem maschinenlesbaren Code (174) versehen ist, wobei der maschinenlesbare Code (174) einen Identifikator des entsprechenden Verbrauchsguts umfasst,
wobei das Blockchain-Netzwerk (100) zur kryptografisch gesicherten Speicherung einer Historie eines ersten Verbrauchsgutes konfiguriert ist zum:
• im Fall einer Herstellung des ersten Verbrauchsgutes, Empfangen einer ersten Registrierungsanforderung (208) zum Registrieren des ersten Verbrauchsgutes in der Blockchain (106) durch einen der Blockchain-Server (102, 132, 142) des Blockchain-Netzwerks (100), wobei die erste Registrierungsanforderung (208) einen Herstellerdatensatz (182) mit Herstellungsdaten (176) des ersten Verbrauchsgutes und mit einem Herstellerprüfwert umfasst, wobei der Herstellerprüfwert zur Prüfung einer Berechtigung zur Übertragung des ersten Verbrauchsgutes durch einen Hersteller des ersten Verbrauchsgutes dient,
• Ausführen erster Programminstruktionen des Programmoduls (108) durch den die erste Registrierungsanforderung (208) empfangenden Blockchain-Server (102, 132, 142), wobei das Ausführen der ersten Programminstruktionen eine Prüfung umfasst, ob die erste Registrierungsanforderung (208) ein oder mehrere erste Voraussetzungen (110) erfüllen, und, falls die ersten Voraussetzungen (110) erfüllt sind, ein Hinzufügen des Herstellerdatensatzes (182) zu einem Eintrag (116, 181, 201) in einem zusätzlichen Block (180, 189) der Blockchain (106), wodurch das erste Verbrauchsgut in der Blockchain (106) registriert, der Herstellerprüfwert dem ersten Verbrauchsgut als ein aktueller Prüfwert zugeordnet und dem Hersteller als einem aktuellen Besitzer des ersten Verbrauchsgutes die Berechtigung zur Übertragung des ersten Verbrauchsgutes zugeordnet wird,
ferner ist das Blockchain-Netzwerk (100) konfiguriert zum ein- oder mehrmaligen:
• im Fall eines Besitzerwechsels des ersten Verbrauchsgutes, Empfangen einer Übertragungsbestätigungsanforderung (208, 228, 248) zur Bestätigung des Besitzerwechsels des ersten Verbrauchsgutes durch einen der Blockchain-Server (102, 132, 142) des Blockchain-Netzwerks (100), wobei die Übertragungsbestätigungsanforderung (208, 228, 248) einen Identifikator des ersten Verbrauchsgut, einen Berechtigungsnachweis des aktuellen Besitzers vor dem Besitzerwechsel zur Übertragung des ersten Verbrauchsgutes und einen weiteren Prüfwert umfasst, wobei der weitere Prüfwert zur zukünftigen Prüfung einer Berechtigung zur Übertragung des ersten Verbrauchsgutes durch einen Empfänger des ersten Verbrauchsgutes als zukünftigem Besitzers dient,
• Ausführen zweiter Programminstruktionen des Programmoduls (108) durch den die Übertragungsbestätigungsanforderung (208, 228, 248) empfangenden Blockchain-Server (102, 132, 142), wobei das Ausführen der zweiten Programminstruktionen eine Prüfung unter Verwendung des aktuellen Prüfwerts umfasst, ob die Übertragungsbestätigungsanforderung (208, 228, 248) ein oder mehrere zweite Voraussetzungen (110) erfüllt, und, falls die zweiten Voraussetzungen (110) erfüllt sind, ein Hinzufügen des weiteren Prüfwerts und des Identifikators des ersten Verbrauchsgutes zu einem Eintrag (116, 185) in einem zusätzlichen Block (184) der Blockchain (106), wodurch der weitere Prüfwert dem ersten Verbrauchsgut als aktueller Prüfwert in Ersatz zu dem bisherigen aktuellen Prüfwert zugeordnet wird und wobei dem weiteren Besitzer als aktuellem Besitzer des ersten Verbrauchsgutes in Ersatz zu dem bisherigen aktuellen Besitzer die Berechtigung zur Übertragung des ersten Verbrauchsgutes zugeordnet wird,
ferner ist das Blockchain-Netzwerk (100) konfiguriert zum:
• im Fall einer Endabgabe des ersten Verbrauchsgutes an einen Endverbraucher, Empfangen einer Endabgabebestätigungsanforderung (228, 248) zur Bestätigung der Endabgabe des ersten Verbrauchsgutes durch einen der Blockchain-Server (102, 132, 142) des Blockchain-Netzwerks (100), wobei die Endabgabebestätigungsanforderung (228, 248) den Identifikator des ersten Verbrauchsgutes und einen Berechtigungsnachweis des aktuellen Besitzers vor der Endabgabe zur Übertragung des ersten Verbrauchsgutes umfasst,
• Ausführen dritter Programminstruktionen des Programmoduls (108) durch den die Endabgabebestätigungsanforderung (228, 248) empfangenden Blockchain-Server (102, 132, 142), wobei das Ausführen der dritten Programminstruktionen eine Prüfung unter Verwendung des aktuellen Prüfwerts umfasst, ob die Endabgabebestätigungsanforderung (228, 248) des aktuellen Besitzers ein oder mehrere dritte Voraussetzungen (110) erfüllt, und, falls die dritten Voraussetzungen (110) erfüllt sind, ein Hinzufügen des Identifikators des ersten Verbrauchsgutes und eines Endabgabevermerk zu einem Eintrag (116) in einem zusätzlichen Block der Blockchain (106), wobei der Endabgabevermerk anstatt eines neuen Prüfwerts eingetragen wird, wodurch der Endabgabevermerk dem ersten Verbrauchsgut zugeordnet wird, wobei der Endabgabevermerk festlegt, dass das erste Verbrauchsgut von weiteren Übertragungen ausgeschlossen ist,
ferner ist das Blockchain-Netzwerk (100) konfiguriert:
• im Fall eines Ausstellens eines Rezepts für ein erstes Medikament (172), Empfangen einer zweiten Registrierungsanforderung (268) zum Registrieren des ersten Rezepts in der Blockchain (106) durch einen der Blockchain-Server (102, 132, 142) des Blockchain-Netzwerks (100), wobei die zweite Registrierungsanforderung (268) einen Austellerdatensatz mit Ausstellungsdaten des Rezepts umfasst, wobei die Ausstellungdaten einen Rezeptidentifikator umfassen, wobei es sich bei dem Rezept um ein Rezept für ein erstes Medikament (172) in Form des ersten Verbrauchsguts handelt,
• Ausführen achter Programminstruktionen des Programmoduls (108) durch den die zweite Registrierungsanforderung (268) empfangenden Blockchain-Server (102, 132, 142), wobei das Ausführen der achten Programminstruktionen eine Prüfung umfasst, ob die zweite Registrierungsanforderung (268) ein oder mehrere achte Voraussetzungen (110) erfüllen, und, falls die achten Voraussetzungen (110) erfüllt sind, ein Hinzufügen des Ausstellerdatensatzes zu einem Eintrag (116) in einem zusätzlichen Block der Blockchain (106), wodurch das erste Rezept in der Blockchain (106) registriert wird,
• im Fall der Endabgabe des ersten Verbrauchsgutes an den Endverbraucher, falls die dritten Voraussetzungen (110) durch die Endabgabebestätigungsanforderung (268) zur Bestätigung der Endabgabe des ersten Verbrauchsgutes erfüllt sind, Hinzufügen des Rezeptidentifikators zu dem Eintrag (116) in dem zusätzlichen Block der Blockchain (106) mit dem Identifikator des ersten Verbrauchsguts und dem Endabgabevermerk,
wobei das in der Blockchain (106) registrierte Rezept mit einem maschinenlesbaren Code versehen ist, wobei der maschinenlesbare Code des Rezepts einen Identifikator des Rezepts umfasst,
wobei die zweite Registrierungsanforderung (268) zum Registrieren des Rezepts einen Endabgabeprüfwert umfasst, wobei der Endabgabeprüfwert zur Prüfung einer Berechtigung zur Endabgabe eines Medikaments (172) gemäß dem Rezept dient,
wobei der maschinenlesbare Code des Rezepts einen Nachweis zur Berechtigung zur Endabgabe eines Medikaments (172) gemäß dem Rezept umfasst,
wobei die im Fall der Endabgabe des ersten Verbrauchsgutes an den Endverbraucher empfangene Endabgabebestätigungsanforderung (228, 248) den von dem ersten Rezept maschinengelesenen Code umfasst,
wobei eine der achten Voraussetzungen (110) eine gültige Signatur der zweiten Registrierungsanforderung (268) ist und wobei das Ausführen der achten Programminstruktionen das Prüfen der Signatur der zweiten Registrierungsanforderung (268) unter Verwendung eines dritten öffentlichen kryptografischen Schlüssel (114) des Ausstellers des Rezepts umfasst,
wobei der dritte öffentliche kryptografische Schlüssel (114) des Ausstellers des Rezepts zur Prüfung der Signatur der zweiten Registrierungsanforderung (268) von der Blockchain (106) zur Verfügung gestellt wird.

## Claims

1. A method for counterfeit protection and for the controlled dispensing of consumables using a blockchain (106), the consumables being pharmaceuticals (172), the blockchain (106) storing a cryptographically protected history for each of the consumables, the blockchain (106) being provided by a blockchain network (100), which comprises a plurality of blockchain servers (102, 132, 142), the blockchain servers (102, 132, 142) in each case being configured to create additional blocks for the blockchain (106), using a program module (108) encompassed by the blockchain (106), and to add these to the blockchain (106), each consumable registered in the blockchain (106) being provided with a machine-readable code (174), and the machine-readable code (174) comprising an identifier of the corresponding consumable,
the method for storing a history of a first consumable in a cryptographically protected manner comprising:
• in the case of a production of the first consumable, receiving a first registration request (208) for registering the first consumable in the blockchain (106) by one of the blockchain servers (102, 132, 142) of the blockchain network (100), the first registration request (208) comprising a manufacturer data set (182) including production data (176) of the first consumable and including a manufacturer check value, and the manufacturer check value being used to check an authorization for transferring the first consumable by a manufacturer of the first consumable;
• executing first program instructions of the program module (108) by the blockchain server (102, 132, 142) receiving the first registration request (208), the execution of the first program instructions comprising a check as to whether the first registration request (208) meets one or more first requirements (110), and, if the first requirements (110) are met, adding the manufacturer data set (182) to an entry (116, 181, 201) in an additional block (180, 189) of the blockchain (106), whereby the first consumable is registered in the blockchain (106), the manufacturer check value is assigned to the first consumable as a current check value, and the authorization for transferring the first consumable is assigned to the manufacturer as a current owner of the first consumable;
the method furthermore comprising once or multiple times:
• in the case of a change in owner of the first consumable, receiving a transfer confirmation request (208, 228, 248) for confirming the change in owner of the first consumable by one of the blockchain servers (102, 132, 142) of the blockchain network (100), the transfer confirmation request (208, 228, 248) comprising an identifier of the first consumable, a proof of authorization of the current owner, prior to the change in owner, for transferring the first consumable, and a further check value, and the further check value being used to check, in the future, an authorization for transferring the first consumable by a recipient of the first consumable as the future owner;
• executing second program instructions of the program module (108) by the blockchain server (102, 132, 142) receiving the transfer confirmation request (208, 228, 248), the execution of the second program instructions comprising a check, using the current check value, as to whether the transfer confirmation request (208, 228, 248) meets one or more second requirements (110), and, if the second requirements (110) are met, adding the further check value and the identifier of the first consumable to an entry (116, 185) in an additional block (184) of the blockchain (106), whereby the further check value is assigned to the first consumable as a current check value, in place of the existing current check value, and the authorization for transferring the first consumable is assigned to the further owner as the current owner of the first consumable, in place of the existing current owner;
the method furthermore comprising:
• in the case of final dispensing of the first consumable to an end user, receiving a final dispensing confirmation request (228, 248) for confirming the final dispensing of the first consumable by one of the blockchain servers (102, 132, 142) of the blockchain network (100), the final dispensing confirmation request (228, 248) comprising the identifier of the first consumable and a proof of authorization of the current owner, prior to the final dispensing, for transferring the first consumable;
• executing third program instructions of the program module (108) by the blockchain server (102, 132, 142) receiving the final dispensing confirmation request (228, 248), the execution of the third program instructions comprising a check, using the current check value, as to whether the final dispensing confirmation request (228, 248) of the current owner meets one or more third requirements (110), and, if the third requirements (110) are met, adding the identifier of the first consumable and a final dispensing annotation to an entry (116) in an additional block of the blockchain (106), the final dispensing annotation being entered instead of a new check value, whereby the final dispensing annotation is assigned to the first consumable, and the final dispensing annotation establishing that the first consumable is precluded from further transfers;
the method furthermore comprising:
• in the case that a prescription is issued for a first pharmaceutical (172), receiving a second registration request (268) for registering the first prescription in the blockchain (106) by one of the blockchain servers (102, 132, 142) of the blockchain network (100), the second registration request (268) comprising an issuer data set including issue data (176) of the prescription, the issue data comprising a prescription identifier, and the prescription being a prescription for a first pharmaceutical (172) in form of the first consumable;
• executing eighth program instructions of the program module (108) by the blockchain server (102, 132, 142) receiving the second registration request (268), the execution of the eighth program instructions comprising a check as to whether the second registration request (268) meets one or more eighth requirements (110), and, if the eighth requirements (110) are met, adding the issuer data set to an entry (116) in an additional block of the blockchain (106), whereby the first prescription is registered in the blockchain (106),
• in the case of final dispensing of the first consumable to the end user, if the third requirements (110) by the final dispensing confirmation request (268) for confirming the final dispensing of the first consumable are met, adding the prescription identifier to the entry (116) in the additional block of the blockchain (106) with the identifier of the first consumable and the final dispensing annotation;
the prescription registered in the blockchain (106) being provided with a machine-readable code, and the machine-readable code of the prescription comprising an identifier of the prescription,
the second registration request (268) for registering the prescription comprising a final dispensing check value, and the final dispensing check value being used to check an authorization for the final dispensing of a pharmaceutical (172) according to the prescription,
the machine-readable code of the prescription comprising a proof of authorization for the final dispensing of a pharmaceutical (172) according the prescription,
the final dispensing confirmation request (228, 248) received in the case of the final dispensing of the first consumable to an end user comprising the code that is machine-read from the first prescription,
one of the eighth requirements (110) being a valid signature of the second registration request (268), and the execution of the eighth program instructions comprising checking the signature of the second registration request (268), using a third public cryptographic key (114) of the issuer of the prescription,
the third public cryptographic key (114) of the issuer of the prescription being made available by the blockchain (106) for checking the signature of the second registration request (268).

2. The method according to claim 1, the method furthermore comprising:
• in the case of a destruction of a second consumable registered in the blockchain (106), receiving a destruction confirmation request for confirming the destruction of the second consumable by one of the blockchain servers (102, 132, 142) of the blockchain network (100), the destruction confirmation request comprising the identifier of the second consumable and a proof of authorization of the current owner, prior to the destruction, for transferring the second consumable;
• executing fourth program instructions of the program module (108) by the blockchain server (102, 132, 142) receiving the destruction confirmation request, the execution of the fourth program instructions comprising a check, using the current check value, as to whether the destruction confirmation request of the current owner meets one or more fourth requirements (110), and, if the fourth requirements (110) are met, adding the identifier of the second consumable and a destruction annotation to an entry (116) in an additional block of the blockchain (106), whereby the destruction annotation is assigned to the second consumable, and the destruction annotation confirming the destruction of the second consumable and establishing that the second consumable is precluded from further transfers, and/or
in each case no more than one current check value being assigned to the consumables registered in the blockchain (106), which in each case is the check value most recently assigned to the corresponding consumable.

3. The method according to claim according to any one of the preceding claims, wherein the manufacturer check values of the consumables registered in the blockchain (106) are unambiguous for the individual consumables, and in each case are used as identifiers of the corresponding consumables, or
wherein a manufacturer check value is used for a plurality of the consumables registered in the blockchain (106), and the individual identifiers of the consumables stored in the blockchain (106) are independent of the manufacturer check values.

4. The method according to any one of the preceding claims, wherein the manufacturer check values are in each case derived from a manufacturer-specific seed key and, using the manufacturer-specific seed key, can be checked for the validity thereof.

5. The method according to any one of the preceding claims, wherein the check values are in each case derived from an owner-specific seed key and, using the owner-specific seed key, can be checked for the validity thereof, and/or
wherein the manufacturer data (176) of the first consumable comprises one or more of the following pieces of information: a designation of the first consumable, a designation of the manufacturer of the first consumable, an item number of the first consumable, a status of the first consumable, an expiration date of the first consumable, and/or
wherein the final dispensing confirmation request (228, 248) comprises an identifier of the end user, and the execution of the third program instructions of the program module (108), if the third requirements (110) are met, in addition to the identifier of the first consumable and the final dispensing annotation, comprises adding the identifier of the end user to the corresponding entry (116) in the corresponding additional block of the blockchain (106), whereby the identifier of the first consumable is assigned to the identifier of the end user.

6. The method according to any one of claims 2 to 5, wherein the destruction confirmation request comprises identifiers of one or more witnesses of the destruction of the second consumable, and the execution of the fourth program instructions of the program module (108), if the fourth requirements (110) are met, in addition to the identifier of the second consumable and the destruction annotation, comprises adding the identifiers of the one or more witnesses to the corresponding entry (116) in the corresponding additional block of the blockchain (106), whereby the identifiers of the one or more witnesses are assigned to the destruction annotation of the second consumable.

7. The method according to any one of the preceding claims, wherein one of the first requirements (110) is a valid signature of the manufacturer data set (182), and the execution of the first program instructions comprises checking the signature of the manufacturer data set (182), using a first public cryptographic key (114) of the manufacturer of the first consumable,
the first public cryptographic key of the manufacturer (114), for example, being made available by the blockchain (106) for checking the signature of the manufacturer data set (182).

8. The method according to any one of the preceding claims, the method furthermore comprising:
• in the case that a group of consumables registered in the blockchain (106) is packaged in a shared packaging container, receiving a bundling confirmation request (208) for confirming a bundling of authorizations for transferring the consumables of the group of consumables, the bundling confirmation request (208) comprising an identifier of each consumable of the group of consumables, a proof of authorization of the current owner of each consumable of the group of consumables, prior to packaging, for transferring the corresponding consumable, a group identifier, and a group creator check value, the group creator check value being used to check an authorization for transferring the consumables of the group of consumables together, as a group, by a group creator as a current group owner of the group of consumables;
• executing fifth program instructions of the program module (108) by the blockchain server (102, 132, 142) receiving the bundling confirmation request (208, the execution of the fifth program instructions comprising a check, using the current check values of the consumables of the group of consumables, as to whether the bundling confirmation request (208) meets one or more fifth requirements (110), and, if the fifth requirements (110) are met, adding the group creator check value, the group identifier, and the identifiers of the consumable of the group of consumables to an entry (116) in an additional block (180, 189) of the blockchain (106), whereby a bundling of the consumables of the group of consumables is registered in the blockchain (106), whereby the group creator check value is assigned to the consumables of the group of consumables as a shared current check value, in place of the existing current check values of the individual consumables of the group of consumables, and the authorization for transferring the consumables of the group of consumables together, as a group, is assigned to the group creator as the current group owner of the group of consumables, in place of the existing current owner of the individual consumables of the group of consumables.

9. The method according to claim 8, the method furthermore comprising:
• in the case of a change in owner of the packaging container including the group of consumables, receiving a group transfer confirmation request (208, 228, 248) for transferring the group by one of the blockchain servers (102, 132, 142) of the blockchain network (100), the group transfer confirmation request (208, 228, 248) comprising the group identifier, a proof of authorization of the current group owner, prior to the change in owner, for transferring the group of consumables, and a further check value, and the further group check value being used to check an authorization for transferring the group of consumables by a recipient of the group of consumables as the future group owner;
• executing sixth program instructions of the program module (108) by the blockchain server (102, 132, 142) receiving the group transfer confirmation request (208, 228, 248), the execution of the sixth program instructions comprising a check, using the current group check value, as to whether the group transfer confirmation request (208, 228, 248) meets one or more sixth requirements (110), and, if the sixth requirements (110) are met, adding the further group check value and the group identifier to an entry (116) in an additional block (184) of the blockchain (106), whereby the further group check value is assigned to the first consumable as the current group check value, in place of the existing current group check value, and the authorization for transferring the group of consumables is assigned to the further group owner as the current group owner, in place of the existing current group owner; and/or
the method furthermore comprising:
• in the case of unpacking of the packaging container, receiving an unbundling confirmation request (228, 248) for confirming a dissolution of the bundling of the authorizations for transferring the consumables of the group of consumables, the unbundling confirmation request (228, 248) comprising the group identifier of the group, a proof of authorization of the current group owner of the group, prior to unpacking, and one or more further check values for checking an authorization for transferring the consumables of the group of consumable by a future owner of the consumables of the group of consumables;
• executing seventh program instructions of the program module (108) by the blockchain server (102, 132, 142) receiving the unbundling confirmation request (208, 248), the execution of the seventh program instructions comprising a check, using the current group check value, as to whether the unbundling confirmation request (228, 248) meets one or more seventh requirements (110), and, if the seventh requirements (110) are met, adding the one or more check values and the identifiers of the consumables of the dissolved group of consumables to one or more entries (116) in one or more additional blocks of the blockchain (106), whereby the one or more further check values in each case are assigned to one or more of the consumables of the dissolved group of consumables as a current check value, in place of the shared group check value, and the authorization for individually transferring the individual consumables of the dissolved group of consumables is assigned to the existing current group owner as the current owner of the individual consumables of the dissolved group of consumables; and/or
no more than one current group check value being assigned to the groups of consumables registered in the blockchain (106), which in each case is the group check value most recently assigned to the corresponding group, and/or
the group creator check values of the groups of consumables registered in the blockchain (106) being unambiguous for the individual groups, and in each case being used as group identifiers of the corresponding groups of consumables, and/or
a group creator check value being used for a plurality of groups of consumables registered in the blockchain (106), and the individual group identifiers of the groups registered in the blockchain (106) being independent of the group manufacturer check values, and/or
the group check values in each case being group-specific group check values, and/or
a group check value being used for a plurality of groups of consumables, and/or
the group creator check values in each case being derived from a group creator-specific seed key and, using the group creator-specific seed key, being checkable for the validity thereof, and/or
each packaging container of a group of consumables registered in the blockchain (106) being provided with a machine-readable code (174), the machine-readable code (174) comprising a group identifier of the corresponding group, and/or
one of the firth requirements (110) being a valid signature of the bundling confirmation request (208), and the execution of the fifth program instructions comprising checking the signature of the bundling confirmation request (208), using a second public cryptographic key (114) of the group creator of the group of consumables, and
the second public cryptographic key (114) of the group creator, for example, being made available by the blockchain (106) for checking the signature of the bundling confirmation request (208).

10. The method according to any one of the preceding claims, wherein the pharmaceutical (172) is a narcotic, and
the prescriptions are prescriptions for narcotics.

11. The method according to any one of the preceding claims, the method furthermore comprising:
• receiving a third registration request (308) for registering a manufacturer of consumables by a blockchain server (102, 132, 142), the third registration request (308) being signed,
• executing ninth program instructions of the program module (108) by the blockchain server (102, 132, 142) receiving the third registration request (308), the execution of the ninth program instructions comprising a check, using a fourth public cryptographic key (112, 114), as to whether the signature of the third registration request (308) is valid, and, if the signature of the third registration request (308) is valid, registering the manufacturer in the blockchain (106), the registration comprising adding a manufacturer identifier to an entry (116) in an additional block of the blockchain (106),
the fourth public cryptographic key (112, 114), for example, being made available by the blockchain (106) for checking the signature of the third registration request (308).

12. The method according to any one of the preceding claims, the method furthermore comprising:
• receiving a fourth registration request (308) for registering a potential owner by a blockchain server (102, 132, 142), the fourth registration request (308) being signed;
• executing tenth program instructions of the program module (108) by the blockchain server (102, 132, 142) receiving the fourth registration request (308), the execution of the tenth program instructions comprising a check, using a fifth public cryptographic key (112, 114), as to whether the signature of the fourth registration request (308) is valid, and, if the signature of the fourth registration request (308) is valid, registering the potential owner in the blockchain (106), the registration comprising adding an identifier of the potential owner to an entry (116) in an additional block of the blockchain (106),
the fifth public cryptographic key (112, 114), for example, being made available by the blockchain (106) for checking the signature of the third registration request (308).

13. The method according to any one of the preceding claims, the method furthermore comprising:
• receiving a fifth registration request (308) for registering an issuer of prescriptions by a blockchain server (102, 132, 142), the fifth registration request (308) being signed;
• executing eleventh program instructions of the program module (108) by the blockchain server (102, 132, 142) receiving the fifth registration request (308), the execution of the eleventh program instructions comprising a check, using a sixth public cryptographic key (112, 114), as to whether the signature of the fifth registration request (308) is valid, and, if the signature of the fifth registration request (308) is valid, registering the issuer in the blockchain (106), the registration comprising adding an issuer identifier to an entry (116) in an additional block of the blockchain (106),
the sixth public cryptographic key (112, 114), for example, being made available by the blockchain (106) for checking the signature of the fifth registration request (308).

14. The method according to any one of the preceding claims, the method furthermore comprising:
• receiving a read inquiry (328) for reading entries (330) regarding the first consumable by one of the blockchain servers (102, 132, 142), the read request (328) comprising an identifier of the first consumable;
• executing twelfth program instructions of the program module (108) by the blockchain server (102, 132, 142) receiving the read inquiry (328), the execution of the twelfth program instructions comprising a check by the blockchain server (102, 132, 142) as to whether the first consumable is registered in the blockchain (106), and, if the first consumable is registered in the blockchain (106), transmitting copies of the entries (330) regarding the first consumable to a sender of the read inquiry (328),
the entries (330) regarding the first consumable, for example, comprising an identifier of the end user and/or an issue data set of a prescription assigned to the first consumable, the check including a signature check of the read inquiry (328), using a seventh public key (114),
which is made available, for example, by the blockchain (106) for checking the signature of the read inquiry (328).

15. A blockchain network (100) comprising a plurality of blockchain servers (102, 132, 142) for counterfeit protection and for the controlled dispensing of consumables using a blockchain (106) provided by the blockchain network (100), the consumables being pharmaceuticals (172), the blockchain (106) storing a cryptographically protected history for each of the consumables, the blockchain servers (102, 132, 142) in each case being configured to create additional blocks for the blockchain (106), using a program module (108) encompassed by the blockchain (106), and to add these to the blockchain (106), each consumable registered in the blockchain (106) being provided with a machine-readable code (174), and the machine-readable code (174) comprising an identifier of the corresponding consumable,
the blockchain network (100) being configured to store a history of a first consumable in a cryptographically protected manner, so as to:
• in the case of a production of the first consumable, receive a first registration request (208) for registering the first consumable in the blockchain (106) by one of the blockchain servers (102, 132, 142) of the blockchain network (100), the first registration request (208) comprising a manufacturer data set (182) including production data (176) of the first consumable and including a manufacturer check value, and the manufacturer check value being used to check an authorization for transferring the first consumable by a manufacturer of the first consumable;
• execute first program instructions of the program module (108) by the blockchain server (102, 132, 142) receiving the first registration request (208), the execution of the first program instructions comprising a check as to whether the first registration request (208) meets one or more first requirements (110), and, if the first requirements (110) are met, adding the manufacturer data set (182) to an entry (116, 181, 201) in an additional block (180, 189) of the blockchain (106), whereby the first consumable is registered in the blockchain (106), the manufacturer check value is assigned to the first consumable as a current check value, and the authorization for transferring the first consumable is assigned to the manufacturer as a current owner of the first consumable,
the blockchain network (100) furthermore being configured to, once or multiple times:
• in the case of a change in owner of the first consumable, receive a transfer confirmation request (208, 228, 248) for confirming the change in owner of the first consumable by one of the blockchain servers (102, 132, 142) of the blockchain network (100), the transfer confirmation request (208, 228, 248) comprising an identifier of the first consumable, a proof of authorization of the current owner, prior to the change in owner, for transferring the first consumable, and a further check value, and the further check value being used to check, in the future, an authorization for transferring the first consumable by a recipient of the first consumable as the future owner;
• execute second program instructions of the program module (108) by the blockchain server (102, 132, 142) receiving the transfer confirmation request (208, 228, 248), the execution of the second program instructions comprising a check, using the current check value, as to whether the transfer confirmation request (208, 228, 248) meets one or more second requirements (110), and, if the second requirements (110) are met, adding the further check value and the identifier of the first consumable to an entry (116, 185) in an additional block (184) of the blockchain (106), whereby the further check value is assigned to the first consumable as the current check value, in place of the existing current check value, and the authorization for transferring the first consumable is assigned to the further owner as the current owner of the first consumable, in place of the existing current owner,
the blockchain network (100) furthermore being configured to:
• in the case of final dispensing of the first consumable to an end user, receive a final dispensing confirmation request (228, 248) for confirming the final dispensing of the first consumable by one of the blockchain servers (102, 132, 142) of the blockchain network (100), the final dispensing confirmation request (228, 248) comprising the identifier of the first consumable and a proof of authorization of the current owner, prior to the final dispensing, for transferring the first consumable;
• execute third program instructions of the program module (108) by the blockchain server (102, 132, 142) receiving the final dispensing confirmation request (228, 248), the execution of the third program instructions comprising a check, using the current check value, as to whether the final dispensing confirmation request (228, 248) of the current owner meets one or more third requirements (110), and, if the third requirements (110) are met, adding the identifier of the first consumable and a final dispensing annotation to an entry (116) in an additional block of the blockchain (106), the final dispensing annotation being entered instead of a new check value, whereby the final dispensing annotation is assigned to the first consumable, the final dispensing annotation establishing that the first consumable is precluded from further transfers,
the blockchain network (100) furthermore being configured to:
• in the case that a prescription for a first pharmaceutical (172) is issued, receive a second registration request (268) for registering the first prescription in the blockchain (106) by one of the blockchain servers (102, 132, 142) of the blockchain network (100), the second registration request (268) comprising an issuer data set including issue data of the prescription, the issue data comprising a prescription identifier, and the prescription being a prescription for a first pharmaceutical (172) in form of the first consumable;
• execute eighth program instructions of the program module (108) by the blockchain server (102, 132, 142) receiving the second registration request (268), the execution of the eighth program instructions comprising a check as to whether the second registration request (268) meets one or more eighth requirements (110), and, if the eighth requirements (110) are met, adding the issuer data set to an entry (116) in an additional block of the blockchain (106), whereby the first prescription is registered in the blockchain (106);
• in the case of the final dispensing of the first consumable to the end user, if the third requirements (110) are met by the final dispensing confirmation request (268) for confirming the final dispensing of the first consumable, add the prescription identifier to the entry (116) in the additional block of the blockchain (106) with the identifier of the first consumable and the final dispensing annotation,
the prescription registered in the blockchain (106) being provided with a machine-readable code, and the machine-readable code of the prescription comprising an identifier of the prescription,
the second registration request (268) for registering the prescription comprising a final dispensing check value, the final dispensing check value being used to check an authorization for the final dispensing of a pharmaceutical (172) according to the prescription,
the machine-readable code of the prescription comprising a proof of authorization for the final dispensing of a pharmaceutical (172) according the prescription,
the final dispensing confirmation request (228, 248) received in the case of the final dispensing of the first consumable to the end user comprising the code that is machine-read from the first prescription,
one of the eighth requirements (110) being a valid signature of the second registration request (268), and the execution of the eighth program instructions comprising checking the signature of the second registration request (268), using a third public cryptographic key (114) of the issuer of the prescription, and
the third public cryptographic key (114) of the issuer of the prescription being made available by the blockchain (106) for checking the signature of the second registration request (268).

## Revendications

1. Procédé de protection contre la falsification et de contrôle de la livraison de biens de consommation, moyennant l'utilisation d'une chaîne de blocs (106), où il s'agit, dans le cas des biens de consommations, de médicaments (172), dans lequel la chaîne de blocs (106) enregistre un historique sécurisé de manière cryptographique pour chacun des biens de consommation, où la chaîne de blocs (106) est fournie par un réseau de chaînes de blocs (100), lequel comprend une multiplicité de serveurs de chaînes de blocs (102, 132, 142), où les serveurs de chaînes de blocs (102, 132, 142) sont chacun configurés pour, moyennant l'emploi d'un module de programme (108) compris par la chaîne de blocs (106), établir des blocs supplémentaires pour la chaîne de blocs (106) et les ajouter à la chaîne de blocs (106), où chaque bien de consommation enregistré dans la chaine de blocs (106) est muni d'un code (174) lisible par machine, où le code (174) lisible par machine comprend un identificateur du bien de consommation correspondant,
le procédé pour le stockage sécurisé par cryptographie comprenant un historique d'un premier bien de consommation :
• dans le cas d'une production du premier bien de consommation, la réception d'une première demande d'enregistrement (208) pour enregistrer le premier bien de consommation dans la chaîne de blocs (106) par l'un des serveurs de chaîne de blocs (102, 132, 142) du réseau de chaînes de blocs (100), où la première demande d'enregistrement (208) comprend un ensemble de données de fabricant (182) avec des données de fabrication (176) du premier bien de consommation et avec une valeur de vérification de fabricant, où la valeur de vérification de fabricant sert à vérifier une autorisation pour la transmission du premier bien de consommation par un fabricant du premier bien de consommation,
• l'exécution de premières instructions de programme du module de programme (108) par le serveur de chaînes de blocs (102, 132, 142) recevant la première demande d'enregistrement (208), où l'exécution des premières instructions de programme comprend une vérification si la première demande d'enregistrement (208) satisfait une ou plusieurs premières conditions (110), et, si les premières conditions (110) sont satisfaites, un ajout de l'ensemble de données de fabricant (182) à une entrée (116, 181, 201) dans un bloc supplémentaire (180, 189) de la chaîne de blocs (106), ce par quoi le premier bien de consommation est enregistré dans la chaîne de blocs (106), la valeur de vérification de fabricant est associée au premier bien de consommation en tant que valeur de vérification actuelle et l'autorisation pour la transmission du premier bien de consommation devient associée au fabricant en tant que propriétaire actuel du premier bien de consommation,
le procédé comprenant, en outre, une ou plusieurs fois :
• dans le cas d'un changement de propriétaire du premier bien de consommation, la réception d'une demande de confirmation de transmission (208, 228, 248) pour la confirmation du changement de propriétaire du premier bien de consommation par un des serveurs de chaînes de blocs (102, 132, 142) du réseau de chaines de blocs (100), où la demande de confirmation de transmission (208, 228, 248) comprend un identificateur du premier bien de consommation, une preuve d'autorisation du propriétaire actuel avant le changement de propriétaire pour la transmission du premier bien de consommation et une autre valeur de vérification, où l'autre valeur de vérification sert à la vérification future d'une autorisation pour la transmission du premier bien de consommation par un réceptionnaire du premier bien de consommation en tant que futur propriétaire,
• l'exécution de deuxièmes instructions de programme du module de programme (108) par le serveur de chaînes de blocs (102, 132, 142) recevant la demande de confirmation de transmission (208, 228, 248), où l'exécution des deuxièmes instructions de programme comprend une vérification, moyennant l'emploi de la valeur de vérification actuelle, si la demande de confirmation de transmission (208, 228, 248) satisfait une ou plusieurs deuxièmes conditions (110), et, si les deuxièmes conditions (110) sont satisfaites, un ajout de l'autre valeur de vérification et de l'identificateur du premier bien de consommation à une entrée (116, 185) dans un bloc supplémentaire (184) de la chaîne de blocs (106), ce par quoi l'autre valeur de vérification est associée au premier bien de consommation en tant que valeur de vérification actuelle en remplacement de la valeur de vérification actuelle précédente et où l'autorisation pour la transmission du premier bien de consommation est associée à l'autre propriétaire en tant que propriétaire actuel du premier bien de consommation en remplacement du propriétaire actuel précédent,
le procédé comprenant en outre :
• dans le cas d'une livraison finale du premier bien de consommation à un consommateur final, la réception d'une demande de confirmation de livraison finale (228, 248) pour la confirmation de la livraison finale du premier bien de consommation par un des serveurs de chaînes de blocs (102, 132, 142) du réseau de chaines de blocs (100), où la demande de confirmation de livraison finale (228, 248) comprend l'identificateur du premier bien de consommation et une preuve d'autorisation du propriétaire actuel avant la livraison finale pour la transmission du premier bien de consommation,
• l'exécution de troisièmes instructions de programme du module de programme (108) par le serveur de chaînes de blocs (102, 132, 142) recevant la demande de confirmation de livraison finale (228, 248), où l'exécution des troisièmes instructions de programme comprend une vérification, moyennant l'emploi de la valeur de vérification actuelle, si la demande de confirmation de livraison finale (228, 248) du propriétaire actuel satisfait une ou plusieurs troisièmes conditions (110), et, si les troisièmes conditions (110) sont satisfaites, un ajout de l'identificateur du premier bien de consommation et d'une note de livraison finale à une entrée (116) dans un bloc supplémentaire de la chaîne de blocs (106), où la note de livraison finale est enregistrée à la place d'une nouvelle valeur de vérification, ce par quoi la note de livraison finale est associée au premier bien de consommation, où la note de livraison finale statue que le premier bien de consommation est exclu pour des nouvelles transmissions,
le procédé comprenant en outre :
• dans le cas d'un établissement d'une prescription pour un premier médicament (172), la réception d'une deuxième demande d'enregistrement (268) pour enregistrer la première prescription dans la chaîne de blocs (106) par l'un des serveurs de chaîne de blocs (102, 132, 142) du réseau de chaînes de blocs (100), où la deuxième demande d'enregistrement (268) comprend un ensemble de données d'éditeur avec des données d'édition de la prescription, où les données d'édition comprennent un identificateur de prescription, où, dans le cas de la prescription, il s'agit d'une prescription pour un premier médicament (172) sous la forme du premier bien de consommation,
• l'exécution de huitièmes instructions de programme du module de programme (108) par le serveur de chaînes de blocs (102, 132, 142) recevant la deuxième demande d'enregistrement (268), où l'exécution des huitièmes instructions de programme comprend une vérification si la deuxième demande d'enregistrement (268) satisfait une ou plusieurs huitièmes conditions (110), et, si les huitièmes conditions (110) sont satisfaites, un ajout de l'ensemble de données d'édition à une entrée (116) dans un bloc supplémentaire de la chaîne de blocs (106),ce par quoi la première prescription est enregistrée dans la chaine de blocs (106),
• dans le cas de la livraison finale du premier bien de consommation à un consommateur final, si les troisièmes conditions (110) sont satisfaites pour la demande de confirmation de la livraison finale (268) du premier bien de consommation, l'ajout de l'identificateur de prescription avec l'entrée (116) dans le bloc supplémentaire de la chaine de blocs (106) avec l'identificateur du premier bien de consommation et la note de livraison finale,
dans lequel la prescription enregistrée dans la chaine de blocs (106) est munie d'un code lisible par machine, où le code lisible par machine de la prescription comprend un identificateur de la prescription,
dans lequel la deuxième demande d'enregistrement (268) comprend une valeur de vérification de livraison finale pour l'enregistrement de la prescription, où la valeur de vérification de livraison finale sert pour la vérification d'une autorisation pour la livraison finale d'un médicament (172) selon la prescription,
dans lequel le code lisible par machine de la prescription comprend une information pour l'autorisation de la livraison finale d'un médicament (172) selon la prescription,
dans lequel, la demande de confirmation de livraison finale (228, 248) reçue dans le cas de la livraison finale du premier bien de consommation au consommateur final comprend le code lisible par machine de la première prescription,
dans lequel une des huitièmes conditions est une signature valide de la deuxième demande d'enregistrement (268) et dans lequel l'exécution des huitièmes instructions de programme comprend la vérification de la signature de la deuxième demande d'enregistrement (268) moyennant l'emploi d'une troisième clé cryptographique publique (114) de l'éditeur de la prescription,
dans lequel la troisième clé cryptographique publique (114) de l'éditeur de la prescription est mise à disposition pour la vérification de la signature de la deuxième demande d'enregistrement (268) par la chaine de blocs (106).

2. Procédé selon la revendication 1, le procédé comprenant en outre :
• dans le cas d'une destruction d'un deuxième bien de consommation enregistré dans la chaine de blocs (106), la réception d'une demande de confirmation de destruction pour la confirmation de la destruction du deuxième bien de consommation par un des serveurs de chaînes de blocs (102, 132, 142) du réseau de chaines de blocs (100), où la demande de confirmation de destruction comprend l'identificateur du deuxième bien de consommation et une preuve d'autorisation du propriétaire actuel avant la destruction pour la transmission du deuxième bien de consommation,
• l'exécution de quatrièmes instructions de programme du module de programme (108) par le serveur de chaînes de blocs (102, 132, 142) recevant la demande de confirmation de destruction, où l'exécution des quatrièmes instructions de programme comprend une vérification, moyennant l'emploi de la valeur de vérification actuelle, si la demande de confirmation de destruction satisfait une ou plusieurs des quatrièmes conditions (110), et, si les quatrièmes conditions (110) sont satisfaites, un ajout de l'identificateur du deuxième bien de consommation et d'une note de destruction à une entrée (116) dans un bloc supplémentaire de la chaîne de blocs (106), ce par quoi la note de destruction est associée au deuxième bien de consommation, où la note de destruction confirme la destruction du deuxième bien de consommation et statue que le deuxième bien de consommation est exclu pour des nouvelles transmissions, et/ou
dans lequel une valeur de vérification actuelle respectivement maximale est associée aux biens de consommation enregistrés dans la chaine de blocs (106), pour laquelle il s'agit respectivement de la valeur de vérification associée en dernier lieu au bien de consommation correspondant.

3. Procédé selon la revendication selon l'une des revendications précédentes, dans lequel les valeurs de vérification de fabricant des biens de consommation enregistrés dans la chaine de blocs (106) sont précis pour les biens de consommation individuels et peuvent être respectivement employés en tant qu'identificateurs des biens de consommation correspondants, ou
dans lequel une valeur de vérification de fabricant est employée pour plusieurs des biens de consommation enregistrés dans la chaine de blocs (106) et dans lequel les identificateurs individuels des biens de consommation enregistrés dans la chaine de blocs (106) sont indépendants des valeurs de vérification de fabricant.

4. Procédé selon l'une des revendications précédentes, dans lequel les valeurs de vérification de fabricant sont respectivement dérivées d'une clé de diversification individuelle pour le propriétaire et peuvent être vérifiées en ce qui concerne leur validité moyennant l'emploi des clés de diversification individuelle pour le propriétaire.

5. Procédé selon l'une des revendications précédentes, dans lequel les valeurs de vérification de fabricant sont respectivement dérivées d'une clé de diversification individuelle pour le propriétaire et peuvent être vérifiées en ce qui concerne leur validité moyennant l'emploi des clés de diversification individuelle pour le propriétaire, et/ou
dans lequel les données de fabrication (176) du premier bien de consommation comprennent une ou plusieurs des indications suivantes : une désignation du premier bien de consommation, une désignation du fabricant du premier bien de consommation, un numéro d'article du premier bien de consommation, un état du premier bien de consommation, une date de conservation limite du premier bien de consommation, et/ou
dans lequel la demande de confirmation de livraison finale (228, 248) comprend un identificateur du consommateur final et l'exécution des troisièmes instructions de programme du module de programmes (108), dans le cas où les troisièmes conditions (110) sont satisfaites, en complément de l'identificateur du premier bien de consommation et de la note de livraison finale, un ajout de l'identificateur du consommateur final avec l'entrée (116) correspondante dans le bloc supplémentaire correspondant de la chaine de blocs (106), ce par quoi l'identificateur du premier bien de consommation est associé à l'identificateur du consommateur final.

6. Procédé selon l'une des revendications 2 à 5, dans lequel la demande de confirmation de destruction comprend des identificateurs d'un ou de plusieurs témoins de la destruction du deuxième bien de consommation et l'exécution des quatrièmes instructions de programme du module de programmes (108), si les quatrièmes conditions (110) sont satisfaites, en complément à l'identificateur du deuxième bien de consommation et de la note de destruction, un ajout d'identificateurs du ou des témoins avec l'entrée (116) correspondante dans le bloc supplémentaire correspondant de la chaine de blocs (106), ce par quoi les identificateurs sont associés au témoin ou aux témoins de la note de destruction du deuxième bien de consommation.

7. Procédé selon l'une des revendications précédentes, dans lequel une des premières conditions (110) est une signature valide de l'ensemble de données de fabricant (182) et dans lequel l'exécution des premières instructions de programme comprend la vérification de la signature de l'ensemble de données de fabricant (182) moyennant l'emploi d'une première clé cryptographique publique (114) du fabricant du premier bien de consommation,
dans lequel, par exemple, la première clé cryptographique publique du fabricant (114) est mise à disposition pour la vérification de la signature de l'ensemble de données de fabricant (182) par la chaine de blocs (106).

8. Procédé selon l'une des revendications précédentes, le procédé comprenant en outre :
• dans le cas d'un conditionnement d'un groupe de biens de consommation enregistrés dans la chaine de blocs (106) dans un récipient de conditionnement commun, la réception d'une demande de confirmation de regroupement (208) pour la confirmation d'un regroupement d'autorisations pour la transmission de biens de consommation du groupe des biens de consommation, où la demande de confirmation de regroupement (208) comprend un identificateur de chaque bien de consommation du groupe de biens de consommation, une preuve d'autorisation du propriétaire actuel de chaque bien de consommation du groupe de biens de consommation avant le conditionnement pour la transmission du bien de consommation correspondant, un identificateur de groupe et une valeur de vérification de créateur de groupes, où la valeur de vérification de créateur de groupes sert pour la vérification d'une autorisation pour la transmission des biens de consommation du groupe de biens de consommation conjointement en tant que groupe par un créateur de groupes en tant que propriétaire de groupes actuel du groupe de biens de consommation,
• l'exécution de cinquièmes instructions de programme du module de programme (108) par le serveur de chaînes de blocs (102, 132, 142) recevant la demande de confirmation de regroupement (208), où l'exécution des cinquièmes instructions de programme comprend une vérification, moyennant l'emploi de la valeur de vérification actuelle des biens de consommation du groupe de biens de consommation, si la demande de confirmation de regroupement (208) satisfait une ou plusieurs des cinquièmes conditions (110), et, si les cinquièmes conditions (110) sont satisfaites, un ajout de la valeur de vérification de créateur de groupe, de l'identificateur de groupe et des identificateurs des biens de consommation du groupe de biens de consommation à une entrée (116) dans un bloc supplémentaire (180,189) de la chaîne de blocs (106), ce par quoi un regroupement des biens de consommation du groupe des biens de consommation est enregistré dans la chaine de blocs (106), ce par quoi la valeur de vérification du créateur de groupe est associée aux biens de consommation du groupe de biens de consommation en tant que valeur de vérification actuelle commune en remplacement des valeurs de vérification actuelles précédentes des biens de consommation individuels du groupe de biens de consommation et dans lequel l'autorisation pour la transmission des biens de consommation du groupe de biens de consommation est associée en tant que groupe au créateur de en tant que propriétaire de groupe actuel du groupe de biens de consommation en remplacement du propriétaire actuel précédent des biens de consommation individuels du groupe de biens de consommation.

9. Procédé selon la revendication 8, le procédé comprenant en outre :
• dans le cas d'un changement de propriétaire du contenant du conditionnement avec le groupe de biens de consommation, la réception d'une demande de confirmation de transmission de groupe (208, 228, 248) pour la transmission du groupe par un des serveurs de chaines de blocs (102, 132, 142) du réseau de chaines de blocs (100), où la demande de confirmation de transmission de groupes (208, 228, 248) comprend l'identificateur de groupe, une preuve d'autorisation du propriétaire de groupe actuel avant le changement de propriétaire pour la transmission du groupe de biens de consommation et une autre valeur de vérification de groupe, où l'autre valeur de vérification de groupe sert à la vérification d'une autorisation pour la transmission du groupe de biens de consommation par un récipiendaire du groupe de biens de consommation en tant que propriétaire de groupe futur,
• l'exécution de sixièmes instructions de programme du module de programme (108) par le serveur de chaînes de blocs (102, 132, 142) recevant la demande de confirmation de transmission de groupe (208, 228, 148), où l'exécution des sixièmes instructions de programme comprend une vérification, moyennant l'emploi de la valeur de vérification de groupe actuelle, si la demande de confirmation de transmission de groupe (208, 228, 248) satisfait une ou plusieurs des sixièmes conditions (110), et, si les sixièmes conditions (110) sont satisfaites, un ajout de l'autre valeur de vérification de groupe et de l'identificateur de groupe d'une entrée (116) dans un bloc supplémentaire (184) de la chaine de blocs (106), ce par quoi l'autre valeur de vérification de groupe est associée au premier bien de consommation en tant que valeur de vérification de groupe actuelle en remplacement de la valeur de vérification de groupe actuelle précédente, et dans lequel l'autorisation pour la transmission du groupe de biens de consommation est associée à l'autre propriétaire de groupe en tant que propriétaire de groupe actuel en remplacement du propriétaire de groupe actuel précédent, et/ou
le procédé comprenant en outre :
• dans le cas d'un déballage du contenu de conditionnement, la réception d'une demande de confirmation de dégroupement (228, 248) pour la confirmation d'une dissolution du regroupement des autorisations pour la transmission des biens de consommation du groupe de biens de consommation, où la demande de confirmation du dégroupement (228, 248) l'identificateur de groupe du groupe, une preuve d'autorisation du propriétaire de groupe actuel du groupe avant le déballage et une ou plusieurs autres valeurs de vérification servent pour la vérification d'une autorisation pour la transmission des biens de consommation du groupe de biens de consommation par un propriétaire futur des biens de consommation du groupe des biens de consommation,
• l'exécution de septièmes instructions de programme du module de programme (108) par le serveur de chaînes de blocs (102, 132, 142) recevant la demande de confirmation de dégroupement (228, 148), où l'exécution des septièmes instructions de programme comprend une vérification, moyennant l'emploi de la valeur de vérification de groupe actuelle, si la demande de confirmation de dégroupement (228, 248) satisfait une ou plusieurs des septièmes conditions (110), et, si les septièmes conditions (110) sont satisfaites, un ajout d'une ou de plusieurs autres valeurs de vérification et des identificateurs de biens de consommation du groupe de biens de consommation à dissoudre à une ou plusieurs entrées (116) dans une ou plusieurs blocs supplémentaires de la chaine de blocs (106), ce par quoi une ou plusieurs autre valeurs de vérification sont respectivement associées à un ou plusieurs des biens de consommation du groupe dissout de biens de consommation en tant que valeur de vérification actuelle en remplacement de la valeur de vérification de groupe commune, et où l'autorisation pour la transmission des biens de consommation individuels du groupe de biens de consommation dissout est associée au propriétaire de groupe actuel précédent en tant que propriétaire actuel des biens de consommation individuels du groupe de biens de consommation dissout, et/ou
dans lequel, au maximum une valeur de vérification de groupe est associée aux groupes de biens de consommation enregistrés dans la chaine de blocs (106), pour laquelle il s'agit respectivement de la valeur de vérification de groupe associée en dernier lieu au groupe correspondant, et/ou
dans lequel les valeurs de vérification de créateur de groupes des groupes de biens de consommation enregistrés dans la chaine de blocs (106) sont uniques pour les groupes individuels et sont respectivement employées en tant qu'identificateurs de groupe des groupes de biens de consommation correspondants, et/ou
dans lequel une valeur de vérification de créateur de groupes est employée pour plusieurs des groupes de biens de consommation enregistrés dans la chaine de blocs (106) et dans lequel les identificateurs de groupes individuels des groupes enregistrés dans la chaine de blocs (106) sont indépendants des valeurs de vérification de créateur de groupes, et/ou
dans lequel, dans le cas des valeurs de vérification de groupes, il s'agit respectivement de valeurs de vérification de groupe individuelles pour le groupe, et/ou
dans lequel une valeur de vérification de groupe est employée pour plusieurs groupes de biens de consommation, et/ou
dans lequel les valeurs de vérification de créateur de groupe sont respectivement dérivées d'une clé de diversification individuelle pour chaque créateur de groupe et peuvent être vérifiées en ce qui concerne leur validité moyennant l'emploi des clés de diversification individuelles pour les créateurs de groupes, et/ou
dans lequel chaque contenu de conditionnement d'un groupe de biens de consommation enregistré dans la chaine de blocs (106) est muni d'un code (174) lisible par machine, où le code (174) lisible par machine comprend un identificateur de groupe du groupe correspondant, et/ou
dans lequel une des cinquièmes conditions (110) est une signature valide de la demande de confirmation de regroupement (208) et dans lequel l'exécution des cinquièmes instructions de programme comprend la vérification de la signature de la demande de confirmation de regroupement (208) moyennant l'emploi d'une deuxième clé cryptographique publique (114) du créateur de groupes du groupe de biens de consommation,
dans lequel, par exemple, la deuxième clé cryptographique publique (114) du créateur de groupes est mise à disposition pour la vérification de la signature de la demande de confirmation de regroupement (208) par la chaine de blocs (106).

10. Procédé selon l'une des revendications précédentes, dans lequel, dans le cas des médicaments (172), il s'agit d'anesthésiques, et
dans lequel, dans le cas des prescriptions, il s'agit de prescriptions d'anesthésiques.

11. Procédé selon l'une des revendications précédentes, le procédé comprenant en outre :
• la réception d'une troisième demande d'enregistrement (308) pour l'enregistrement d'un fabricant de biens de consommation par un serveur de chaine de blocs (102, 132, 142), où la troisième demande d'enregistrement (308) est signée,
• l'exécution de neuvièmes instructions de programme du module de programmes (108) par le serveur de chaine de blocs (102, 132, 142) recevant la troisième demande d'enregistrement (308), où l'exécution des neuvièmes instructions de programme comprend une vérification, moyennant l'emploi de la quatrième clé cryptographique publique (112, 114) si la signature de la troisième demande d'enregistrement (308) est valide, et, dans le cas où la signature de la troisième demande d'enregistrement (308) est valide, un enregistrement du fabricant dans la chaine de blocs (106), où l'enregistrement comprend un ajout d'un identificateur de fabricant à une entrée (116) dans un bloc supplémentaire de la chaine de blocs (106),
dans lequel, par exemple, la quatrième clé cryptographique (112, 114) est mise à disposition pour la vérification de la signature de la troisième demande d'enregistrement (308) par la chaine de blocs (106).

12. Procédé selon l'une des revendications précédentes, le procédé comprenant en outre :
• la réception d'une quatrième demande d'enregistrement (308) pour l'enregistrement d'un propriétaire potentiel par un serveur de chaine de blocs (102, 132, 142), où la quatrième demande d'enregistrement (308) est signée,
• l'exécution de dixièmes instructions de programme du module de programmes (108) par le serveur de chaine de blocs (102, 132, 142) recevant la quatrième demande d'enregistrement (308), où l'exécution des dixièmes instructions de programme comprend une vérification, moyennant l'emploi d'une cinquième clé cryptographique publique (112, 114) si la signature de la quatrième demande d'enregistrement (308) est valide, et, dans le cas où la signature de la quatrième demande d'enregistrement (308) est valide, un enregistrement du propriétaire potentiel dans la chaine de blocs (106), où l'enregistrement comprend un ajout d'un identificateur du propriétaire potentiel à une entrée (116) dans un bloc supplémentaire de la chaine de blocs (106),
dans lequel, par exemple, la cinquième clé cryptographique (112, 114) est mise à disposition pour la vérification de la signature de la troisième demande d'enregistrement (308) par la chaine de blocs (106).

13. Procédé selon l'une des revendications précédentes, le procédé comprenant en outre :
• la réception d'une cinquième demande d'enregistrement (308) pour l'enregistrement d'un éditeur de prescriptions par un serveur de chaine de blocs (102, 132, 142), où la cinquième demande d'enregistrement (308) est signée,
• l'exécution de onzièmes instructions de programme du module de programmes (108) par le serveur de chaine de blocs (102, 132, 142) recevant la cinquième demande d'enregistrement (308), où l'exécution des onzièmes instructions de programme comprend une vérification, moyennant l'emploi d'une sixième clé cryptographique publique (112, 114) si la signature de la cinquième demande d'enregistrement (308) est valide, et, dans le cas où la signature de la cinquième demande d'enregistrement (308) est valide, un enregistrement de l'éditeur dans la chaine de blocs (106), où l'enregistrement comprend un ajout d'un identificateur d'éditeur à une entrée (116) dans un bloc supplémentaire de la chaine de blocs (106),
dans lequel, par exemple, la sixième clé cryptographique (112, 114) est mise à disposition pour la vérification de la signature de la cinquième demande d'enregistrement (308) par la chaine de blocs (106).

14. Procédé selon l'une des revendications précédentes, le procédé comprenant en outre :
• la réception d'une demande de lecture (328) pour la lecture d'entrées (330) avec le premier bien de consommation par un des serveurs de chaine de blocs (102, 132, 142), où la demande de lecture (308) comprend un identificateur du premier bien de consommation,
• l'exécution de douzièmes instructions de programme du module de programmes (108) par le serveur de chaine de blocs (102, 132, 142) recevant la demande de lecture (328), où l'exécution des douzièmes instructions de programme comprend une vérification, par le serveur de chaine de blocs (102, 132, 142) si le premier bien de consommation est enregistré dans la chaine de blocs (106), et si le premier bien de consommation est enregistré dans la chaine de blocs (106), un envoi de copies des entrées (330) avec le premier bien de consommation à un expéditeur de la demande de lecture (328),
dans lequel, par exemple, les entrées (330) avec le premier bien de consommation comprennent un identificateur du consommateur final et/ou d'un ensemble de données d'édition d'une prescription associée au premier bien de consommation, où la vérification comprend une vérification de la signature de la demande de lecture (328) moyennant l'emploi d'une septième clé publique (114),
laquelle est mise à disposition pour la vérification de la signature de la demande de lecture (328), par exemple, par la chaine de blocs (106).

15. Réseau de chaine de blocs (100) pourvu d'une multiplicité de serveurs de chaines de blocs (102, 132, 142) pour la protection contre la falsification et la contrôle de la livraison de biens de consommation moyennant l'emploi d'une chaine de blocs (106) fournie par le réseau de chaines de blocs (100), où, dans le cas des biens de consommation, il s'agit de médicaments (172), où la chaine de blocs (106) enregistre pour chacun des biens de consommation un historique protégé cryptographiquement, où les serveurs de chaines de blocs (102, 132, 142) sont respectivement conçus, moyennant l'emploi d'un module de programme (108) compris par la chaine de blocs (106), pour établir des blocs supplémentaires pour la chaine de blocs (106) et les ajouter à la chaine de blocs (106), où chaque bien de consommation enregistré dans la chaine de blocs (106) est muni d'un code (174) lisible par machine, où le code (174) lisible par machine comprend un identificateur du bien de consommation correspondant,
le réseau de chaines de blocs (100), pour l'enregistrement protégé cryptographiquement d'un historique d'un premier bien de consommation, est conçu pour :
• dans le cas d'une production du premier bien de consommation, la réception d'une première demande d'enregistrement (208) pour enregistrer le premier bien de consommation dans la chaîne de blocs (106) par l'un des serveurs de chaîne de blocs (102, 132, 142) du réseau de chaînes de blocs (100), où la première demande d'enregistrement (208) comprend un ensemble de données de fabricant (182) avec des données de fabrication (176) du premier bien de consommation et avec une valeur de vérification de fabricant, où la valeur de vérification de fabricant sert à vérifier une autorisation pour la transmission du premier bien de consommation par un fabricant du premier bien de consommation,
• l'exécution de premières instructions de programme du module de programme (108) par le serveur de chaînes de blocs (102, 132, 142) recevant la première demande d'enregistrement (208), où l'exécution des premières instructions de programme comprend une vérification si la première demande d'enregistrement (208) satisfait une ou plusieurs premières conditions (110), et, si les premières conditions (110) sont satisfaites, un ajout de l'ensemble de données de fabricant (182) à une entrée (116, 181, 201) dans un bloc supplémentaire (180, 189) de la chaîne de blocs (106), ce par quoi le premier bien de consommation est enregistré dans la chaîne de blocs (106), la valeur de vérification de fabricant est associée au premier bien de consommation en tant que valeur de vérification actuelle et l'autorisation pour la transmission du premier bien de consommation est associée au fabricant en tant que propriétaire actuel du premier bien de consommation,
en outre le réseau de chaines de blocs (100) est conçu pour, en une ou plusieurs fois :
• dans le cas d'un changement de propriétaire du premier bien de consommation, la réception d'une demande de confirmation de transmission (208, 228, 248) pour la confirmation du changement de propriétaire du premier bien de consommation par un des serveurs de chaînes de blocs (102, 132, 142) du réseau de chaines de blocs (100), où la demande de confirmation de transmission (208, 228, 248) comprend un identificateur du premier bien de consommation, une preuve d'autorisation du propriétaire actuel avant le changement de propriétaire pour la transmission du premier bien de consommation et une autre valeur de vérification, où l'autre valeur de vérification sert à la vérification ultérieure d'une autorisation pour la transmission du premier bien de consommation par un réceptionnaire du premier bien de consommation en tant que propriétaire ultérieur,
• l'exécution de deuxièmes instructions de programme du module de programme (108) par le serveur de chaînes de blocs (102, 132, 142) recevant la demande de confirmation de transmission (208, 228, 248), où l'exécution des deuxièmes instructions de programme comprend une vérification, moyennant l'emploi de la valeur de vérification actuelle, si la demande de confirmation de transmission (208, 228, 248) satisfait une ou plusieurs deuxièmes conditions (110), et, si les deuxièmes conditions (110) sont satisfaites, un ajout de l'autre valeur de vérification et de l'identificateur du premier bien de consommation à une entrée (116, 185) dans un bloc supplémentaire (184) de la chaîne de blocs (106), ce par quoi l autre valeur de vérification est associée au premier bien de consommation en tant que valeur de vérification actuelle en remplacement à la valeur de vérification actuelle précédente et où l'autorisation pour la transmission du premier bien de consommation devient associée à l'autre propriétaire en tant que propriétaire actuel du premier bien de consommation en remplacement du propriétaire actuel précédent,
en outre le réseau de chaines de blocs (100) est conçu pour :
• dans le cas d'une livraison finale du premier bien de consommation à un consommateur final, la réception d'une demande de confirmation de livraison finale (228, 248) pour la confirmation de la livraison finale du premier bien de consommation par un des serveurs de chaînes de blocs (102, 132, 142) du réseau de chaines de blocs (100), où la demande de confirmation de livraison finale (228, 248) comprend l'identificateur du premier bien de consommation et une preuve d'autorisation du propriétaire actuel avant la livraison finale pour la transmission du premier bien de consommation,
• l'exécution de troisièmes instructions de programme du module de programme (108) par le serveur de chaînes de blocs (102, 132, 142) recevant la demande de confirmation de livraison finale (228, 248), où l'exécution des troisièmes instructions de programme comprend une vérification, moyennant l'emploi de la valeur de vérification actuelle, si la demande de confirmation de livraison finale (228, 248) du propriétaire actuel satisfait une ou plusieurs troisièmes conditions (110), et, si les troisièmes conditions (110) sont satisfaites, un ajout de l'identificateur du premier bien de consommation et d'une note de livraison finale à une entrée (116) dans un bloc supplémentaire de la chaîne de blocs (106), où la note de livraison finale est enregistrée à la place d'une nouvelle valeur de vérification, ce par quoi la note de livraison finale est associée au premier bien de consommation, où la note de livraison finale statue que le premier bien de consommation est exclu pour des nouvelles transmissions,
en outre le réseau de chaines de blocs (100) est conçu pour :
• dans le cas d'une édition d'une prescription pour un premier médicament (172), la réception d'une deuxième demande d'enregistrement (268) pour enregistrer la première prescription dans la chaîne de blocs (106) par l'un des serveurs de chaîne de blocs (102, 132, 142) du réseau de chaînes de blocs (100), où la deuxième demande d'enregistrement (268) comprend un ensemble de données d'éditeur avec des données d'édition de la prescription, où les données d'édition comprennent un identificateur de prescription, où dans le cas de la prescription, il s'agit d'une prescription pour un premier médicament (172) sous la forme du premier bien de consommation,
• l'exécution de huitièmes instructions de programme du module de programme (108) par le serveur de chaînes de blocs (102, 132, 142) recevant la deuxième demande d'enregistrement (268), où l'exécution des huitièmes instructions de programme comprend une vérification si la deuxième demande d'enregistrement (268) satisfait une ou plusieurs huitièmes conditions (110), et, si les huitièmes conditions (110) sont satisfaites, un ajout de l'ensemble de données d'édition à une entrée (116) dans un bloc supplémentaire de la chaîne de blocs (106),ce par quoi la première prescription est enregistrée dans la chaine de blocs (106),
• dans le cas de la livraison finale du premier bien de consommation à un consommateur final, si les troisièmes conditions (110) sont satisfaites par la demande de confirmation de la livraison finale (268) pour la confirmation de la livraison finale du premier bien de consommation, l'ajout de l'identificateur de prescription avec l'entrée (116) dans le bloc supplémentaire de la chaine de blocs (106) avec l'identificateur du premier bien de consommation et la note de livraison finale,
dans lequel la prescription enregistrée dans la chaine de blocs (106) est munie d'un code lisible par machine, où le code lisible par machine de la prescription comprend un identificateur de la prescription,
dans lequel la deuxième demande d'enregistrement (268) comprend une valeur de vérification de livraison finale pour l'enregistrement de la prescription, où la valeur de vérification de livraison finale sert pour la vérification d'une autorisation pour la livraison finale d'un médicament (172) selon la prescription,
dans lequel le code lisible par machine de la prescription comprend une information pour l'autorisation de la livraison finale d'un médicament (172) selon la prescription,
dans lequel, la demande de confirmation de livraison finale (228, 248) reçue dans le cas de la livraison finale du premier bien de consommation au consommateur final comprend le code lisible par machine de la première prescription,
dans lequel une des huitièmes conditions (110) est une signature valide de la deuxième demande d'enregistrement (268) et dans lequel l'exécution des huitièmes instructions de programme comprend la vérification de la signature de la deuxième demande d'enregistrement (268) moyennant l'emploi d'une troisième clé cryptographique publique (114) de l'éditeur de la prescription,
dans lequel la troisième clé cryptographique publique (114) de l'éditeur de la prescription est mise à disposition pour la vérification de la signature de la deuxième demande d'enregistrement (268) par la chaine de blocs (106).
